(19) Europäisches Patentamt / European Patent Office / Office européen des brevets

(11) EP 1 900 821 A1

(12) EUROPEAN PATENT APPLICATION
published in accordance with Art. 153(4) EPC

(43) Date of publication:
19.03.2008 Bulletin 2008/12

(21) Application number: 06756916.0

(22) Date of filing: 02.06.2006

(51) Int Cl.:
C12P 13/04 (2006.01) C12N 15/09 (2006.01)
C12N 1/15 (2006.01) C12N 1/19 (2006.01)
C12N 1/21 (2006.01) C12N 5/10 (2006.01)
C12R 1/01 (2006.01) C12R 1/07 (2006.01)
C12R 1/19 (2006.01) C12R 1/645 (2006.01)
C12R 1/72 (2006.01)

(86) International application number:
PCT/JP2006/311081

(87) International publication number:
WO 2006/132145 (14.12.2006 Gazette 2006/50)

(84) Designated Contracting States:
DE

(30) Priority: 09.06.2005 JP 2005169919

(71) Applicant: DAICEL CHEMICAL INDUSTRIES, LTD.
Osaka-shi, Osaka 530-0001 (JP)

(72) Inventors:
- KIMOTO, Norihiro
3053261 (JP)
- YAMAMOTO, Hiroaki
3050047 (JP)
- HAYASHI, Motoko
3050047 (JP)

(74) Representative: Grünecker, Kinkeldey,
Stockmair & Schwanhäusser
Anwaltssozietät
Maximilianstrasse 58
80538 München (DE)

# (54) PROCESS FOR PRODUCTION OF L-AMINO ACID

(57) Disclosed is a process for producing the L-form of an amino acid enzymatically from an enantiomeric mixture of the amino acid. The process includes the step of contacting a transformant or a treated product thereof with an enantiomeric mixture of the amino acid. The transformant includes a single host and one or more recombinant vectors introduced thereinto, in which the one or more recombinant vectors include one or more expression vectors and nucleotide sequences integrated thereinto respectively, and the nucleotide sequences are a nucleotide sequence encoding an enzyme capable of converting the D-form of an amino acid into a keto acid and a nucleotide sequence encoding an enzyme capable of converting a keto acid into the L-form of an amino acid. According to this process, a reaction for converting the D-form of an amino acid into a keto acid and a reaction for converting the keto acid into the L-form of the amino acid can be conducted in a single step. Disclosed also is a transformant capable of converting into the L-form of an amino acid as accumulated in a high concentration. The L-form of an amino acid can be efficiently produced from an inexpensive starting material by using the transformant.



Printed by Jouve, 75001 PARIS (FR)

SEQUENCE LISTING

<110> DAICEL CHEMICAL INDUSTRIES, LTD.

<120> PROCESS FOR PRODUCING L-AMINO ACIDS

<130> EP55550HV163pau

<140> EP 06 756 916.0
<141> 2006-06-02

<150> PCT/JP2006/311081
<151> 2006-06-02

<150> JP 2005-169919
<151> 2005-06-09

<160> 44

<170> PatentIn version 3.3

<210> 1
<211> 69
<212> DNA
<213> Artificial Sequence

<220>
<223> an artificially synthesized primer sequence

<400> 1
cgattaactt tattattaaa aattaaagag gtatatacat atgaaatatt taattaagga 60

ggaataatc 69

<210> 2
<211> 71
<212> DNA
<213> Artificial Sequence

<220>
<223> an artificially synthesized primer sequence

<400> 2
catggattat tcctccttaa ttaaatattt catatgtata tacctcttta atttttaata 60

ataaagttaa t 71

<210> 3
<211> 35
<212> DNA
<213> Artificial Sequence

<220>
<223> an artificially synthesized primer sequence

<400> 3
caccatatgg gtgatcaaat tgttgttctt ggttc 35

<210> 4
<211> 54
<212> DNA
<213> Artificial Sequence

<220>
<223> an artificially synthesized primer sequence

<400> 4
cgacttaagt ctagattaaa gtttagcttt aactttttgg ttatcaacta aaag 54

<210> 5
<211> 26
<212> DNA
<213> Artificial Sequence

<220>
<223> an artificially synthesized primer sequence

<400> 5
gacgccggcg ttgcaggttt aactac 26

<210> 6
<211> 40
<212> DNA
<213> Artificial Sequence

<220>
<223> an artificially synthesized primer sequence

<400> 6
ctcaagcttc tagattaaag atttggacga gtaagagctc 40

<210> 7
<211> 30
<212> DNA
<213> Artificial Sequence

<220>
<223> an artificially synthesized primer sequence

<400> 7
cagccatggc taaaatcgtt gttattggtg 30

<210> 8
<211> 34
<212> DNA
<213> Artificial Sequence

<220>
<223> an artificially synthesized primer sequence

<400> 8
ccggcaccaa taacaacgat tttagccatg gctg 34

<210> 9
<211> 38
<212> DNA
<213> Artificial Sequence

<220>
<223> an artificially synthesized primer sequence

<400> 9
tggccatggc tcgtgttgta attctgggaa gtggtgtg 38

<210> 10
<211> 37
<212> DNA
<213> Artificial Sequence

<220>
<223> an artificially synthesized primer sequence

<400> 10
cagtctagat taagaatgtg caccatgtaa atggccc 37

<210> 11
<211> 50
<212> DNA
<213> Artificial Sequence

<220>
<223> an artificially synthesized primer sequence

<400> 11
gaggaattct gtcatgaaaa ttggtattcc aaaagaaatc aaaaacaatg 50

<210> 12
<211> 43
<212> DNA
<213> Artificial Sequence

<220>
<223> an artificially synthesized primer sequence

<400> 12
ctgaagcttc tagattatcc atgtaacaac gaatgaacat ctg 43

<210> 13
<211> 1119
<212> DNA
<213> Geobacillus stearothermophilus

<400> 13
atgaaaattg gtattccaaa agaaatcaaa aacaatgaaa accgcgtcgc catcactccg 60
gcaggcgtga tgacgctcgt caaagcgggg catgacgtgt atgtggagac ggaagccggc 120
gctgggtcgg ggttttccga ttccgagtat gaaaaagccg gggcagtgat cgtgccgaac 180
gcggaagatg cttggacggc ggagatggtg ttgaaagtga aagagccgct ggctgaggag 240
ttccgctatt ttcgccccgg attgattttg tttacgtatt tgcatttagc cgcggccgaa 300
gcgctcacga aagcgctcgt cgagcaaaaa gtggtcggca tcgcttacga gacggtgcag 360
ctggcgaacg gctcgctgcc actgttgacg ccgatgagtg aagtcgccgg ccgcatgtcg 420
gtgcaagtcg gcgcccagtt tctcgagaag ccgcacggcg ggaaaggcat tttgcttggc 480
ggcgtgcccg gagtgcggcg cggcaaagtg acgatcatcg gcggcggaac ggcggggacg 540
aacgcggcga aaatcgcggt cggtctcggg tcagacgtga cgattttgga cattaacgcc 600
gagcggctgc gcgagctcga tgatttgttc ggcgaccacg tgacgacgct catgtccaac 660
tcgtaccata tcgccgagtg cgtgcgcgaa tcggatttgg tcgtcggtgc cgtcttgatc 720
ccgggggcga aagcgccgaa gctggtgacg gaagagatgg tgcgctcgat gacgccggga 780
tcggtgttgg tcgacatcgc cattgaccaa ggcggcattt tcgaaacgac cgaccgcgtc 840
acgacgcacg acgatccgac atacgtcaag cacggcgtcg tccattacgc cgtcgccaac 900
atgccgggcg cggtgccgcg cacgtcgaca ttcgcgctta cgaacgtcac gatcccatac 960

gccttgcaaa tcgccaacaa aggctaccgc gccgcgtgct tggataaccc ggcgctgtta 1020

aaagggatca acacgctcga cgggcacatc gtgtacgaag cggtcgcggc ggcgcacaac 1080

atgccgtata cagatgttca ttcgttgtta catggataa 1119

<210> 14
<211> 38
<212> DNA
<213> Artificial Sequence

<220>
<223> an artificially synthesized primer sequence

<400> 14
gaggaattct attcatgatt attggtgtgc caaaggaa 38

<210> 15
<211> 37
<212> DNA
<213> Artificial Sequence

<220>
<223> an artificially synthesized primer sequence

<400> 15
cagaagcttc tagattaatt agcagccaac gttttcc 37

<210> 16
<211> 50
<212> DNA
<213> Artificial Sequence

<220>
<223> an artificially synthesized primer sequence

<400> 16
gtggaattct atcatgaaaa ttggtattcc aaaggaaatt aaaaacaacg 50

<210> 17
<211> 47
<212> DNA
<213> Artificial Sequence

<220>
<223> an artificially synthesized primer sequence

<400> 17
ctgaagcttc tagattattg gattaattca tccacattca catatgg 47

<210> 18
<211> 1116
<212> DNA
<213> Bacillus sphaericus

<400> 18
atgaaaattg gtattccaaa ggaaattaaa aacaacgaaa atcgcgtagc aatgacacca 60

gcaggagttg tatccttaac gcatgctggg cacgaagtgt atattgaaac aggagctggt 120

atcggttcaa gttttacaga tgcagattac gtagcagcag gtgcacatat cgttgcatcg 180

gcaaaagaag cgtgggctca agaaatgatt ttaaaagtaa aagaacccgt agcatcggaa 240

tacgattact tttatgaggg acaaatctta tttacctact tgcacttagc gccagagcgt 300
gaattaacgc aggcattaat agataaaaaa gttgtaggta ttgcctatga aacggttcaa 360
cttgcaaatg gttcactacc tttattaaca ccaatgagtg aagtagctgg taaaatggca 420
acacaaattg gtgcgcaata tttagagaaa aatcacggtg gtaaagggat tttactaggc 480
ggtgtatcag gtgtacaacg cggtaaagta acagtaattg gtggcggaat cgccggaaca 540
aacgctgcga aaattgcagt tggtatggga gcagacgtaa cagttattga tttaagtcca 600
gaacgtctac gtcaattaga agatatgttt ggtcgcgatg ttcaaacatt aatgtctaac 660
ccgtataata ttgcagaatc tgtgaaacac tcagatttag ttgtcggtgc tgttctaatt 720
cctggtgcaa aggctccaaa gttagtttcg gaagaaatga ttcaatcgat gcaaccaggt 780
tctgttgttg tggatattgc gattgaccaa ggtggaattt ttgcgacatc tgatcgtgtt 840
acaacacatg atgatccaac gtatgttaaa catggggtag tccactatgc tgttgcaaat 900
atgccagggg ctgtgccacg tacttcaacg attgctttaa caaataatac aattccttat 960
gcgttgcaaa ttgccaataa aggctataag caagcatgta ttgacaatcc tgcattgaaa 1020
aaaggtgtga atgcattaga ggggcatatt acttataaag cggtagcaga agcacaaggc 1080
ttgccatatg tgaatgtgga tgaattaatc caataa 1116

<210> 19
<211> 40
<212> DNA
<213> Artificial Sequence

<220>
<223> an artificially synthesized primer sequence

<400> 19
agagaattct atcatgatta ttggtgttcc aacagaaatc 40

<210> 20
<211> 40
<212> DNA
<213> Artificial Sequence

<220>
<223> an artificially synthesized primer sequence

<400> 20
gacaagcttc tagattaagc aagtaggctt tttggttcag 40

<210> 21
<211> 60
<212> DNA
<213> Artificial Sequence

<220>
<223> an artificially synthesized primer sequence

<400> 21
gtctctagag gaattctacc atggaattgt tcaaatatat ggaaacttac gattatgagc 60

<210> 22
<211> 36

<212> DNA
<213> Artificial Sequence

<220>
<223> an artificially synthesized primer sequence

<400> 22
ctgaagcttc tagattatat tgccgaagca cctgcc 36

<210> 23
<211> 1104
<212> DNA
<213> Geobacillus stearohtermophilus

<400> 23
atggaattgt tcaaatatat ggaaacttac gattatgagc aagtgctgtt ttgccaagat 60

aaagaatcgg gtttgaaagc gatcattgcc attcatgaca caacgctcgg cccggcgctc 120

ggcgggacgc gcatgtggat gtacaattcg gaagaagaag cgcttgaaga cgccttgcgc 180

ctcgcccgcg gcatgacgta caaaaacgcg gccgccggcc tcaacttggg cgggggcaaa 240

acggtcatca tcggcgaccc gcgcaaagat aaaaacgaag cgatgttccg ggcgttcggc 300

cgcttcattc aagggctgaa cggccgctac atcacggcgg aagacgtcgg cacgaccgtc 360

gccgatatgg atatcatcta tcaagaaacc gactatgtca ccggcatttc gcccgaattc 420

ggctcatccg gcaacccatc gccggcgacc gcctacggcg tataccgcgg catgaaggcg 480

gcggcaaaag aggcattcgg cagcgattcg ctcgaaggaa aagtcgtcgc cgtccaagga 540

gtcggcaatg tcgcgtatca tttgtgccgc catttgcacg aagaaggagc gaaactcatc 600

gtgactgaca tcaacaagga agcggtggcg cgcgcagtcg aggaattcgg agcgaaagcg 660

gtcgacccga acgacattta cggcgtggag tgcgacattt ttgctccatg cgcgctcggc 720

ggcatcatca acgatcaaac gattccgcaa ctgaaagcga aagtgatcgc cggatcggcg 780

aacaaccagc tgaaagagcc gcgccatggc gacatcatcc atgaaatggg catcgtctat 840

gccccggatt atgtgatcaa cgccggcggc gtcatcaatg tcgcggacga actgtacggc 900

tacaatcggg aacgggcgat gaaaaaaatc gagcaaattt atgacaacat cgaaaaagtg 960

tttgccatcg ccaagcgcga caacattcca acgtatgtgg ccgccgaccg gatggcggaa 1020

gaacggattg aaacgatgcg caaagcgcgc agtcaatttt tgcaaaatgg tcaccatatt 1080

ttaagccgcc gtcgcgcccg ctaa 1104

<210> 24
<211> 18
<212> DNA
<213> Artificial Sequence

<220>
<223> an artificially synthesized primer sequence

<400> 24
gcggtcgacc cgaacgac 18

<210> 25
<211> 38

<212> DNA
<213> Artificial Sequence

<220>
<223> an artificially synthesized primer sequence

<400> 25
ctgaagcttc tagattaacg tgcacgacgg cggcttaa 38

<210> 26
<211> 44
<212> DNA
<213> Artificial Sequence

<220>
<223> an artificially synthesized primer sequence

<400> 26
gtggaattct accatggaaa tcttcaagta tatggaaaag tatg 44

<210> 27
<211> 57
<212> DNA
<213> Artificial Sequence

<220>
<223> an artificially synthesized primer sequence

<400> 27
ctccttaagt ctagattaac gaccgttcaa aatgtttttt tcatttttta agaactg 57

<210> 28
<211> 40
<212> DNA
<213> Artificial Sequence

<220>
<223> an artificially synthesized primer sequence

<400> 28
caggaattct ataatgcgtg atgtatttga aatgatggac 40

<210> 29
<211> 47
<212> DNA
<213> Artificial Sequence

<220>
<223> an artificially synthesized primer sequence

<400> 29
ctgaagcttc tagattaacg acgtgcacta ttacggggat cctccaa 47

<210> 30
<211> 43
<212> DNA
<213> Artificial Sequence

<220>
<223> an artificially synthesized primer sequence

<400> 30
ctgtcatgac tactaagaaa gctgattaca tttggttcaa tgg 43

<210> 31
<211> 39
<212> DNA
<213> Artificial Sequence

<220>
<223> an artificially synthesized primer sequence

<400> 31
gtctctagat tattgattaa cttgatctaa ccagcccca 39

<210> 32
<211> 31
<212> DNA
<213> Artificial Sequence

<220>
<223> an artificially synthesized primer sequence

<400> 32
tgatcatgac taaacaaaca attcgcgttg a 31

<210> 33
<211> 33
<212> DNA
<213> Artificial Sequence

<220>
<223> an artificially synthesized primer sequence

<400> 33
ttctagatta tttactttca gtcagcgctg cga 33

<210> 34
<211> 43
<212> DNA
<213> Artificial Sequence

<220>
<223> an artificially synthesized primer sequence

<400> 34
gtcttaatta aagtttagct ttaacttttt ggttatcaac taa 43

<210> 35
<211> 33
<212> DNA
<213> Artificial Sequence

<220>
<223> an artificially synthesized primer sequence

<400> 35
ctagtagagg tacctatata tgcatgtgca cgc 33

<210> 36
<211> 33
<212> DNA
<213> Artificial Sequence

<220>
<223> an artificially synthesized primer sequence

<400> 36
ttaagcgtgc acatgcatat ataggtacct cta 33

<210> 37
<211> 43
<212> DNA
<213> Artificial Sequence

<220>
<223> an artificially synthesized primer sequence

<400> 37
gacggtacct atacatgtct caacataacg aaaagaaccc aca 43

<210> 38
<211> 45
<212> DNA
<213> Artificial Sequence

<220>
<223> an artificially synthesized primer sequence

<400> 38
tcgaagctta agattatgca ggaattttgt caatcttagg aatgc 45

<210> 39
<211> 2289
<212> DNA
<213> Escherichia coli

<220>
<221> EckatE catalase
<222> (15)..(2276)
<223> Peroxidase activity of EcatE is lower than that of EcatG.

<400> 39
gacggtacct atacatgtct caacataacg aaaagaaccc acatcagcac cagtcaccac 60

tacacgattc cagcgaagcg aaaccgggga tggactcact ggcacctgag gacggctctc 120

atcgtccagc ggctgaacca acaccgccag gtgcacaacc taccgcccca gggagcctga 180

aagcccctga tacgcgtaac gaaaaactta attctctgga agacgtacgc aaaggcagtg 240

aaaattatgc gctgaccact aatcagggcg tgcgcatcgc cgacgatcaa aactcactgc 300

gtgccggtag ccgtggtcca acgctgctgg aagattttat tctgcgcgag aaaatcaccc 360

actttgacca tgagcgcatt ccggaacgta ttgttcatgc acgcggatca gccgctcacg 420

gttatttcca gccatataaa agcttaagcg atattaccaa agcggatttc ctctcagatc 480

cgaacaaaat caccccagta tttgtacgtt tctctaccgt tcagggtggt gctggctctg 540

ctgataccgt gcgtgatatc cgtggctttg ccaccaagtt ctataccgaa gagggtattt 600

ttgacctcgt tggcaataac acgccaatct tctttatcca ggatgcgcat aaattccccg 660

attttgttca tgcggtaaaa ccagaaccgc actgggcaat tccacaaggg caaagtgccc 720

acgatacttt ctgggattat gtttctctgc aacctgaaac tctgcacaac gtgatgtggg 780

cgatgtcgga tcgcggcatc cccgcagtt accgcaccat ggaaggcttc ggtattcaca 840

ccttccgcct gattaatgcc gaagggaagg caacgtttgt acgtttccac tggaaaccac 900

tggcaggtaa agcctcactc gtttgggatg aagcacaaaa actcaccgga cgtgacccgg 960
acttccaccg ccgcgagttg tgggaagcca ttgaagcagg cgattttccg gaatacgaac 1020
tgggcttcca gttgattcct gaagaagatg aattcaagtt cgacttcgat cttctcgatc 1080
caaccaaact tatcccggaa gaactggtgc ccgttcagcg tgtcggcaaa atggtgctca 1140
atcgcaaccc ggataacttc tttgctgaaa acgaacaggc ggctttccat cctgggcata 1200
tcgtgccggg actggacttc accaacgatc cgctgttgca gggacgtttg ttctcctata 1260
ccgatacaca aatcagtcgt cttggtgggc cgaatttcca tgagattccg attaaccgtc 1320
cgacctgccc ttaccataat ttccagcgtg acggcatgca tcgcatgggg atcgacacta 1380
acccggcgaa ttacgaaccg aactcgatta acgataactg gccgcgcgaa acaccgccgg 1440
ggccgaaacg cggcggtttt gaatcatacc aggagcgcgt ggaaggcaat aaagttcgcg 1500
agcgcagccc atcgtttggc gaatattatt cccatccgcg tctgttctgg ctaagtcaga 1560
cgccatttga gcagcgccat attgtcgatg gtttcagttt tgagttaagc aaagtcgttc 1620
gtccgtatat tcgtgagcgc gttgttgacc agctggcgca tattgatctc actctggccc 1680
aggcggtggc gaaaaatctc ggtatcgaac tgactgacga ccagctgaat atcaccccac 1740
ctccggacgt caacggtctg aaaaaggatc catccttaag tttgtacgcc attcctgacg 1800
gtgatgtgaa aggtcgcgtg gtagcgattt tacttaatga tgaagtgaga tcggcagacc 1860
ttctggccat tctcaaggcg ctgaaggcca aaggcgttca tgccaaactg ctctactccc 1920
gaatgggtga agtgactgcg gatgacggta cggtgttgcc tatagccgct acctttgccg 1980
gtgcaccttc gctgacggtc gatgcggtca ttgtcccttg cggcaatatc gcggatatcg 2040
ctgacaacgg cgatgccaac tactacctga tggaagccta caaacacctt aaaccgattg 2100
cgctggcggg tgacgcgcgc aagtttaaag caacaatcaa gatcgctgac cagggtgaag 2160
aagggattgt ggaagctgac agcgctgacg gtagttttat ggatgaactg ctaacgctga 2220
tggcagcaca ccgcgtgtgg tcacgcattc ctaagattga caaaattcct gcataatctt 2280
aagcttcga 2289

<210> 40
<211> 43
<212> DNA
<213> Artificial Sequence

<220>
<223> an artificially synthesized primer sequence

<400> 40
gacggtacca tatcatgagt acttcagacg atatccataa cac 43

<210> 41
<211> 38
<212> DNA
<213> Artificial Sequence

<220>
<223> an artificially synthesized primer sequence

<400> 41
gacaagctta agattaaagc agatcaaaac ggtcgagg 38

<210> 42
<211> 2208
<212> DNA
<213> Escherichia coli

<220>
<221> EckatG catalase
<222> (15)..(2195)
<223> Peroxidase activity of EcatG is higher than that of EcatE.

<400> 42
gacggtacca tatcatgagt acttcagacg atatccataa caccacagcc actggcaaat 60

gcccgttcca tcagggcggt cacgaccaga gtgcgggggc gggcacaacc actcgcgact 120

ggtggccaaa tcaacttcgt gttgacctgt taaaccaaca ttctaatcgt tctaacccac 180

tgggtgagga ctttgactac cgcaaagaat tcagcaaatt agattactac ggcctgaaaa 240

aagatctgaa agccctgttg acagaatctc aaccgtggtg gccagccgac tggggcagtt 300

acgccggtct gtttattcgt atggcctggc acggcgcggg gacttaccgt tcaatcgatg 360

gacgcggtgg cgcgggtcgt ggtcagcaac gttttgcacc gctgaactcc tggccggata 420

acgtaagcct cgataaagcg cgtcgcctgt tgtggccaat caaacagaaa tatggtcaga 480

aaatctcctg ggccgacctg tttatcctcg cgggtaacgt ggcgctagaa aactccggct 540

tccgtacctt cggttttggt gccggtcgtg aagacgtctg ggaaccggat ctggatgtta 600

actggggtga tgaaaaagcc tggctgactc accgtcatcc ggaagcgctg gcgaaagcac 660

cgctgggtgc aaccgagatg ggtctgattt acgttaaccc ggaaggcccg gatcacagcg 720

gcgaaccgct ttctgcggca gcagctatcc gcgcgacctt cggcaacatg ggcatgaacg 780

acgaagaaac cgtggcgctg attgcgggtg gtcatacgct gggtaaaacc cacggtgccg 840

gtccgacatc aaatgtaggt cctgatccag aagctgcacc gattgaagaa caaggtttag 900

gttgggcgag cacttacggc agcggcgttg gcgcagatgc cattacctct ggtctggaag 960

tagtctggac ccagacgccg acccagtgga gcaactattt cttcgagaac ctgttcaagt 1020

atgagtgggt acagacccgc agcccggctg gcgcaatcca gttcgaagcg gtagacgcac 1080

cggaaattat cccggatccg tttgatccgt cgaagaaacg taaaccgaca atgctggtga 1140

ccgacctgac gctgcgtttt gatcctgagt tcgagaagat ctctcgtcgt ttcctcaacg 1200

atccgcaggc gttcaacgaa gcctttgccc gtgcctggtt caaactgacg cacagggata 1260

tggggccgaa atctcgctac atcgggccgg aagtgccgaa agaagatctg atctggcaag 1320

atccgctgcc gcagccgatc tacaacccga ccgagcagga cattatcgat ctgaaattcg 1380

cgattgcgga ttctggtctg tctgttagtg agctggtatc ggtggcctgg gcatctgctt 1440

ctaccttccg tggtggcgac aaacgcggtg gtgccaacgg tgcgcgtctg gcattaatgc 1500

cgcagcgcga ctgggatgtg aacgccgcag ccgttcgtgc tctgcctgtt ctggagaaaa 1560

```
tccagaaaga gtctggtaaa gcctcgctgg cggatatcat agtgctggct ggtgtggttg    1620
gtgttgagaa agccgcaagc gccgcaggtt tgagcattca tgtaccgttt gcgccgggtc    1680
gcgttgatgc gcgtcaggat cagactgaca ttgagatgtt tgagctgctg gagccaattg    1740
ctgacggttt ccgtaactat cgcgctcgtc tggacgtttc caccaccgag tcactgctga    1800
tcgacaaagc acagcaactg acgctgaccg cgccggaaat gactgcgctg gtgggcggca    1860
tgcgtgtact gggtgccaac ttcgatggca gcaaaaacgg cgtcttcact gaccgcgttg    1920
gcgtattgag caatgacttc ttcgtgaact tgctggatat gcgttacgag tggaaagcga    1980
ccgacgaatc gaaagagctg ttcgaaggcc gtgaccgtga aaccggcgaa gtgaaattta    2040
cggccagccg tgcggatctg gtgtttggtt ctaactccgt cctgcgtgcg gtggcggaag    2100
tttacgccag tagcgatgcc cacgagaagt ttgttaaaga cttcgtggcg gcatgggtga    2160
aagtgatgaa cctcgaccgt tttgatctgc tttaatctta agcttgtc                 2208
```

```
<210>  43
<211>  40
<212>  DNA
<213>  Artificial Sequence

<220>
<223>  an artificially synthesized primer sequence

<400>  43
caggaattct ataatgcgtg atgtatttga aatgatggac                          40


<210>  44
<211>  34
<212>  DNA
<213>  Artificial Sequence

<220>
<223>  an artificially synthesized primer sequence

<400>  44
gtcaggtacc tcttaacgac gtgcactatt acgg                                34
```

## Description

Technical Field

The present invention relates to a process for producing the L-form of an amino acid (L-amino acid) from an enantiomeric mixture of the amino acid as a starting material and using a transformant that expresses an enzyme capable of converting the D-form of an amino acid into a keto acid and an enzyme capable of converting a keto acid into the L-form of an amino acid.

Background Art

Importance of the synthesis of optically active substances has recently increased in the fields typically of medicinal products, agricultural chemicals, foodstuffs, animal feeds, and flavors. This is because some pairs of enantiomers have different bioactivities between them. How to obtain pure optically active substances has therefore been an industrially important issue. Among such optically active substances, L-amino acids typified by naturally-occurring L-amino acids have been positively used as easily-available chiral synthons for the synthetically preparation of their derivatives. In addition, those having nonnatural structures have also been used. Obtaining pure optically active substances takes on increasing importance.

There have been known the following processes for synthetically preparing nonnatural L-amino acids:

(1) a process of producing the L-form of an amino acid from an acylated racemate of the amino acid as a starting material by the action of an L-aminoacylase (Method Enzymol., 3, 554, (1957));
(2) a process of producing the L-form of an amino acid by subjecting hydantoin to ring-opening by the catalysis of L-hydantoinase and eliminating carbamoyl group by the catalysis typically of an L-carbamoylase (Agric. Biol. Chem., 51, 729 (1087));
(3) a process of producing the L-form of an amino acid from an amidated racemate as a starting material by the catalysis of an amidase (Method Enzymol., 3, 554, (1957));
(4) a process of selectively decomposing the D-form of an amino acid (D-amino acid) in a racemate as a starting material to leave the L-form of the amino acid (Biotechnol. Bioeng., 14, 1288 (1994)); and
(5) a process of synthetically preparing the L-form of an amino acid by reductive amination of a keto acid (Japanese Unexamined Patent Application Publication (JP-A) No. H10-23896).

All these processes, however, are not satisfactory for industrial use. The process (5), for example, uses keto acids as starting materials, but they are generally expensive and unsuitable as industrial starting materials. Productions of L-amino acids may be carried out at lower cost using racemic amino acids as starting materials than that using keto acids in some structures of the amino acids. On the other hand, a process of subjecting a racemate as a starting material simply to optical resolution has a theoretical yield of a target L-amino acid of not more than 50%. To improve the yield, an extra racemization is required. However, if optical resolution alone is conducted, an unnecessary D-amino acid turns into a waste, and it is difficult to bring the theoretical yield close to 100%.

As a process of recycling an unnecessary D-form to improve the yield, there have been proposed processes which include the step of stereoinverting the D-form of an amino acid and processes which include a combination of the steps of enzymatically converting the unnecessary D-form into a keto acid, and aminating and thereby converting a keto acid into the L-form of an amino acid. For example, the following processes have been reported as processes for converting all of a racemate as a starting material into the L-form of an amino acid:

(6) a process of enzymatically synthesizing the L-form of an amino acid by converting the D-form of the amino acid in a racemate of the amino acid as a starting material into a keto acid by the actions of a catalase, and yeast cells or a D-amino acid oxidase, and converting the keto acid into the corresponding L-form of the amino acid by the actions of an L-amino acid dehydrogenase and a glucose dehydrogenase (Biomolecular Engineering, 17, 167 (2001)); and
(7) a process of preparing the L-from of an amino acid using both an enzymatic technique and a chemical technique by repeating the steps of oxidizing the D-form of an amino acid contained in a racemate of the amino acid as a starting material into an imine as an intermediate by the action of a D-amino acid oxidase, and reducing the imine by using a hydride reducing agent or by catalytic hydrogenation with a metal catalyst to thereby regenerate a racemate of the amino acid (racemization) (Chem. Comm., 246 (2002)).

However, the process (6) is economically disadvantageous, because there is need of preparing purified enzymes by expressing large amounts of at least two enzymes used in the steps using, for example, microorganisms, and recovering and purifying the expressed enzymes, or by purchasing commercially available enzymes. In addition, this process includes a complicated procedure of preparing a reaction mixture, because at least two enzymes should be added thereto. The process (7) is disadvantageous in that a harmful metal catalyst is used in the reduction reaction, the reaction should be conducted under pressurized conditions, and the product accumulates in a low concentration.

PCT International Publication Number WO 2005-090590 discloses a recombinant microorganism having increased concentrations or activities of a D-amino acid oxidase, an L-amino acid dehydrogenase, a cosubstrate-NADH regenerating enzyme and, optionally, a catalase; and a process for producing L-amino acids using the recombinant microorganism. In Examples, there is described an example of the production of L-methionine or L-leucine by a process of using a transformant which includes introduced two recombinant vectors expressing one or two enzymes selected from a D-amino acid oxidase derived from *Arthrobacter protophormiae* or *Trigonopsis variabilis,* a leucine dehydrogenase derived from *Bacillus cereus,* and a cosubstrate-NADH regenerating enzyme. It is difficult, however, to use this process in commercial production, because the substrate concentration is very low as low as about 25 mM. This document lacks an example of the combination use of a catalase and fails to teach an effect of the combination use of a catalase concretely.

[Non-patent Document 1] Method Enzymol., 3, 554, (1957)
[Non-patent Document 2] Agric. Biol. Chem., 51, 729 (1087)
[Non-patent Document 3] Biotechnol. Bioeng., 14, 1288 (1994)
[Non-patent Document 4] Biomolecular Engineering, 17, 167 (2001)
[Non-patent Document 5] Chem. Comm., 246 (2002)
[Patent Document 1] JP-A No. H10-23896
[Patent Document 2] PCT International Publication Number WO 2005-090590

## Disclosure of Invention

### Problems to be Solved by the Invention

An object of the present invention is to provide a transformant that has a capability of carrying out a reaction for converting the D-form of an amino acid into a keto acid and a reaction for converting a keto acid into the L-form of an amino acid in one pot (in a single system) while the converted L-form of the amino acid being accumulated in a high concentration; and to provide a process for producing the L-form of an amino acid which is capable of efficiently producing the L-form of an amino acid from an inexpensive starting material using the transformant.

Another object of the present invention is to provide a transformant that has, in addition to the above capabilities, a capability of converting a starting material into the L-from of an amino acid at a higher efficiency by using an enzymatic activity that supplements main reactions; and to provide a process for producing the L-form of an amino acid using this transformant.

Yet another object of the present invention is to provide a process for producing the L-form of an amino acid which can produce the L-form of an amino acid in a high yield and has excellent productivity even when a substrate is used in a high concentration; and to provide a transformant for use in the process.

### Means for Solving the Problems

After intensive investigations to achieve the objects, the present inventors have found that the two reactions can be conducted in one pot (in a single system) and the target L-form of the amino acid can be accumulated in a high concentration using an inexpensive starting material, by using a transformant that coexpresses an enzyme capable of converting the D-form of an amino acid into a keto acid, and an enzyme capable of converting a keto acid into the L-form of an amino acid. The present invention has been made based on these findings.

Specifically, there is provided a process for producing the L-form of an amino acid enzymatically from an enantiomeric mixture of the amino acid. The process includes the step of contacting a transformant or a treated product thereof with an enantiomeric mixture of the amino acid. The transformant includes a single host and one or more recombinant vectors introduced into the single host, in which the one or more recombinant vectors include one or more expression vectors and nucleotide sequences, and the nucleotide sequences include a nucleotide sequence encoding an enzyme capable of converting the D-form of an amino acid into a keto acid and a nucleotide sequence encoding an enzyme capable of converting a keto acid into the L-form of an amino acid. The nucleotide sequences are integrated into the one expression vector or the different expression vectors, respectively. This process is hereinafter also referred to as "L-amino acid production process 1 according to the present invention" or "process 1".

In the process 1 according to the present invention, the transformant may be a transformant which includes a single host and one or more recombinant vectors introduced into the single host, in which the one or more recombinant vectors include one or more expression vectors and nucleotide sequences, and the nucleotide sequences include a nucleotide sequence encoding an enzyme capable of converting the D-form of an amino acid into a keto acid, a nucleotide

sequence encoding an enzyme capable of converting a keto acid into the L-form of an amino acid, and a nucleotide sequence encoding a coenzyme-regenerating enzyme. The nucleotide sequences are integrated into the one expression vector or the different expression vectors, respectively. The transformant may also be a transformant which includes a single host and one or more recombinant vectors introduced into the single host, in which the one or more recombinant vectors include one or more expression vectors and nucleotide sequences, and the nucleotide sequences include a nucleotide sequence encoding an enzyme capable of converting the D-form of an amino acid into a keto acid, a nucleotide sequence encoding an enzyme capable of converting a keto acid into the L-form of an amino acid, and a nucleotide sequence encoding a hydrogen peroxide catabolic enzyme. The nucleotide sequences are integrated into the one expression vector or the different expression vectors, respectively.

The enzyme capable of converting the D-form of an amino acid into a keto acid for use in the present invention may be a D-amino acid oxidase and/or a D-amino acid dehydrogenase. The D-amino acid oxidase includes, for example, at least one D-amino acid oxidase derived from a microorganism selected from the group consisting of *Candida boidinii, Trigonopsis variabilis,* and *Schizosaccharomyces pombe.* The D-amino acid dehydrogenase may be a D-amino acid dehydrogenase derived from *Escherichia coli.* The enzyme capable of converting a keto acid into the L-form of an amino acid may be an L-amino acid dehydrogenase and/or an L-amino acid transaminase. The L-amino acid dehydrogenase includes, for example, a phenylalanine dehydrogenase derived from *Thermoactinomyces intermedius,* an alanine dehydrogenase derived from *Geobacillus stearothermophilus,* an alanine dehydrogenase derived from *Bacillus thermocatenulatus,* an alanine dehydrogenase derived from *Bacillus sphaericus,* an alanine dehydrogenase derived from *Shewanella species,* a leucine dehydrogenase derived from *Geobacillus stearothermophilus,* and a leucine dehydrogenase derived from *Bacillus sphaericus.*

The coenzyme-regenerating enzyme for use in the present invention may be a formate dehydrogenase. The formate dehydrogenase includes a formate dehydrogenase derived from *Mycobacterium vaccae.* The hydrogen peroxide catabolic enzyme may be a catalase. The catalase may be a catalase derived from *Escherichia coli.*

In a preferred embodiment, the process 1 according to the present invention may be a process in which the enzyme capable of converting the D-form of an amino acid into a keto acid is a D-amino acid oxidase, the enzyme capable of converting a keto acid into the L-form of an amino acid is an L-amino acid dehydrogenase, the coenzyme-regenerating enzyme is a formate dehydrogenase, and the hydrogen peroxide catabolic enzyme is a catalase. In a more preferred embodiment, the process may be a process in which the D-amino acid oxidase is one derived from *Candida boidinii,* the L-amino acid dehydrogenase is a phenylalanine dehydrogenase derived from *Thermoactinomyces intermedius,* the formate dehydrogenase is one derived from *Mycobacterium vaccae,* and the catalase is one derived from *Escherichia coli.*

A preferred example of the L-form of the amino acid in the present invention includes L-norvaline.

According to the present invention, there is further provided a transformant which includes a single host and one or more recombinant vectors introduced into the single host. The one or more recombinant vectors herein include one or more expression vectors and nucleotide sequences, and the nucleotide sequences include a nucleotide sequence encoding an enzyme capable of converting the D-form of an amino acid into a keto acid, a nucleotide sequence encoding an enzyme capable of converting a keto acid into the L-form of an amino acid, a nucleotide sequence encoding a coenzyme-regenerating enzyme, and a nucleotide sequence encoding a hydrogen peroxide catabolic enzyme. The nucleotide sequences are integrated into the one expression vector or the different expression vectors, respectively.

In a preferred embodiment, the transformant according to the present invention is, for example, a transformant in which the enzyme capable of converting the D-form of an amino acid into a keto acid is a D-amino acid oxidase, the enzyme capable of converting a keto acid into the L-form of an amino acid is an L-amino acid dehydrogenase, the coenzyme-regenerating enzyme is a formate dehydrogenase, and the hydrogen peroxide catabolic enzyme is a catalase. In a more preferred embodiment, the transformant is, for example, a transformant in which the D-amino acid oxidase is one derived from *Candida boidinii,* the L-amino acid dehydrogenase is a phenylalanine dehydrogenase derived from *Thermoactinomyces intermedius,* the formate dehydrogenase is one derived from *Mycobacterium vaccae,* and the catalase is one derived from *Escherichia coli.*

According to the present invention, there is also provided a recombinant vector which includes a single expression vector and nucleotide sequences integrated into the single expression vector, in which the nucleotide sequences include a nucleotide sequence encoding an enzyme capable of converting the D-form of an amino acid into a keto acid, a nucleotide sequence encoding an enzyme capable of converting a keto acid into the L-form of an amino acid, a nucleotide sequence encoding a coenzyme-regenerating enzyme, and a nucleotide sequence encoding a hydrogen peroxide catabolic enzyme.

In a preferred embodiment, the recombinant vector according to the present invention is, for example, a recombinant vector in which the enzyme capable of converting the D-form of an amino acid into a keto acid is a D-amino acid oxidase, the enzyme capable of converting a keto acid into the L-form of an amino acid is an L-amino acid dehydrogenase, the coenzyme-regenerating enzyme is a formate dehydrogenase, and the hydrogen peroxide catabolic enzyme is a catalase. In a more preferred embodiment, the recombinant vector is a recombinant vector in which the D-amino acid oxidase is one derived from *Candida boidinii,* the L-amino acid dehydrogenase is a phenylalanine dehydrogenase derived from *Thermoactinomyces intermedius,* the formate dehydrogenase is one derived from *Mycobacterium vaccae,* and the catalase is one derived from *Escherichia coli.*

There is further provided, according to the present invention, a process for producing the L-form of an amino acid enzymatically from an enantiomeric mixture of the amino acid. The process includes two or more different reaction steps including an oxidation step and a reduction step. The oxidation step mainly includes oxidizing the D-form of an amino acid using a transformant or a treated product thereof, in which the transformant coexpresses at least an enzyme capable of oxidizing the D-form of an amino acid to a keto acid and a hydrogen peroxide catabolic enzyme. The reduction step mainly includes synthetically producing the L-form of an amino acid from a keto acid using a transformant or a treated product thereof, in which the transformant expresses an enzyme capable of producing the L-form of an amino acid from a keto acid and optionally coexpresses a coenzyme-regenerating enzyme according to necessity. This process is hereinafter also referred to as "L-amino acid production process 2 according to the present invention" or "process 2". In the process 2 according to the present invention, the transformant according to the present invention may be used.

The process 2 according to the present invention may be a process in which the oxidation step and the reduction step are carried out under different reaction conditions. In the process 2 according to the present invention, for example, the oxidation step may be carried out under aeration of an oxygen-containing gas, and the reduction step may be carried out without aeration of an oxygen-containing gas or under aeration of an oxygen-containing gas at an aeration rate lower than that in the oxidation step.

The process 2 according to the present invention includes, for example, a process in which an aeration rate of the oxygen-containing gas in the oxidation step is 1 percent by volume per minute or more relative to a reaction mixture, and an aeration rate of the oxygen-containing gas in the reduction step is less than 10 percent by volume per minute relative to a reaction mixture. In a preferred embodiment, the process may be a process in which an aeration rate of the oxygen-containing gas in the oxidation step is 2 percent by volume per minute or more relative to a reaction mixture, and an aeration rate of the oxygen-containing gas in the reduction step is less than 2 percent by volume per minute relative to a reaction mixture. In a more preferred embodiment, the process may be a process in which an aeration rate of the oxygen-containing gas in the oxidation step is 5 percent by volume per minute or more relative to a reaction mixture, and an aeration rate of the oxygen-containing gas in the reduction step is less than 5 percent by volume per minute relative to a reaction mixture. The process 2 according to the present invention may also be a process in which an aeration rate of the oxygen-containing gas in the oxidation step is 1 percent by volume per minute or more relative to a reaction mixture, and substantially no aeration of the oxygen-containing gas is conducted in the reduction step.

The process 2 according to the present invention may be a process in which the reaction mixture in the oxidation step is controlled to have a pH of 7.0 to 9.5, and the reaction mixture in the reduction step is controlled to have a pH falling within a range of 6.0 to 8.5 and being lower than the pH of the reaction mixture in the oxidation step. In a more preferred embodiment, the process 2 may be a process in which the reaction mixture in the oxidation step is controlled to have a pH of 7.5 to 9.0, and the reaction mixture in the reduction step is controlled to have a pH falling within a range of 6.5 to 8.0.

In a preferred embodiment of the process 2 according to the present invention, the transformant according to the present invention or a treated product thereof may be supplemented to the reaction mixture before or after a switch from the oxidation step to the reduction step.

In the process 1 and process 2 according to the present invention, the pH control in a reaction for converting the D-form of an amino acid into a keto acid may be carried out using formic acid or a salt thereof.

In the process 1 and process 2 according to the present invention may use a reaction mixture containing 0.2 equivalent or more of formate ion and/or ammonium ion relative to the material enantiomeric mixture of the amino acid.

The "enzyme" in the present description may also be a mutant which has been varied in a genetic engineering manner by deletion, substitution, and/or addition of a part of amino acids constituting the enzyme, as long as it has a target enzymatic activity. The term "process according to the present invention" is used as generically meaning the process 1 and process 2 according to the present invention. Advantages

According to the present invention, a reaction for converting the D-form of an amino acid into a keto acid and a reaction for converting a keto acid into the L-form of an amino acid can be conducted in one pot (in a single system) using a single transformant that expresses specific enzymes and using their enzymatic activities. Thus, the target L-amino acid can efficiently accumulate in a high concentration, and the L-amino acid can be produced in a theoretical yield of 100% through a smaller number of steps using an inexpensive enantiomeric mixture of the amino acid as a starting material.

In particular, when the process includes an oxidation step of oxidizing the D-form of an amino acid to a keto acid, reactions can be conducted under mild conditions to thereby prevent the keto acid from decomposition, because a hydrogen peroxide catabolic enzyme is expressed in a single host. The target L-amino acid can therefore be obtained with satisfactory productivity.

## Brief Description of the Drawings

[Fig. 1] Fig. 1 is a restriction map of a plasmid pSE420U used in Preparation Example 1.
[Fig. 2] Fig. 2 is a restriction map of a plasmid pSUCBDO1 used in Preparation Example 1.
[Fig. 3] Fig. 3 is a restriction map of a plasmid pUCTVDOT used in Preparation Example 2.
[Fig. 4] Fig. 4 is a restriction map of a plasmid pSUTVDO1 used in Preparation Example 2.
[Fig. 5] Fig. 5 is a restriction map of a plasmid pUCECDD1 used in Preparation Example 3.
[Fig. 6] Fig. 6 is a restriction map of a plasmid pETECDD1 used in Preparation Example 3.
[Fig. 7] Fig. 7 is a restriction map of a plasmid pSU-MF26 used in Preparation Example 4.
[Fig. 8] Fig. 8 is a restriction map of a plasmid pSF-GSA2 used in Preparation Example 5.
[Fig. 9] Fig. 9 is a restriction map of a plasmid pSF-BTA1 used in Preparation Example 6.
[Fig. 10] Fig. 10 is a restriction map of a plasmid pSFBPAD1 used in Preparation Example 7.
[Fig. 11] Fig. 11 is a restriction map of a plasmid pSF-SAD1 used in Preparation Example 8.
[Fig. 12] Fig. 12 is a restriction map of a plasmid pEGSLED2 used in Preparation Example 9.
[Fig. 13] Fig. 13 is a restriction map of a plasmid pFGSLED1 used in Preparation Example 9.
[Fig. 14] Fig. 14 is a restriction map of a plasmid pSFBPLD1 used in Preparation Example 10.
[Fig. 15] Fig. 15 is a restriction map of a plasmid pSU-TIP2 used in Preparation Example 11.
[Fig. 16] Fig. 16 is a restriction map of a plasmid pSF-TIP2 used in Preparation Example 11.
[Fig. 17] Fig. 17 is a restriction map of a plasmid pSE-ECB1 used in Preparation Example 12.
[Fig. 18] Fig. 18 is a restriction map of a plasmid pSE-BSB1 used in Preparation Example 13.
[Fig. 19] Fig. 19 is a restriction map of a plasmid pSFTPCO1 constructed in Example 1.
[Fig. 20] Fig. 20 is a restriction map of a plasmid pSFGACO1 constructed in Example 2.

## Best Mode for Carrying Out the Invention

The L-amino acid production process 1 according to the present invention is a process for producing the L-form of an amino acid enzymatically from an enantiomeric mixture of the amino acid, which includes the step of contacting a transformant or a treated product thereof with an enantiomeric mixture of the amino acid, in which the transformant includes a single host and one or more recombinant vectors introduced into the single host, the one or more recombinant vectors include one or more expression vectors and nucleotide sequences, and the nucleotide sequences include a nucleotide sequence encoding an enzyme capable of converting the D-form of an amino acid into a keto acid and a nucleotide sequence encoding an enzyme capable of converting a keto acid into the L-form of an amino acid. The nucleotide sequences are integrated into the one expression vector or the different expression vectors, respectively. Specifically, according to this process, the target L-amino acid can be produced in a theoretical yield of 100%. This is because the process carries out a reaction for converting the D-form of an amino acid into a keto acid and a reaction for converting a keto acid into the L-form of an amino acid (hereinafter the two reactions together are also referred to as "main reaction(s)") in one pot (in a single system) by the actions of enzymes expressed in a single transformant, in which an enantiomeric mixture of the amino acid is used as a starting material.
The L-amino acid production process 1 according to the present invention includes a cultivation step and a reaction step, in which the cultivation step includes inducing the expression of the transformant having the above structure to yield a culture containing target converting enzymes, and the reaction step includes converting a D-form of an amino acid contained in an enantiomeric mixture of the amino acid into the L-form of the amino acid by enzymatic reactions using the culture.
The cultivation step is a step of inducing the expression of a transformant to yield a culture containing target converting enzymes, in which the transformant is composed of a single host and one or more recombinant vectors introduced thereinto, the one or more recombinant vectors are composed of one or more expression vectors and nucleotide sequences, and the nucleotide sequences include a nucleotide sequence encoding an enzyme capable of converting the D-form of an amino acid into a keto acid and a nucleotide sequence encoding an enzyme capable of converting a keto acid into the L-form of an amino acid. The nucleotide sequences are integrated into the one expression vector or the different expression vectors, respectively.
The enzyme capable of converting the D-form of an amino acid into a keto acid for use in the present invention is not specifically limited, as long as it is an enzyme capable of acting on the D-form of an amino acid to form a corresponding keto acid. Examples thereof include D-amino acid oxidases and D-amino acid dehydrogenases.
Such D-amino acid oxidase can be, for example, a D-amino acid oxidase, or a polypeptide which has an amino acid sequence corresponding to the amino acid sequence of the enzyme, except with a part of amino acids constituting the enzyme being deleted, substituted, and/or added, and is capable of expressing the D-amino acid oxidase.

Such D-amino acid oxidases are classified as, for example, EC 1.4.3.1, EC 1.4.3.3, EC 1.4.3.7, EC 1.4.3.15, and EC 1.4.3.19 and each have an enzymatic activity of oxidatively deaminating a D-amino acid with oxygen as an electron acceptor in the presence of water to a corresponding keto acid and hydrogen peroxide. There have also been found D-amino acid oxidases that oxidize an amino acid with oxygen as an electron acceptor to an imine but do not yield hydrogen peroxide. These enzymes are also included D-amino acid oxidases for use in the present invention.

The D-amino acid oxidases may be derived from organisms of every kind. Examples of usable D-amino acid oxidases include enzymes derived from microorganisms belonging typically to the genus Candida, the genus Trigonopsis, the genus Schizosaccharomyces, the genus Arthrobacter, the genus Corynebacterium, the genus Pseudomonas, and the genus Rhodotorula; and enzymes derived from the livers of mammals such as humans, mice, pigs, and rabbits (Biochemistry, 26, 3612 (1987)).

Specific examples of the enzymes derived from microorganisms include one derived from *Arthrobacter protophormiae* (EP 1375649), one derived from *Candida boidinii* (Yeast, 16, 1217 (2000)), one derived from *Corynebacterium glutamicum* (J. Biotechnol. 104, 5 (2003)), one derived from *Pseudomonas putida* (Environ. Microbiol. 4, 799 (2002)), one derived from *Rhodotorula gracilis* (J. Bacteriol., 58, 115 (1997)), one derived from *Trigonopsis variabilis* (Japanese Examined Patent Application Publication (JP-B) No. H07-108225), one derived from *Fusarium solani* (J. Biochem., 108, 1063 (1990)), and one derived from *Schizosaccharomyces pombe.*

Among them, preferred examples include D-amino acid oxidases derived from microorganisms belonging to the genus Candida, such as the *Candida boidinii* strain DSM 70026; and D-amino acid oxidases derived from microorganisms belonging to the genus Trigonopsis, such as *Trigonopsis variabilis* strain CBS 4095.

The D-amino acid dehydrogenase for use herein can be, for example, a D-amino acid dehydrogenase, or a polypeptide which has an amino acid sequence corresponding to the amino acid sequence of the enzyme, except with a part of amino acids constituting the enzyme being deleted, substituted, and/or added, and is capable of expressing the D-amino acid dehydrogenase.

Such D-amino acid dehydrogenases are classified as, for example, EC 1.4.99.1 and each have an enzymatic activity capable of oxidatively deaminating a D-amino acid typically with 2,6-dichloroindophenol (DCIP) as an electron acceptor to yield a corresponding keto acid.

The D-amino acid dehydrogenases may be derived from organisms of every kind. Examples thereof include enzymes derived from microorganisms belonging typically to the genus Escherichia, the genus Pseudomonas, the genus Salmonella, and the genus Flavobacterium.

Specific examples of the enzymes derived from microorganisms include one derived from *Escherichia coli* (J. Bacteriol., 176, 1500 (1994)), one derived from *Pseudomonas aeruginosa* (Curr. Microbiol. 41, 290 (2000)), one derived from *Salmonella typhimurium* (Nature, 413, 852 (2001)), and one derived from *Flavobacterium* sp. (J. Gen. Microbiol. 133, 745-754 (1987)).

Among them, D-amino acid dehydrogenases derived from microorganisms belonging to the genus Escherichia, such as *Escherichia coli* strain K-12, are preferably used.

The activity of an enzyme capable of converting the D-form of an amino acid into a keto acid for use in the present invention can be determined according to the following procedure. Specifically, the enzymatic activity of a D-amino acid oxidase is determined by a process of reacting a reaction mixture at 30°C, in which the reaction mixture contains 100 mM potassium phosphate buffer (pH 8.0), 10 mM of the D-amino acid, 0.5 mM 4-aminoantipyrine, 2 mM phenol, and 5 U/mL peroxidase, and the amount of the enzyme that catalyzes the production of 0.5 μmol of quinonimine per one minute is defined as one unit (1 U). The enzymatic activity of a D-amino acid dehydrogenase is determined by a process of reacting a reaction mixture at 30°C, in which the reaction mixture contains 100 mM Tris-HCl buffer (pH 8.0), 10 mM D-amino acid, and 0.1 mM 2,6-dichloroindophenol (DCIP) sodium salt, and the amount of the enzyme that catalyzes decrease of 1 μmol of DCIP per one minute is defined as 1 U.

The enzyme capable of converting a keto acid into the L-form of an amino acid for use in the present invention is not specifically limited as long as it is an enzyme capable of acting on a keto acid to yield a corresponding L-amino acid. Examples thereof include L-amino acid dehydrogenase, and polypeptides having L-amino acid transaminase activities.

The L-amino acid dehydrogenases each have an enzymatic activity capable of reductively aminating an α-keto acid in the presence of, for example, NAD(P)H and ammonia to yield a corresponding L-amino acid. Examples of such L-amino acid dehydrogenases include alanine dehydrogenases (EC 1.4.1.1), glutamate dehydrogenases (EC 1.4.1.2, EC 1.4.1.3, EC 1.4.1.4), L-amino acid dehydrogenases (EC 1.4.1.5), serine dehydrogenases (EC 1.4.1.7), valine dehydrogenases (EC 1.4.1.8), leucine dehydrogenases (EC 1.4.1.9), glycine dehydrogenases (EC 1.4.1.10), lysine dehydrogenases (EC 1.4.1.15), tryptophan dehydrogenases (EC 1.4.1.19), and phenylalanine dehydrogenases (EC 1.4.1.20).

The L-amino acid dehydrogenases may be derived from organisms of every kind. Examples of usable L-amino acid dehydrogenases include enzymes derived from microorganisms; and enzymes derived from mammals such as humans, mice, pigs, and cattle.

Examples of the alanine dehydrogenases (EC 1.4.1.1) include alanine dehydrogenases derived from organisms,

such as one derived from *Geobacillus stearothermophilus* (Biochemistry, 29, 1009 (1990)), one derived from *Bacillus thermocatenulatus* (Biochimie, 71, 559 (1989)), one derived from *Shewanella* sp. (Appl. Environ. Microbiol., 65, 4014 (1999)), and one derived from *Bacillus sphaericus,* such as *Bacillus sphaericus* IFO 3525 (Biochemistry, 29, 1009 (1990); Accession No. M33298).

The glutamate dehydrogenases (EC 1.4.1.2, EC 1.4.1.3, EC 1.4.1.4) include glutamate dehydrogenases derived from organisms, such as one derived from the bovine liver (J. Biochem. Mol. Biol., 36, 545 (2003), one derived from *Bacillus subtilis* (J. Bacteriol., 180, 6298 (1998)), one derived from *Sulfolobus solfataricus* (Biochemistry, 203, 81 (1992)), and one derived from *Saccharomyces cerevisiae* (Biochemistry, 217, 469 (1993)).

The L-amino acid dehydrogenases (EC 1.4.1.5) include L-amino acid dehydrogenases derived from organisms such as obligate anaerobes.

Examples of the serine dehydrogenases (EC 1.4.1.7) include enzymes derived from microorganisms, such as one derived from *Agrobacterium tumefaciens* (Accession No. AB032242).

Examples of the valine dehydrogenases (EC 1.4.1.8) include valine dehydrogenases derived from organisms, such as one derived from *Cytophaga* sp. (Accession No. AF339150).

Examples of the leucine dehydrogenases (EC 1.4.1.9) include leucine dehydrogenases derived from organisms, such as one derived from *Geobacillus stearothermophilus* (Biochemistry, 27, 9056 (1988)) and one derived from *Bacillus sphaericus.*

Examples of the glycine dehydrogenases (EC 1.4.1.10) include glycine dehydrogenases derived from organisms, such as one derived from *Escherichia coli* (Eur. J. Biochem. 216, 539 (1993)).

Examples of the lysine dehydrogenases (EC 1.4.1.15) include enzymes derived from organisms, such as one derived from *Geobacillus stearothermophilus* (Appl. Environ. Microbiol. 70, 937 (2004)).

Examples of the phenylalanine dehydrogenases (EC 1.4.1.20) include phenylalanine dehydrogenases derived from organisms, such as one derived from *Thermoactinomyces intermedius* (J. Biochem., 109, 371 (1991)), one derived from *Brevibacterium* sp. (JP-A No. S63-157986), one derived from *Sporosarcina urea* (JP-A No. S61-239887), one derived from *Bacillus sphaericus* (JP-A No. S63-157986), one derived from *Bacillus badius* (JP-A No. S63-157986), one derived from *Rhodococcus* sp. (JP-A No. S61-146183), one derived from *Nocardia* sp. (Arch. Microbiol., 153, 12 (1989)), and one derived from *Halomona pacifica* (JP-A No. 2002-65270).

Among them, preferred are L-amino acid dehydrogenases derived from microorganisms belonging to, for example, the genus Geobacillus, the genus Bacillus, the genus Shewanella, and the genus Thermoactinomyces. Examples of preferred L-amino acid dehydrogenases include a phenylalanine dehydrogenase derived from *Thermoactinomyces intermedius,* an alanine dehydrogenase derived from *Geobacillus stearothermophilus,* an alanine dehydrogenase derived from *Bacillus thermocatenulatus,* an alanine dehydrogenase derived from *Bacillus sphaericus,* an alanine dehydrogenase derived from *Shewanella* sp., a leucine dehydrogenase derived from *Geobacillus stearothermophilus,* and a leucine dehydrogenase derived from *Bacillus sphaericus.*

L-Amino acid transaminases each have an enzymatic activity of transferring amino group from an amino donor, such as L-glutamic acid or L-aspartic acid, to an $\alpha$-keto acid to yield a corresponding L-amino acid. Such L-amino acid transaminases include alanine transaminases (EC 2.6.1.2), cysteine transaminases (EC 2.6.1.3), glycine transaminases (EC 2.6.1.4), tyrosine transaminases (EC 2.6.1.5), leucine transaminases (EC 2.6.1.6), tryptophan transaminases (EC 2.6.1.27), histidine transaminases (EC 2.6.1.38), branched-chain-amino-acid transaminases (EC 2.6.1.42), and aromatic-L-amino-acid transaminases (EC 2.6.1.57).

Such L-amino acid transaminases may be derived from organisms of every kind. Examples usable L-amino acid transaminases include enzymes derived from microorganisms; and enzymes derived from mammals such as humans, rats, and mice.

Representative examples of L-amino acid transaminases include an alanine transaminase derived from *Oryza sativa* (Plant Mol. Biol. 39, 149 (1999)); a cysteine transaminase derived from a mammal such as a rat (Physiol. Chem. Phys., 10, 483 (1978)); a glycine transaminase derived from a mammal such as a human or a rat (J. Biol. Chem., 242, 3614 (1967)); a tyrosine transaminase derived from a mammal such as a rat (Anal. Biochem., 95, 188 (1979)); a leucine transaminase derived from a mammal such as a rat (Biochim. Biophys. Acta, 445, 622 (1976)); a tryptophan transaminase derived from *Streptomyces griseus* (J. Biol. Chem., 250, 7819 (1975)); and a histidine transaminase derived from *Pseudomonas testosteroni* (Biochem. J., 147, 327 (1975)).

Examples of the branched-chain-amino-acid transaminases include branched-chain-amino-acid transaminases derived from organisms, such as one derived from *Escherichia coli* (J. Biochem., 97, 993 (1985)) and one derived from *Bacillus subtilis* (J. Bacteriol., 179, 496 (1997)). Examples of the aromatic-amino-acid transaminases include aromatic-amino-acid transaminases derived from organisms, such as one derived from *Escherichia coli* (Biochem. Biophys. Res. Commun., 133, 134 (1985)) and one derived from *Paracoccus denitrificans* (JP-A No. H01-153084).

Among them, preferred are branched-chain-amino-acid transaminases, of which particularly preferred are amino acid transaminases derived from microorganisms, such as a branched-chain-amino-acid transaminase derived from a microorganism belonging to the genus Escherichia such as *Escherichia coli* strain K-12; and a branched-chain-amino-acid transaminase derived from a microorganism belonging to the genus Bacillus, such as *Bacillus subtilis.*

The activity of an enzyme capable of converting a keto acid into the L-form of an amino acid for use in the present invention can be determined, for example, according to the following processes. Specifically, the activity of an L-amino acid dehydrogenase can be determined by a process of reacting a reaction mixture at 30°C, in which the reaction mixture contains 200 mM glycine-potassium chloride-potassium hydroxide buffer (pH 10.5), 10 mM of an L-amino acid, and 2.5 mM NAD+, and the amount of the enzyme that yields 1 μmol of reduced-form nicotinamide adenine dinucleotide (NADH) per one minute is defined as one unit (1 U). The activity of an L-amino acid transaminase can be determined by a process of reacting a reaction mixture at 30°C, in which the reaction mixture contains 100 mM Tris-HC1 buffer (pH 8.0), 50 mM ammonium chloride, 10 mM sodium α-ketoisocaproate, 40 mM of an L-amino acid (amino donor), 0.05 mM pyridoxal-5'-phosphate, 0.2 mM reduced-form nicotinamide adenine dinucleotide phosphate (NADPH), and 5 U/mL glutamate dehydrogenase derived from *Proteus* species (TOYOBO CO., LTD.), and the amount of the enzyme that catalyzes decrease of 1 μmol of NADPH per one minute is defied as one unit (1 U).

In the present invention, a nucleotide sequence encoding another enzyme may be integrated into an expression vector, in which the expression vector may be the same as or different from the expression vector(s) containing target converting enzymes integrated thereinto, within ranges not adversely affecting the enzymatic activities. Examples of usable enzymes as the other enzymes include enzymes capable of decomposing a substance inhibiting a main reaction from proceeding, such as a hydrogen peroxide catabolic enzyme; and enzymes capable of regenerating a substrate to be consumed in a main reaction, such as a coenzyme-regenerating enzyme.

The hydrogen peroxide catabolic enzyme acts to efficiently proceed main reactions by its activity of decomposing and removing hydrogen peroxide, which hydrogen peroxide is by-produced in a reaction using a D-amino acid oxidase and inhibits main reactions from proceeding. The hydrogen peroxide catabolic enzyme can be, for example, a catalase or a polypeptide (catalase-like enzyme) which has an amino acid sequence corresponding to the amino acid sequence of the catalase, except with a part of amino acids constituting the catalase being deleted, substituted, and/or added, and is capable of expressing a hydrogen peroxide catabolic (decomposing) activity. Examples of usable catalases include known catalases derived typically from microorganisms, such as a catalase derived from *Escherichia coli*. Specific preferred examples of the catalase derived from *Escherichia coli* include katE (J. Biol. Chem., 254, 11664-11668 (1979)) and katG (J. Biol. Chem., 254, 4245-4252 (1979)).

The activity of decomposing hydrogen peroxide (catalase activity) can be determined, for example, by a process of reacting a reaction mixture at 30°C, in which the reaction mixture contains 50 mM potassium phosphate buffer (pH 7.0) and 0.035% hydrogen peroxide, and the amount of the enzyme that catalyzes decomposition of 1 μmol of hydrogen peroxide per one minute is defined as one unit (1 U).

The coenzyme-regenerating enzyme can proceed a main reaction by its activity of regenerating coenzyme NAD(P)H, which NAD(P)H is consumed with the proceeding of a reaction using a L-amino acid dehydrogenase. A transformant which is capable of regenerating coenzyme NAD(P)H for use in the present invention can be, for example, a transformant composed of a host having an activity of regenerating coenzyme NAD(P)H, and one or more recombinant vectors introduced thereinto, in which the one or more recombinant vectors have nucleotide sequences corresponding to target converting enzymes integrated thereinto; and/or a transformant composed of a single host and one or more recombinant vectors introduced thereinto, in which the one or more recombinant vectors are composed of one or more expression vectors and nucleotide sequences, and the nucleotide sequences include a nucleotide sequence encoding an enzyme capable of converting the D-form of an amino acid into a keto acid, a nucleotide sequence encoding an L-amino acid dehydrogenase, and a nucleotide sequence encoding a coenzyme-regenerating enzyme. The nucleotide sequences are integrated into the one expression vector or the different expression vectors, respectively.

The host having an activity of regenerating NAD(P)H can be a microorganism within a broad range, in which the coenzyme is regenerated typically by the action of a glycolytic pathway or a methylotrophic single-carbon (C1) utilization pathway of the microorganism.

The coenzyme-regenerating enzyme can be, for example, a coenzyme-regenerating enzyme or a polypeptide which has an amino acid sequence corresponding to the amino acid sequence of the coenzyme-regenerating enzyme, except with a part of amino acids constituting the enzyme being deleted, substituted, and/or added, and is capable of expressing an enzymatic activity of regenerating a coenzyme.

Examples of such coenzyme-regenerating enzymes include formate dehydrogenases, glucose dehydrogenases, alcohol dehydrogenases, amino acid dehydrogenases, and organic acid dehydrogenases such as malate dehydrogenases. The coenzyme-regenerating enzymes may be derived from organisms of every kind. Among them, preferred are a formate dehydrogenase derived from a microorganism belonging to the genus Mycobacterium; and glucose dehydrogenases derived from microorganisms belonging to the genus Bacillus and the genus Thermoplasma. Specific examples of usable coenzyme-regenerating enzymes include a formate dehydrogenase derived typically from *Mycobacterium vaccae* as a NADH regenerating enzyme; and glucose dehydrogenases such as one derived from *Bacillus subtilis,* one derived from *Thermoplasma acidphilum,* one derived from *Bacillus megaterium,* and one derived from *Bacillus cereus,* as NAD(P)H regenerating enzymes.

The activity of a coenzyme-regenerating enzyme can be determined, for example, in the following manner. The activity of a formate dehydrogenase, for example, can be determined by a process of reacting a reaction mixture at 30°C, in which the reaction mixture contains 100 mM potassium phosphate buffer (pH 7.0), 100 mM sodium formate, and 2.5 mM NAD+, and the amount of the enzyme that catalyzes the production of 1 μmol of NADH per one minute is defined as one unit (1 U).

The nucleotide sequence encoding an enzyme for use in the present invention can be any nucleotide sequence that encodes a polypeptide expressing a target enzymatic activity. Such nucleotide sequences include known nucleotide sequences, part or all of which are in public in DNA Data Bank of Japan (DDBJ); and nucleotide sequences which are determined as corresponding to amino acid sequences identified from purified enzymes. These nucleotide sequences can be prepared using known processes such as processes using polymerase chain reactions (PCR) and other amplification procedures; and chemical synthesis processes.

The expression vectors for use herein can be plasmid vectors and phage vectors. Such expression vectors can be selected as appropriate according to the type of a host, and the types and number of genes to be integrated. Each of these expression vectors can be used alone or in combination. Specific examples of expression vectors corresponding to a host will be mentioned later.

One or more recombinant vectors are used as recombinant vectors in the present invention, in which the one or more recombinant vectors are composed of one or more expression vectors and nucleotide sequences, and the nucleotide sequences include a nucleotide sequence encoding an enzyme capable of converting the D-form of an amino acid into a keto acid, and a nucleotide sequence encoding an enzyme capable of converting a keto acid into the L-form of an amino acid. The nucleotide sequences are integrated into the one expression vector or the different expression vectors, respectively. Among such recombinant vectors, preferred is a recombinant vector composed of a single expression vector and nucleotide sequences integrated thereinto, in which the nucleotide sequences include a nucleotide sequence encoding an enzyme capable of converting the D-form of an amino acid into a keto acid, and a nucleotide sequence encoding an enzyme capable of converting a keto acid into the L-form of an amino acid. In such recombinant vectors, a nucleotide sequence corresponding to another enzyme may further be integrated into an expression vector which is the same as or different from the expression vector(s) containing the above-mentioned nucleotide sequences integrated thereinto.

Representative examples of recombinant vectors for use in the present invention include: a two-gene-expressing recombinant vector, which is composed of a single expression vector and nucleotide sequences integrated thereinto, in which the nucleotide sequences include a nucleotide sequence corresponding to an enzyme capable of converting the D-form of an amino acid into a keto acid and a nucleotide sequence corresponding to an enzyme capable of converting a keto acid into the L-form of an amino acid; a multiple-gene-expressing recombinant vector, which is composed of a single expression vector and nucleotide sequences integrated thereinto, in which the nucleotide sequences include a nucleotide sequence corresponding to an enzyme capable of converting the D-form of an amino acid into a keto acid, a nucleotide sequence corresponding to an enzyme capable of converting a keto acid into the L-form of an amino acid, and a nucleotide sequence corresponding to another arbitrary enzyme; a combination of a first one-gene-expressing recombinant vector and a second one-gene-expressing recombinant vector, in which the first one-gene-expressing recombinant vector is composed of an expression vector and a nucleotide sequence corresponding to an enzyme capable of converting the D-form of an amino acid into a keto acid integrated thereinto, and the second one-gene-expressing recombinant vector is composed of another expression vector than that in the first vector and a nucleotide sequence corresponding to an enzyme capable of converting a keto acid into the L-form of an amino acid integrated thereinto; a combination of a multiple-gene-expressing recombinant vector and a one-gene-expressing recombinant vector, in which the multiple-gene-expressing recombinant vector is composed of a single expression vector and at least two or more nucleotide sequences integrated thereinto, the at least two or more nucleotide sequences are selected from a nucleotide sequence corresponding to an enzyme capable of converting the D-form of an amino acid into a keto acid, a nucleotide sequence corresponding to an enzyme capable of converting a keto acid into the L-form of an amino acid, and a nucleotide sequence corresponding to another arbitrary enzyme, and the one-gene-expressing recombinant vector is composed of another expression vector than the former and one nucleotide sequence integrated thereinto; and a combination of a first one-gene-expressing recombinant vector, a second one-gene-expressing recombinant vector, and a third recombinant vector, in which the first one-gene-expressing recombinant vector is composed of an expression vector and a nucleotide sequence corresponding to an enzyme capable of converting the D-form of an amino acid into a keto acid integrated thereinto, the second one-gene-expressing recombinant vector is composed of another expression vector than the former and a nucleotide sequence corresponding to an enzyme capable of converting a keto acid into the L-form of an amino acid integrated thereinto, and the third recombinant vector is composed of yet another expression vector than the former two and a nucleotide sequence corresponding to another arbitrary enzyme integrated thereinto.

Among them, a single recombinant vector expressing multiple genes is preferably used, for yielding a transformant without problems such as incompatibility and for better workability and handleability. Examples of recombinant vectors of this type include a recombinant vector composed of a single expression vector and nucleotide sequences

integrated thereinto, in which the nucleotide sequences include a nucleotide sequence encoding an enzyme capable of converting the D-form of an amino acid into a keto acid and a nucleotide sequence encoding an enzyme capable of converting a keto acid into the L-form of an amino acid; and a multiple-gene-expressing recombinant vector composed of a single expression vector and nucleotide sequences integrated thereinto, in which the nucleotide sequences include a nucleotide sequence corresponding to an enzyme capable of converting the D-form of an amino acid into a keto acid, a nucleotide sequence corresponding to an enzyme capable of converting a keto acid into the L-form of an amino acid, and a nucleotide sequence corresponding to another arbitrary enzyme.

When the enzyme capable of converting the D-form of an amino acid into a keto acid is a D-amino acid oxidase, preferred is a recombinant vector using a hydrogen peroxide catabolic enzyme, of which a catalase is preferred, as the "other arbitrary enzyme", or a combination of this recombinant vector and one or more other recombinant vectors. This configuration is for suppressing the decomposition of the keto acid by the action of by-produced hydrogen peroxide and reducing oxygen required for an oxidation reaction of the D-amino acid to thereby proceed main reactions smoothly. Specific examples of such recombinant vectors and combinations thereof include: a four-gene-expressing recombinant vector which is composed of a single expression vector and nucleotide sequences integrated thereinto, in which the nucleotide sequences include a nucleotide sequence corresponding to an enzyme capable of converting the D-form of an amino acid into a keto acid, a nucleotide sequence corresponding to an L-amino acid dehydrogenase, a nucleotide sequence corresponding to a hydrogen peroxide catabolic enzyme, and a nucleotide sequence corresponding to a coenzyme-regenerating enzyme; a three-gene-expressing recombinant vector which is composed of a single expression vector and nucleotide sequences integrated thereinto, in which the nucleotide sequences include a nucleotide sequence corresponding to an enzyme capable of converting the D-form of an amino acid into a keto acid, a nucleotide sequence corresponding to an L-amino acid dehydrogenase, and a nucleotide sequence corresponding to a hydrogen peroxide catabolic enzyme; and a combination of a first two-gene-expressing recombinant vector and a second two-gene-expressing recombinant vector, in which the first two-gene-expressing recombinant vector is composed of a single expression vector and nucleotide sequences integrated thereinto, the nucleotide sequences include a nucleotide sequence corresponding to an enzyme capable of converting the D-form of an amino acid into a keto acid and a nucleotide sequence corresponding to a hydrogen peroxide catabolic enzyme, and in which the second two-gene-expressing recombinant vector is composed of another expression vector than the first vector and nucleotide sequences integrated thereinto, and the nucleotide sequences include a nucleotide sequence corresponding to an L-amino acid dehydrogenase and a nucleotide sequence corresponding to a coenzyme-regenerating enzyme.

When the enzyme capable of converting a keto acid into the L-form of an amino acid is an L-amino acid dehydrogenase, preferred is a recombinant vector using a coenzyme-regenerating enzyme as the "other arbitrary enzyme", or a combination of the recombinant vector and one or more other vectors. This configuration proceeds main reactions smoothly. Specific examples of such recombinant vectors and combinations thereof include: a three-gene-expressing recombinant vector which is composed of a single expression vector and nucleotide sequences integrated thereinto, in which the nucleotide sequences include a nucleotide sequence corresponding to an enzyme capable of converting the D-form of an amino acid into a keto acid, a nucleotide sequence corresponding to an L-amino acid dehydrogenase, and a nucleotide sequence corresponding to a coenzyme-regenerating enzyme; a combination of a multiple-gene-expressing recombinant vector and a one-gene-expressing recombinant vector, in which the multiple-gene-expressing recombinant vector is composed of a single expression vector and nucleotide sequences integrated thereinto, the nucleotide sequences are at least two or more nucleotide sequences selected from a nucleotide sequence corresponding to an enzyme capable of converting the D-form of an amino acid into a keto acid, a nucleotide sequence corresponding to an L-amino acid dehydrogenase, and a nucleotide sequence corresponding to a coenzyme-regenerating enzyme, and the one-gene-expressing recombinant vector is composed of another single expression vector than above and one of these nucleotide sequences integrated thereinto; a combination of a first one-gene-expressing recombinant vector, a second one-gene-expressing recombinant vector, and a third recombinant vector, in which the first one-gene-expressing recombinant vector is composed of an expression vector and, integrated thereinto, a nucleotide sequence corresponding to an enzyme capable of converting the D-form of an amino acid into a keto acid, the second one-gene-expressing recombinant vector is composed of another expression vector and, integrated thereinto, a nucleotide sequence corresponding to an L-amino acid dehydrogenase, and the third recombinant vector is composed of yet another expression vector and, integrated thereinto, a nucleotide sequence corresponding to a coenzyme-regenerating enzyme.

In such a nucleotide sequence introduced into a host through a recombinant vector, an enzymatic activity thereof may be expressed as a result of direct transcription and translation from the recombinant vector, or the enzymatic activity may be expressed after the introduced nucleotide sequence is integrated into a chromosome of the host. The construction of such a recombinant vector is carried out by a process of integrating a nucleotide sequence corresponding to a target converting enzyme into an expression vector in an expressible manner. The process of integrating a nucleotide sequence corresponding to a target converting enzyme into an expression vector in an expressible manner used herein is generally a process of integrating a region involved in regulation of expression (expression regulation unit), such as

a promoter or a terminator, into the vicinity of a target nucleotide sequence. The process can be selected as appropriate according to the type of an organism used as the host. For example, when nucleotide sequences encoding two or more genes respectively are introduced into a single expression vector in an optional order, the process can be, for example, a process of connecting expression regulation units, such as promoters and/or terminators, to respective genes; or a process of expressing them as an operon containing two or more cistrons, such as lactose operon.

Examples of preferred structures of such recombinant vectors include a structure in which a promoter is arranged in a 5' upstream region of a target nucleotide sequence, and more preferably a structure in which a terminator is arranged in a 3' downstream region of the target nucleotide sequence, in addition to the promoter. Usable vectors, promoters, and terminators according to the type of an organism used as the host are described in detail, for example, in "Biseibutsugaku Kisokoza 8 Idensikougaku (in Japanese; Basic Miclobiology (8), Genetic Engineering), Kyoritsu Shuppan Co., Ltd.", of which yeasts are described in detail in Adv. Biochem. Eng. 43, 75-102 (1990), Yeast 8, 423-488 (1992). In addition, there have been developed systems of expressing a heteroprotein in a large amount in a host, in which the host is an insect such as silkworm (Nature 315, 592-594 (1985)), or another animal, or rapeseed, corn, potato, or another vegetable. These systems are preferably used herein. The construction of a recombinant vector according to the type of a host can be carried out according to a technique commonly used in the fields of molecular biology, biological engineering, and genetic engineering, as described, for example, by Sambrook et al. in Molecular Cloning, Cold Spring Harbor Laboratories.

Specific examples of structures of such recombinant vectors include a plasmid pSFTPC01 which is composed of a three-gene expressing vector pSE420U, which is a modification of a pSE420D (JP 2000-189170A), and, sequentially inserted thereinto, a gene encoding a D-amino acid oxidase derived from *Candida boidinii,* a formate dehydrogenase derived from *Mycobacterium vaccae,* and a gene encoding a phenylalanine dehydrogenase derived from *Thermoactinomyces intermedius;* and plasmids pSFGACO1, pSFTPCO2, and pSFTPCO3 having, inserted thereinto in any order, a gene encoding a D-amino acid oxidase derived from *Candida boidinii,* a formate dehydrogenase derived from *Mycobacterium vaccae,* and a gene encoding an alanine dehydrogenase derived from *Geobacillus stearothermophilus.* Examples of the recombinant vectors further include a recombinant vector pSCCBDO1 which coexpresses a gene encoding a D-amino acid oxidase derived from *Candida boidinii* and a gene encoding catalase derived from *Escherichia coli;* a recombinant vector pSF-TIP2 which coexpresses a gene encoding a formate dehydrogenase derived from *Mycobacterium vaccae* and a gene encoding a gene encoding phenylalanine dehydrogenase derived from *Thermoactinomyces intermedius;* and pSFCPCO1 which coexpresses four genes including a gene encoding a D-amino acid oxidase derived from *Candida boidinii,* a gene encoding a catalase derived from *Escherichia coli,* a gene encoding a phenylalanine dehydrogenase derived from *Thermoactinomyces intermedius,* and a gene encoding a formate dehydrogenase derived from *Mycobacterium vaccae.*

A host into which recombinant vectors are introduced is not specifically limited, as long as it is an organism into which one or more recombinant vectors containing nucleotide sequences corresponding to the target enzymes can be introduced and which can express the target enzymes. The host can be selected from among organisms of wide variety, such as microorganisms, vegetables (plants), and animals.

Microorganisms in which expression vector systems usable as a host have been developed can be used as microorganisms usable as a host. Examples thereof include bacteria such as those belonging to the genus Escherichia, the genus Bacillus, the genus Pseudomonas, the genus Serratia, the genus Brevibacterium, the genus Corynebacterium, the genus Streptococcus, and the genus Lactobacillus; mycobacteria in which host vector systems have been developed, such as those belonging to the genus Rhodococcus and the genus Streptomyces; yeasts in which host vector systems have been developed, such as those belonging to the genus Saccharomyces, the genus Kluyveromyces, the genus Schizosaccharomyces, the genus Zygosaccharomyces, the genus Yarrowia, the genus Trichosporon, the genus Rhodosporidium, the genus Pichia, and the genus Candida; microorganisms belonging to the genus Neurospora; and microorganisms belonging to the genus Aspergillus and the genus Cephalosporium.

Specific examples of combinations of expression vectors with expression regulation units (promoters and terminators) used according to the type of a host in the present invention will be illustrated below.

When the host is a microorganism belonging to the genus Escherichia, say, *Escherichia coli,* examples of the combinations include combinations each composed of: a pBR series or pUC series plasmid vector; a promoter derived from lac (β-galactosidase), trp (tryptophan operon), tac, trc (chimera of lac and trp), or PL or PR of lambda phase; and a terminator derived from trpA, a phage, or rrnB ribosomal RNA. Among them, preferably usable are a vector pSE420D (JP-A No. 2000-189170) in which a multicloning site of a commercially available pSE420 (Invitrogen) is partially modified; a vector pSE420U capable of coexpressing three genes; and a vector pSE420Q capable of coexpressing four genes.

When the host is a microorganism belonging to the genus Bacillus, plasmid vectors such as pUB110 series plasmids and pC194 series plasmids can be used. Such plasmid vectors can be integrated into a chromosome. Usable promoters and terminators include those derived from apr (alkaline protease), npr (neutral protease), and/or amy (α-amylase).

When the host is a microorganism belonging to the genus Pseudomonas, there have been developed host-vector systems typically for *Pseudomonas putida* and *Pseudomonas cepacia,* respectively, as a host. Available vectors include a broad-host vector pKT240 based on a plasmid TOL which participates in the decomposition of toluene compounds. Such a broad-host vector contains genes which are necessary for autologous replication and are derived typically from RSF1010. Available promoters and terminators include the lipase gene (JP-A No. H05-284973).

When the host is a microorganism belonging to the genus Brevibacterium, say, *Brevibacterium lactofermentum,* plasmid vectors such as pAJ43 (Gene 39, 281 (1985)) can be used. Promoters and terminators used in *Escherichia coli* can be used without any modification for Brevibacterium.

When the host is a microorganism belonging to the genus Corynebacterium, say, *Corynebacterium glutamicum,* plasmid vectors such as pCS11 (JP-A No. S57-183799) and pCB101 (Mol. Gen. Genet. 196, 175 (1984)) can be used.

When the host is a microorganism belonging to the genus Streptococcus, available plasmid vectors include pHV1301 (FEMS Microbiol. Lett. 26, 239 (1985) and pGK1 (Appl. Environ. Microbiol. 50, 94 (1985)).

When the host is a microorganism belonging to the genus Lactobacillus, pAMβ1 (J. Bacteriol. 137, 614 (1979)) which has been developed for a microorganism belonging to the genus Streptococcus can be used. The promoters exemplified in the genus Bacillus can also be used herein.

When the host is a microorganism belonging to the genus Rhodococcus, plasmid vectors isolated from *Rhodococcus rhodochrous* (J. Gen. Microbiol. 138, 1003 (1992)) are available.

When the host is a microorganism belonging to the genus Streptomyces, plasmids can be constructed according to the process described by Hopwood et al. in Genetic Manipulation of Streptomyces: A Laboratory Manual Cold Spring Harbor Laboratories (1985). In particular, when the host is *Streptomyces lividans,* pIJ486 (Mol. Gen. Genet. 203, 468-478 (1986)), pKC1064 (Gene 103, 97-99 (1991)), and pUWL-KS (Gene 165,149-150 (1995)) are usable. These plasmids can also be used for *Streptomyces virginiae* (Actinomycetol. 11, 46-53 (1997)).

When the host is a microorganism belonging to the genus Saccharomyces, say, *Saccharomyces cerevisiae,* YRp series, YEp series, YCp series, and YIp series plasmids are usable. Integration vectors (e.g., EP 537456) are very useful, because they are integrated into chromosome via homologous recombination with multicopy-ribosomal genes, allow to introduce a gene of interest in multicopy and the gene incorporated is stably maintained in the microorganism. Usable promoters and terminators include those derived from genes encoding, for example, ADH (alcohol dehydrogenase), GAPDH (glyceraldehyde-3-phosphate dehydrogenase), PHO (acidic phosphatase), GAL (β-galactosidase), PGK (phosphoglycerate kinase), and ENO (enolase).

When the host is a microorganism belonging to the genus Kluyveromyces, say, *Kluyveromyces lactis,* usable plasmids include 2 μm-series plasmids derived from *Saccharomyces cerevisiae;* pKD1 series plasmids derived from *Saccharomyces cerevisiae* (J. Bacteriol. 145, 382-390 (1981)); plasmids derived from pGKl1 involved in the killer activity; plasmids derived from autonomous replication gene KARS in a microorganism belonging to the genus Kluyveromyces; and vector plasmids that can be integrated into chromosome through homologous recombination typically with ribosomal DNA (e.g., EP 537456). Promoters and terminators derived typically from ADH and PGK can be used.

When the host is a microorganism belonging to the genus Schizosaccharomyces, usable are plasmid vectors containing ARS (autonomous replication sequence) derived from *Schizosaccharomyces pombe* and auxotrophy-complementing selectable markers derived from *Saccharomyces cerevisiae* (Mol. Cell. Biol. 6, 80 (1986)). Usable promoters include ADH promoters derived from *Schizosaccharomyces pombe* ( EMBO J. 6, 729 (1987)). Among them, pAUR224 is commercially available from Takara Shuzo Co., Ltd., so that it is easily used.

When the host is a microorganism belonging to the genus Zygosaccharomyces, usable are plasmid vectors derived typically from pSB3 derived from *Zygosaccharomyces rouxii* (Nucleic Acids Res. 13, 4267 (1985)). Usable promoters include PHO5 promoter derived from *Saccharomyces cerevisiae* and GAP-Zr (glyceraldehyde-3-phosphate dehydrogenase) promoter derived from *Zygosaccharomyces rouxii* (Agri. Biol. Chem. 54, 2521 (1990)).

When the host is a microorganism belonging to the genus Pichia, host-vector systems have been developed for *Pichia angusta* (formerly *Hansenula polymorpha).* Usable vectors include those derived from autonomous replication genes (HARS1, HARS2) derived from *Pichia angusta.* However, these are relatively unstable, and multicopy integration of these genes into chromosome is effective (Yeast 7, 431-443 (1991)). Promoters for AOX (alcohol oxidase) and FDH (formate dehydrogenase) genes induced typically with methanol can be used. Host-vector systems using the autonomous replication sequences (PARS1, PARS2) derived from Pichia have been developed typically for *Pichia pastoris* (Mol. Cell. Biol. 5, 3376 (1985)), and highly efficient promoters, such as AOX promoter inducible with methanol and high-cell-density culture, are usable (Nucleic Acids Res. 15, 3859 (1987)).

When the host is a microorganism belonging to the genus Candida, host-vector systems have been developed typically for *Candida maltosa, Candida albicans, Candida tropicalis,* and *Candida utilis.* An autonomous replication sequence (ARS) originating from *Candida maltosa* has been cloned (Agri. Biol. Chem., 51:1587, 1987), and a vector using the sequence has been developed for *Candida maltosa.* Further, a chromosome-integration vector with a highly efficient promoter unit has been developed for Candida utilis (JP-A No. H08-173170).

When the host is a microorganism belonging to the genus Aspergillus, microorganisms such as *Aspergillus niger* and *Aspergillus oryzae* have been most intensively investigated among fungi, and plasmid vectors and chromosome-integration vectors are usable, as well as promoters derived from extracellular protease gene and amylase gene are available (Trends in Biotechnology 7, 283-287 (1989)).

When the host is a microorganism belonging to the genus Trichoderma, host-vector systems using *Trichoderma reesei* have been developed, and promoters such as one derived from the extracellular cellulase gene are usable (Biotechnology 7, 596-603 (1989)).

In addition to the microorganisms, other organisms such as plants and animals can be used as a host. Usable vectors and promoters, for example, have been developed corresponding to a host. For example, there have been developed systems using insects such as silkworm (Nature 315, 592-594 (1985)) or plants such as rapeseed, corn, and potato, and these systems are advantageously used for expression of large amounts of heteroproteins.

A process for introducing a recombinant vector into a host can be a known or common procedure and process as selected according to the type of the host.

According to the above process, a transformant composed of a single host and one or more recombinant vectors capable of expressing target enzymes can be formed.

Transformants according to the present invention are constructed by introducing one or more of the above-exemplified recombinant vectors into a single host. Representative examples of the transformants according to the present invention include: a transformant which contains a single recombinant vector composed of a single expression vector and two nucleotide sequences integrated thereinto, in which the two nucleotide sequences are a nucleotide sequence encoding an enzyme capable of converting the D-form of an amino acid into a keto acid and a nucleotide sequence encoding an enzyme capable of converting a keto acid into the L-form of an amino acid; a single transformant which contains a single host and multiple recombinant vectors introduced thereinto, in which the multiple recombinant vectors are composed of the two nucleotide sequences integrated into different expression vectors; and a single transformant which is composed of a single host and one or more recombinant vectors and the other two or more recombinant vectors introduced thereinto, in which the one or more recombinant vectors are composed of a single expression vector and nucleotide sequences integrated thereinto, the nucleotide sequences are a nucleotide sequence encoding an enzyme capable of converting the D-form of an amino acid into a keto acid and a nucleotide sequence encoding an enzyme capable of converting a keto acid into the L-form of an amino acid, in combination with a nucleotide sequence encoding a coenzyme-regenerating enzyme and/or a nucleotide sequence encoding a hydrogen peroxide catabolic enzyme, and the other two or more recombinant vectors are composed of two or more different expression vectors and the three or four nucleotide sequences integrated thereinto. Among them, a transformant composed of a single recombinant vector introduced into a single host is preferred, for avoiding problems, such as incompatibility, occurring between two or more vectors.

Preferred transformants include transformants in which, in the above-mentioned structure, the enzyme capable of converting the D-form of an amino acid into a keto acid is a D-amino acid oxidase, the enzyme capable of converting a keto acid into the L-form of an amino acid is an L-amino acid dehydrogenase, the coenzyme-regenerating enzyme is a formate dehydrogenase, and the hydrogen peroxide catabolic enzyme is a catalase, of which more preferred are transformants in which the D-amino acid oxidase is one derived from *Candida boidinii,* the L-amino acid dehydrogenase is a phenylalanine dehydrogenase derived from *Thermoactinomyces intermedius,* the formate dehydrogenase is one derived from *Mycobacterium vaccae,* and the catalase is one derived from *Escherichia coli.*

Specific examples of preferred transformants for use in the present invention include a transformant which is capable of coexpressing at least an enzyme capable of oxidizing the D-form of an amino acid to a keto acid, a hydrogen peroxide catabolic enzyme, and an enzyme capable of converting a keto acid into the L-form of an amino acid. By using this transformant, the keto acid is inhibited from decomposition due to hydrogen peroxide and can be formed in a higher yield, which hydrogen peroxide has been by-produced in an oxidation reaction of the D-amino acid. According to another embodiment, the preferred transformant may be capable of further coexpressing a coenzyme-regenerating enzyme according to necessity. In this case, a reductive amination reaction of the keto acid can be efficiently proceeded by the action of the coenzyme-regenerating enzyme to yield a corresponding L-amino acid in a high yield.

According to the present invention, an enzyme capable of converting the D-form of an amino acid into a keto acid and an enzyme capable of converting a keto acid into the L-form of an amino acid can be coexpressed by cultivating a transformant having the above-mentioned structure alone. Consequently, the number of production steps can be reduced, because a reaction for converting the D-form of an amino acid into a keto acid and a reaction for converting the keto acid into an L-amino acid (main reactions) can be carried out in one pot (in a single system). In addition, the main reactions can efficiently proceed by the actions of enzymes accumulated in high concentrations, because a forced expression system using vectors is employed according to the present invention. Accordingly, the L-form of an amino acid can be produced in a theoretical yield of 100% using an inexpensive enantiomeric mixture of the amino acid as a starting material.

Media for use in cultivation of the transformant include liquid media and solid media each containing at least useful components necessary for the growth of a host, such as proteins, lipids, carbohydrates, vitamins, and minerals. Among them, liquid media are preferably used, because the resulting culture can be used without modification in enzymatic reactions. Such media may further contain additives of every kind.

The cultivation step yields a culture containing an enzyme capable of converting the D-form of an amino acid into a keto acid, and enzyme capable of converting a keto acid into the L-form of an amino acid, and, where necessary, one or more other enzymes accumulated in high concentrations.

In the reaction step, enzymatic reactions for converting the D-form of an amino acid contained in an enantiomeric mixture of the amino acid into the L-form of the amino acid proceed using a culture or a treated product thereof obtained in the cultivation step. Specifically, the reaction step is a step in which an enzymatic reaction of converting the D-form of an amino acid into a keto acid, and another enzymatic reaction of converting the formed keto acid into the L-form of the amino acid proceed in a single system using an enantiomeric mixture of the amino acid as a starting material, to thereby yield the L-form of the amino acid accumulated in a high concentration.

A reaction mixture in the reaction step at least contains a culture obtained in the cultivation step or a treated product thereof, and an enantiomeric mixture of the amino acid as a starting material.

The culture obtained in the cultivation step is composed of at least a transformant in which expressions are induced, and a medium. The culture can be used in the reaction step without any treatment, but it is preferred to remove the medium from the culture to thereby yield a transformant as a treated product of the culture, because a reaction mixture suitable for enzymatic reactions can be easily prepared using the resulting transformant. The treated product of the culture can be a treated product of the culture which has been subjected to a suitable treatment such as crushing, concentration, dilution, filtration, centrifugal separation, extraction, and/or immobilization within ranges where target enzymatic activities induced and expressed in the culture can be maintained. More specifically, the treated product of the culture used can be, for example, a microorganism having an increased membrane permeability by the action of a surfactant or an organic solvent, an immobilized microorganism, or a cell-free extract which is prepared by crushing cells typically using a homogenizer or by the application of ultrasound.

The "enantiomeric mixture of (the) amino acid" used as a starting material is a mixture of a pair of the D-form and L-form of an amino acid. Such mixtures include mixtures having the ratio of the D-form of an amino acid to the L-form of the amino acid of, for example, 10:90 to 90:10, preferably 25:75 to 75:25, and more preferably 50:50 (racemate).

Examples of amino acids constituting such enantiomeric mixtures include a compound represented by following Formula (1) :

[Chemical Formula 1]

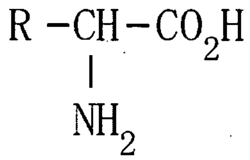

(1)

wherein R represents a hydrocarbon group which may have one or more substituents.

Examples of the hydrocarbon group in R include straight- or branched-chain hydrocarbon groups such as methyl, ethyl, propyl, propenyl, butyl, isobutyl, and butenyl; alicyclic hydrocarbon groups such as cyclopentane and cyclohexane; and aromatic hydrocarbon groups such as phenyl. Examples of substituents which the hydrocarbon group may have include halogen atoms, amino groups, nitro group, alkyl groups, and alkoxy groups.

Representative examples of amino acids for use in the present invention include norvaline, norleucine, homophenylalanine, o-nitrophenylalanine, m-nitrophenylalanine, p-nitrophenylalanine, 2-aminobutyric acid, and β-naphthylalanine. Among them, an enantiomeric mixture of norvaline is preferably used.

The amount of an enantiomeric mixture of the amino acid can be selected according typically to the types and amounts of enzymes to be expressed by the transformant. The amount is, for example, about 0.1 to about 50 percent by weight, preferably about 1 to about 30 percent by weight, and more preferably about 2 to about 20 percent by weight based on the total reaction mixture.

The reaction mixture may further contain one or more other components in addition to a culture or a treated product thereof, and an enantiomeric mixture of the amino acid. Examples of other components include reaction solvents, substrates and coenzymes corresponding to the enzymatic activities, and antifoaming agents. Examples of the reaction solvents include water; suitable organic solvents such as hydrocarbons, esters, and alcohols; and acidity or alkalinity adjusters such as pH adjusters and buffers. Each of these reaction solvents can be used alone or in combination. The

substrates and coenzymes corresponding to enzymatic activities can be suitably selected according to enzymes which the transformant expresses.

A coenzyme to be contained in the reaction mixture can be, for example, NAD(P)H for a transformant that expresses an amino acid dehydrogenase, and NAD(P)+ for a transformant that expresses a coenzyme-regenerating enzyme such as a formate dehydrogenase or a glucose dehydrogenase. These coenzymes, when added the reaction mixture, act to compensate or stabilize respective enzymes to thereby accelerate proceeding of main reactions. The coenzymes (NAD(P)H, NAD(P)+) can be added to the reaction mixture in a concentration of, for example, about 0.001 to about 100 mM, preferably about 0.01 to about 10 mM, and more preferably about 0.1 to about 1 mM, to the total amount of the reaction mixture.

When the transformant expresses an amino acid dehydrogenase and a coenzyme-regenerating enzyme, the system should further contain a substrate corresponding to the activity of the coenzyme-regenerating enzyme. Specific examples of combinations of a coenzyme-regenerating enzyme and a substrate thereof include a combination of formate dehydrogenase with formic acid or a salt thereof; a combination of glucose dehydrogenase with glucose; a combination of an alcohol dehydrogenase with ethanol or 2-propanol; a combination of malate dehydrogenase with malic acid or a salt thereof; a combination of glycerol dehydrogenase with glycerol. These can be added to the reaction system. The substrate can be added in an amount of, for example, about 0.1 to about 20 equivalents, and preferably about 1 to about 5 equivalents, to the D-form of an amino acid contained in an enantiomeric mixture of the amino acid used as a starting material.

When the transformant expresses an L-amino acid transaminase, an amino donor is added. The amino donor can be, for example, any of amino acids such as glutamic acid and aspartic acid, and salts thereof. The amino donor can be added in an amount of, for example, about 0.1 to 100 equivalents, and preferably about 1 to about 20 equivalents, to the D-form of an amino acid contained in the enantiomeric mixture used as a starting material.

When the transformant expresses an L-amino acid dehydrogenase, the system preferably further contain an amino donor such as ammonium ion. The ammonium ion may be contained in any form in the reaction mixture. For example, the ammonium ion may be contained as an ammonium buffer, or may be contained as ammonium formate. In the latter case, ammonium formate can also act to donate formic acid as a substrate for a formate dehydrogenase which is a coenzyme-regenerating enzyme. Alternatively, formic acid and ammonia may be added separately to the reaction mixture. The amount of ammonium ion is, for example, about 0.1 to about 20 equivalents, preferably about 0.2 to about 10 equivalents, and more preferably about 0.5 to about 4 equivalents, to the amount of the D-form of an amino acid in the system at the beginning of a reaction.

The antifoaming agents are effective for improving the properties of the reaction mixture, and examples thereof include ADEKA PLURONIC L-61, ADEKA PLURONIC L-71, ADEKA PLURONIC 25R-1, ADEKA PLURONIC 25R-2, ADEKANOL LG-294, COLORIN, and silicon. The amount of an antifoaming agent is, for example, about 0.001 to about 0.5 percent by weight, preferably about 0.005 to about 0.1 percent by weight, and more preferably about 0.01 to about 0.05 percent by weight, to the total amount of the reaction mixture.

In addition, any of buffers and compounds having buffering capabilities can be added for maintaining the pH of the reaction mixture. In particular, an ammonia buffer and/or ammonium formate is preferably used when an L-amino acid dehydrogenase is used as an enzyme capable of converting a keto acid into the L-form of an amino acid. These substances can be added in a concentration of, for example, about 10 mM to about 3 M, and preferably about 50 mM to about 1.5 M, to the total amount of the medium.

Enzymatic reactions are carried out in the presence of oxygen when the transformant expresses enzymes of some types. When the transformant is a transformant that expresses a D-amino acid oxidase, a reaction can be carried out while aerating, for example, pure oxygen, air whose oxygen partial pressure is increased, or the air as intact to the reaction mixture. In this case, the aeration rate is, for example, about 0.01 to about 10 volume per volume per minute (vvm), and preferably about 0.05 to about 2 vvm. The aeration procedure can be any procedure such as a procedure of contacting, for example, oxygen with the reaction mixture typically by stirring or shaking, and a procedure of pressurization. These conditions may be controlled depending on the dissolved oxygen level.

Reactions in the reaction step are carried out by mixing a culture or a treated product thereof and other components with an enantiomeric mixture of the amino acid as a starting material. The starting material, for example, may be added typically to a reaction solvent simultaneously, sequentially, continuously, or intermittently. The reactions are carried out statically or with stirring.

Reaction conditions are appropriately selected within ranges not adversely affecting the proceeding of enzymatic reactions. The pH of the reaction mixture at the beginning of reaction is selected within ranges where the target enzymatic activities can be maintained, and is generally about pH 5 to about pH 11, preferably about pH 6 to about pH 10, and more preferably about pH 7 to about pH 9. The pH during the reaction and after the completion of the reaction may be controlled within a suitable range or may be varied along with the proceeding of the reaction without control. Reaction temperatures can be selected within ranges where target enzymatic activities can be maintained, and are, for example, about 5°C to about 70°C, preferably about 10°C to about 55°C, and more preferably about 20°C to about 40°C.

In a preferred embodiment of the reaction step, for example, a reaction mixture is stirred under aeration conditions, in which the reaction mixture contains a transformant recovered from a culture obtained in the cultivation step, an enantiomeric mixture of the amino acid, substrates corresponding to the enzymes, and suitable additives.

According to the reaction step, enzymes expressed in the cultivation step are allowed to act upon the enantiomeric mixture of the amino acid, and thereby an enzymatic reaction of converting the D-form of an amino acid into a keto acid and another enzymatic reaction of converting the keto acid into the L-form of the amino acid proceed in one pot (in a single system). Thus, the L-form of the amino acid is formed.

The termination of reactions can be verified at the time when D-amino acid in the reaction mixture decreases in concentration and the corresponding L-amino acid accumulates in a high concentration. Specifically, a reaction termination point in the present invention is such that the concentration of the L-amino acid accumulated in the culture system is, for example, 90% e.e. (enantiomer excess) or more, preferably 95% e.e. or more, and more preferably 99% e.e. or more. The term "% e.e." as used herein refers to the value expressed by the following equation:

$$\text{e.e. (\%)} = \{(\text{Concentration of L-amino acid}) - (\text{Concentration of D-amino acid})\} \times 100/\{(\text{Concentration of L-amino acid}) + (\text{Concentration of D-amino acid})\}$$

The reactions yield a reaction mixture containing the L-amino acid accumulated in a high concentration. The L-amino acid can be recovered, for example, by solubilizing the L-amino acid accumulated in a concentration equal to or higher than its solubility in the reaction mixture, carrying out a treatment such as cell removal and/or protein removal (deproteinization), and purifying the treated mixture according to necessity.

The solubilization treatment of an L-amino acid can be carried out in the following manner. For example, when the target L-amino acid accumulates in a concentration equal to or higher than its solubility in a culture after liquid cultivation, the solubilization treatment can be conducted, for example, by a procedure such as acidification, basification, or dilution of the culture system. The acidification is carried out by adding an acid such as hydrochloric acid or sulfuric acid to the system. The basification is carried out by adding a base such as sodium hydroxide or potassium hydroxide to the system. The treated mixture after the solubilization treatment may be acidic, basic, or neutral. The separation procedure can be a known procedure such as filtration or centrifugal separation.

A solution of the L-amino acid, from which inorganic salts have been removed, can be obtained by adding an additive to the treated mixture after cell removal and/or protein removal to thereby precipitate unnecessary inorganic salts, and carrying out a separation procedure such as filtration. The additive used herein is not particularly limited, as long as the solubility of such unnecessary inorganic salts can be lowered while the solubility of the L-amino acid can be maintained. Among such additives, water-soluble organic solvents such as methanol, ethanol, 2-propanol, acetone, and acetonitrile are preferably used. These additives may be removed from the system by a procedure such as distillation, or the system containing such an additive as intact may be subjected to subsequent purification.

The purification is carried out by a known purification procedure such as concentration, isoelectric precipitation, crystallization through cooling or addition of an additive capable of lowering the solubility of the target, treatment with an ionic exchange resin, membrane separation, extraction with an organic solvent, chromatography such as ionic exchange chromatography, adsorption with an adsorbent, dehydration or flocculation with a flocculant or a dehydrator, or distillation, or any combination of these procedures. The purification procedure can be appropriately selected according to the type of the amino acid.

An example of a preferred process for separating and purifying the L-amino acid from the reaction mixture is a process in which a reaction mixture containing the L-form of an amino acid is concentrated, the pH thereof is adjusted to the vicinity of an isoelectric point, a water-soluble organic solvent as exemplified above is added to carry out crystallization, and precipitated, purified L-form of the amino acid is separated and recovered.

To separate and purify L-norvaline as an amino acid, for example, the following process can be used. Specifically, sulfuric acid is added to a reaction mixture containing L-norvaline to acidify the reaction mixture to thereby solubilize L-norvaline accumulated in the reaction mixture, from which cells are removed by centrifugal separation. The resulting solution containing L-norvaline is combined with methanol to precipitate unnecessary inorganic salts, and these are removed by filtration. The filtrate is recovered, neutralized with a base such as ammonia or sodium hydroxide, combined with acetone for crystallization, and thereby yields purified norvaline.

By the process 1 according to the present invention, an enzymatic reaction of converting the D-form of an amino acid into a keto acid, and another enzymatic reaction of converting the formed keto acid into the L-form of the amino acid proceed in one pot (in a single system). Thus, the enzymatic reaction of forming a keto acid from the D-form

of an amino acid and the enzymatic reaction of forming the L-form of the amino acid from the keto acid can be carried out as a cultivation step using a single transformant, and the target L-amino acid can be obtained as accumulated in a high concentration. Accordingly, the target L-amino acid can be produced in a theoretical yield of 100% even when an inexpensive mixture of D-form and L-form of the amino acid, such as a racemate, is used as a starting material.

The L-amino acid production process 2 according to the present invention is a process for producing the L-form of an amino acid enzymatically from an enantiomeric mixture of the amino acid. The process includes two different reaction steps including an oxidation step and a reduction step. The oxidation step mainly includes oxidizing the D-form of an amino acid using a transformant or a treated product thereof, in which the transformant coexpresses at least an enzyme capable of oxidizing the D-form of an amino acid to a keto acid and a hydrogen peroxide catabolic enzyme. The reduction step mainly includes synthetically producing the L-form of an amino acid from a keto acid using a transformant or a treated product thereof, in which the transformant expresses an enzyme capable of producing the L-form of an amino acid from a keto acid and optionally coexpresses a coenzyme-regenerating enzyme according to necessity.

The term "oxidation step" means a step which mainly contains an oxidation reaction of oxidizing the D-form of an amino acid to a keto acid. In this step, one or more other reactions may proceed in addition to the oxidation reaction. Examples of other reactions in the oxidation step include a reaction for accelerating the oxidation reaction (e.g., a reaction by the catalysis of a hydrogen peroxide catabolic enzyme), and a reduction reaction as mentioned below.

The term "reduction step" means a step which mainly contains a reduction reaction of synthetically producing the L-form of an amino acid by a reductive amination of a keto acid. In the reduction step, one or more other reactions may proceed in addition to the reduction reaction. Examples of other reactions in the reduction step include a reaction for accelerating the reduction reaction (e.g., a reaction by the action of a coenzyme-regenerating enzyme), and the above-mentioned oxidation reaction. The term "transformant (which) expresses an enzyme capable of producing the L-form of an amino acid from a keto acid and optionally coexpresses a coenzyme-regenerating enzyme" in the reduction step means and includes a transformant which expresses an enzyme capable of producing the L-form of an amino acid from a keto acid; and a transformant which coexpresses an enzyme capable of producing the L-form of an amino acid from a keto acid and a coenzyme-regenerating enzyme.

The L-amino acid production process 2 according to the present invention has only to include at least two different reaction steps composed of the oxidation step and the reduction step and may further include any other step such as a purification step.

After investigations, the present inventors have found that an oxidation reaction using an enzyme capable of oxidizing the D-form of an amino acid to a keto acid tends to have a decreased yield of a reaction product (keto acid) [the ratio of the L-form of the amino acid to the starting material enantiomeric mixture of the amino acid (DL-amino acids)] when the substrate concentration in the reaction mixture is high. For example, when the oxidation reaction is carried out under reaction conditions at a substrate concentration of more than 5 percent by weight, the yield is at most about 70%. Accordingly, such oxidation reactions are unsuitable for commercial production. The yield is lowered probably because a keto acid formed by the action of the D-amino acid oxidase is decomposed through a series of reactions, in which the keto acid comes in contact with hydrogen peroxide by-produced in this reaction, undergoes decarboxylation to yield an aldehyde, and the aldehyde further undergoes enzymatic oxidation or air oxidation to yield an organic acid; and as a result, main reactions are inhibited from proceeding.

To solve this problem, the present inventors have found that the yield of an L-amino acid can be markedly increased by using a transformant which coexpresses a D-amino acid oxidase and a hydrogen peroxide catabolic enzyme, in particular, by a catalase. They have also found that, in contrast to this, substantially no effect is obtained when a catalase is added to a reaction mixture containing a transformant which expresses a D-amino acid oxidase alone. This indicates not that an $\alpha$-keto acid leaked extracellularly is gradually decomposed by hydrogen peroxide also leaked extracellularly but that an $\alpha$-keto acid formed within cells is immediately decomposed within the cells by the action of a hydrogen peroxide which is concurrently by-produced. Specifically, for highly effectively inhibiting the $\alpha$-keto acid from decomposing, it is especially important to express a D-amino acid oxidase and a hydrogen peroxide catabolic enzyme, in particular, by a catalase in a single cell (in a single host) rather than to add a hydrogen peroxide catabolic enzyme to a reaction mixture.

Oxidation reactions of D-amino acids by the action of D-amino acid oxidases often require conditions of high aeration rate and high stirring rate, because oxygen aeration generally limits the reaction rates. These conditions lead to increased load on facilities in industrial-scale fermenters, induce microbial lysis and foaming of the reaction mixture, and thereby tend to show decreased yields. The present inventors have also found that the combination use of a hydrogen peroxide catabolic enzyme such as a catalase exhibits a secondary advantage of solving the above problems. This will be illustrated in detail below.

When a D-amino acid oxidase alone is used in an oxidation step of oxidizing the D-form of an amino acid to yield a keto acid, for example, a reaction represented by following Reaction Formula (1) proceeds. With reference to Reaction Formula (1), one molecule of oxygen is necessary for oxidizing one molecule of the D-form of the amino acid, and one molecule of hydrogen peroxide is by-produced simultaneously. In contrast, when a D-amino acid oxidase and

a hydrogen peroxide catabolic enzyme are used in combination, a reaction represented by following Reaction Formula (2) proceeds. With reference to Reaction Formula (2), one molecule of hydrogen peroxide is decomposed by the action of the hydrogen peroxide catabolic enzyme to yield a half molecule of oxygen, and this is supplied to the reaction system. Thus, the amount of oxygen to be introduced from outside of the reaction system can be reduced in half as a whole. This yields advantageous effects in that aeration and stirring in the oxidation step can be carried out under milder conditions and that the reaction time can be shortened.

(1) D-Amino acid + $O_2$ + $H_2O$ → Keto acid + Hydrogen peroxide + Ammonia (2) D-Amino acid + 1/2$O_2$ → Keto acid + Ammonia

It was also expected that the peroxidase activity of a hydrogen peroxide catabolic enzyme such as a catalase, if added, may accelerate the decomposition of a keto acid as an intermediate. However, when a catalase was actually used in combination, the yield of an L-amino acid could be markedly effectively improved. As has been described, the process 2 according to the present invention is very advantageous for production in high yields, because the combination use of a catalase much effectively inhibits the decomposition of a keto acid whereas hydrogen peroxide is decomposed with substantially no adverse effect caused by peroxidase activity (acceleration of the decomposition of the keto acid).

The process 2 according to the present invention has the above-mentioned configuration and can thereby produce a keto acid in a high yield under mild reaction conditions. Enzymes, recombinant vectors, transformants, culture conditions, and other factors can be as with the process 1. The process 2 according to the present invention can employ reaction conditions stated in the process 1.

In the process 2 according to the present invention, an oxidation step and a reduction step are preferably carried out under different reaction conditions. The oxidation step is mainly composed of an oxidation reaction of a D-amino acid which requires oxygen, and the reduction step is mainly composed of a reductive amination reaction of a keto acid which does not require oxygen. Accordingly, a reaction of forming an L-amino acid can be accelerated and the L-amino acid can be produced in a higher yield by setting conditions suitable for the respective steps.

After further investigations, the present inventors have found that a reduction reaction of a keto acid in the reduction step is inhibited from proceeding if it is carried out under high aeration conditions which are set for accelerating an oxidation reaction of the D-form of an amino acid in the oxidation step. This is because a substrate and a coenzyme necessary for the reduction reaction are oxidized under such conditions. They have also found that main causes of this are the following (1) and (2). Specifically, (1) a reduced-form coenzyme, such as NADH, should be primarily effectively used in a reductive amination reaction but is likely to be oxidized and wasted if high aeration conditions are employed in the reduction step, and, in addition, (2) a substrate for a coenzyme-regenerating enzyme (e.g., formic acid as a substrate for a formate dehydrogenase) is wasted, because there proceeds a reaction of regenerating an oxidized-form, such as NAD, of the coenzyme to form a reduced-form thereof, such as NADH, by the action of the coenzyme-regenerating enzyme present in the reaction system. This means that the reductive amination reaction of a keto acid in the reduction step is rather preferably carried out in the absence of oxygen. According to the present invention, the reduction step and the oxidation step are carried out in one pot (in a single system). By conducting these steps under different reaction conditions, the reductive amination reaction of an α-keto acid is accelerated, and the yield of a final target L-amino acid can be further effectively improved. Such reaction conditions to be set differently between the oxidation step and the reduction step include oxygen aeration conditions, stirring conditions, pH, and the addition of catalysts (including enzymes) and the transformant.

In a preferred embodiment, the process 2 according to the present invention is, for example, a process in which the oxidation step is carried out under aeration of an oxygen-containing gas, and the reduction step is carried out without aeration of an oxygen-containing gas or under aeration of an oxygen-containing gas at an aeration rate lower than that in the oxidation step. According to this process, the efficiency of producing the L-form of an amino acid from a keto acid can further be improved, because the above-mentioned problems can be avoided. Specifically, the problems are that a reduced-form coenzyme is oxidized and wasted in the reduction step and that a reductive amination reaction is inhibited from proceeding because the oxidized coenzyme is reduced again by the action of a coenzyme-regenerating enzyme, and thereby a substrate for the enzyme is wasted.

The oxygen-containing gas can be any gas that contains at least oxygen. Examples thereof include oxygen gas, and mixtures of oxygen and one or more other gases, such as the air. The other gases include inert gases such as carbon dioxide and nitrogen. The air, for example, is preferably used as the oxygen-containing gas. The content of oxygen in the oxygen-containing gas is, for example, about 0.1 percent by volume or more (0.1 to 100 percent by volume), preferably about 1 percent by volume or more, and more preferably about 5 percent by volume or more.

Specific examples of aeration conditions of an oxygen-containing gas are such that an aeration rate of the oxygen-containing gas in the oxidation step is 1 percent by volume per minute or more relative to the reaction mixture, and an aeration rate of the oxygen-containing gas in the reduction step is less than 10 percent by volume per minute relative to the reaction mixture; preferably such that an aeration rate of the oxygen-containing gas in the oxidation step

is 2 percent by volume per minute or more relative to the reaction mixture and an aeration rate of the oxygen-containing gas in the reduction step is less than 2 percent by volume per minute relative to the reaction mixture; more preferably such that an aeration rate of the oxygen-containing gas in the oxidation step is 5 percent by volume per minute or more relative to the reaction mixture and an aeration rate of the oxygen-containing gas in the reduction step is less than 5 percent by volume per minute relative to the reaction mixture; and especially preferably such that an aeration rate of the oxygen-containing gas in the oxidation step is 1 percent by volume per minute or more relative to the reaction mixture, and substantially no aeration of an oxygen-containing gas is conducted in the reduction step. When the steps are carried out under these conditions, the yield of the target L-amino acid can be further effectively improved.

Preferred embodiments of the process 2 according to the present invention further include, in addition to the above-mentioned processes, a process in which a reaction mixture in the reduction step is controlled to have a pH different from that of a reaction mixture in the oxidation step. For example, the reaction mixture in the reduction step is controlled to have a pH lower than that of a reaction mixture in the oxidation step. Specific pH conditions include such that the reaction mixture in the oxidation step is controlled to have a pH of 7.0 to 9.5, and the reaction mixture in the reduction step is controlled to have a pH falling within a range of 6.0 to 8.5 and being lower than the pH of the reaction mixture in the oxidation step; and such that the reaction mixture in the oxidation step is controlled to have a pH of 7.5 to 9.0, and the reaction mixture in the reduction step is controlled to have a pH falling within a range of 6.5 to 8.0 and being lower than the pH of the reaction mixture in the oxidation step.

The pH controls in the oxidation step and the reduction step are not particularly limited, within ranges not adversely affecting the proceeding of main reactions, and a known acid or base can be used in a suitable amount according to the type of the acid or base. In the process 1 and process 2 according to the present invention, formic acid or a salt thereof (e.g., ammonium formate) is preferably used for pH control in the reaction of converting the D-form of an amino acid into a keto acid, because formic acid which has been wasted in the oxidation step can be efficiently supplemented.

The process according to the present invention is preferably a process using a reaction mixture containing 0.2 equivalent or more of formate ion and/or ammonium ion relative to the starting material enantiomeric mixture of the amino acid. As the formate ion, a compound capable of forming formate ion in the reaction system can be used. Examples of such compounds usable herein include formic acid; and salts of formic acid such as ammonium formate and sodium formate. As the ammonium ion, a compound capable of forming ammonium ion in the reaction system can be used. Examples of such compounds usable herein include ammonia; and ammonium salts such as ammonium chloride, ammonium formate, and ammonium acetate.

According to the above-mentioned process, a keto acid can be obtained in a good yield, because the formate ion acts as a pH adjuster in the reaction of forming the keto acid from the D-form of an amino acid. In particular, when the process includes a reaction of converting a keto acid into the L-form of an amino acid using a formate dehydrogenase as a coenzyme-regenerating enzyme, the L-form of the amino acid can be obtained in a high yield, because the formate ion acts as a source for supplying a substrate for the formate dehydrogenase, the formate dehydrogenase thereby efficiently regenerates the coenzyme, and the reductive amination reaction of the keto acid is accelerated. Accordingly, the amount of a residual keto acid in the reaction mixture after the completion of the reaction can further be reduced. The above-mentioned process exhibits higher advantages when it includes a reaction using a D-amino acid oxidase.

According to the above process, the L-form of an amino acid can be further efficiently produced, because the ammonia ion acts as a pH adjuster and the ammonium ion acts as a source for supplying amino group to accelerate a reductive amination reaction of a keto acid in a reaction of converting the keto acid into the L-form of the amino acid. In addition, the process is further more effective when an L-amino acid dehydrogenase is used.

The present inventors have found that the formation of a keto acid is accelerated by adding 0.2 equivalent or more of ammonium formate to the starting material enantiomeric mixture of an amino acid (D,L-amino acids). Such ammonium formate in itself is considered not to affect a reaction of converting a D-amino acid into the keto acid (in particular, an oxidation reaction). Specifically, the advantages of processes according to the present invention can further be increased by using a reaction mixture containing both 0.2 equivalent or more of formate ion and 0.2 equivalent or more of ammonium ion relative to the starting material enantiomeric mixture of the amino acid. In particular, the efficiency of a reaction of converting a keto acid into the L-form of an amino acid using a formate enzyme dehydrogenase, if employed in the processes, can be markedly improved. This is because the formate ion and the ammonium ion both act as pH adjusters, and in addition, the former acts as a substrate for the formate enzyme dehydrogenase to efficiently regenerate a coenzyme, and the latter acts as a source for supplying amino group.

In a preferred embodiment of the process 2 according to the present invention, the transformant according to the present invention or a treated product thereof is supplemented to a reaction mixture before or after a switch from the oxidation step to the reduction step, for improving the reaction yield. According to this process, the target L-amino acid can be obtained in a high yield, because enzymes consumed in the oxidation step are supplemented, and a reductive amination reaction smoothly proceeds in the reduction step, and thereby the keto acid as an intermediate is prevented from remaining after the completion of the reaction.

In the process 2 according to the present invention, a D-amino acid oxidase and a hydrogen peroxide catabolic enzyme are coexpressed; and an enzymatic reaction of converting the D-form of an amino acid into a keto acid using the D-amino acid oxidase and an enzymatic reaction of converting the keto acid into an L-amino acid proceed in one pot (in a single system). Thus, the oxidation step of the D-amino acid can be conducted under mild conditions, the formed keto acid can accumulate in a high concentration whereas the decomposition of the keto acid is prevented; and the target L-amino acid can be efficiently obtained in a high yield in the subsequent reduction step. In particular, the reaction of converting the keto acid into the L-amino acid can be accelerated by conducing the reduction step under conditions different from those in the oxidation step. Accordingly, the intermediate keto acid is unlikely to remain in the reaction mixture after the completion of the reaction, and the L-amino acid can be obtained in a further higher yield. The L-amino acid can therefore be produced in a theoretical yield of 100% even when an inexpensive mixture of D,L-amino acids, such as a racemate, is used as a starting material.

EXAMPLES

The present invention will be illustrated in further detail with reference to several examples below. It should be noted, however, that these examples are never construed to limit the scope of the present invention. The "LB medium" used herein was a medium having a composition of 1% Bactotriptone, 0.5% Bactoyeast extract, and 1% sodium chloride (pH 7.2). The "LB medium containing ampicillin" was a medium containing the LB medium of the above composition and further containing ampicillin in a concentration of 50 μg/mL. In Tables 1 to 5, the term "Nva" represents the "concentration of a substrate for norvaline (percent by weight)", the term "Nva residual rate [%]" represents a value represented by the formula: "(molarity of norvaline in the reaction mixture)/(molarity of norvaline upon the preparation of the reaction mixture) x 100"; and the term "Nva formation rate [%]" represents a value represented by the formula: "(molarity of norvaline in the reaction mixture)/(molarity of 2-oxopentanoic acid upon the preparation of the reaction mixture) x 100".

Measurement of Enzymatic Activity

*Escherichia coli* strain HB101 was transformed with a plasmid prepared in any one of Preparation Examples and Examples, the transformant was cultivated in a liquid LB medium containing ampicillin (50 μg/ml) at 30°C overnight, and 0.1 mM isopropyl-β-D-thiogalactopyranoside (IPTG) was added to induce gene expression, followed by cultivation at 30°C for further four hours. Cells were collected, suspended in a 50 mM potassium phosphate buffer (pH 8.0) containing 0.02% 2-mercaptoethanol, crushed in the Closed System Ultrasonic Cell Disrupter UCD-200TM (COSMO BIO Co., Ltd.), subjected to centrifugal separation, and thereby yielded a series of supernatants (cell-free extracts).
Enzymatic activities were measured according to the following methods using the cell-free extracts, respectively.

(Measurement of D-Alanine Oxidase Activity)

D-Alanine oxidation activities were measured by holding reaction mixtures at 30°C to proceed reactions, in which the reaction mixtures contained the cell-free extracts, 100 mM potassium phosphate buffer (pH 8.0), 10 mM D-alanine, 0.5 mM 4-aminoantipyrine, 2 mM phenol, and 5 U/mL peroxidase. One unit (1 U) herein was defined as the amount of the enzyme that catalyzes the formation of 0.5 μmol of quinonimine per one minute.

(Measurement of D-Amino Acid Dehydrogenase Activity)

D-Alanine oxidation activities were measured by holding reaction mixtures at 30°C to proceed reactions, in which the reaction mixtures contained the cell-free extracts, 100 mM Tris-HCl buffer (pH 8.0), 10 mM D-alanine, and 0.1 mM 2,6-dichloroindophenol sodium salt (DCIP). One unit (1 U) herein was defined as the amount of the enzyme that catalyses decrease of 1 μmol of DCIP per one minute.

(Measurement of L-Amino Acid Dehydrogenase Activity)

L-Amino acid dehydrogenase activities were measured by holding reaction mixtures at 30°C to proceed reactions, in which the reaction mixtures contained the cell-free extracts, 200 mM glycine-potassium chloride-potassium hydroxide buffer (pH 10.5), 10 mM L-amino acid, and 2.5 mM NAD+. One unit (1 U) herein was defined as the amount of the enzyme that catalyses the formation of 1 μmol of NADH per one minute. As the L-amino acid, there were used L-alanine for L-alanine dehydrogenase activities, L-leucine for L-leucine dehydrogenase activities, and L-phenylalanine for L-phenylalanine dehydrogenase activities, respectively.

(Measurement of Formate Dehydrogenase Activity)

Formic acid oxidation activities were measured by holding reaction mixtures at 30°C to proceed reactions, in which the reaction mixtures contained the cell-free extracts, 100 mM potassium phosphate buffer (pH 7.0), 100 mM sodium formate, and 2.5 mM NAD+. One unit (1 U) herein was defined as the amount of the enzyme that catalyses the formation of 1 μmol of NADH per one minute.

(Measurement of L-Amino Acid Transaminase Activity)

L-Amino acid transaminase activities were measured by holding reaction mixtures at 30°C to proceed reactions, in which the reaction mixtures contained the cell-free extracts, 100 mM Tris-HCl buffer (pH 8.0), 50 mM ammonium chloride, 10 mM sodium α-ketoisocaproate, 40 mM sodium L-glutamate, 0.05 mM pyridoxal-5'-phosphate, 0.2 mM NADPH, and 5 U/mL glutamate dehydrogenase derived from *Proteus* species (Toyobo). One unit (1 U) herein was defined as the amount of the enzyme that catalyses decrease of 1 μmol of NADPH per one minute.

(Measurement of Catalase Activity)

Catalase activities were measured by holding reaction mixtures at 30°C to proceed reactions, in which the reaction mixtures contained the cell-free extracts, 50 mM potassium phosphate buffer (pH 7.0), and 0.035% hydrogen peroxide. One unit (1 U) herein was defined as the amount of the enzyme that catalyzes decomposition of 1 μmol of hydrogen peroxide per one minute.

PREPARATION EXAMPLE 1

Construction of Plasmid pSUCBDO1 Containing D-Amino Acid Oxidase Gene

A vector pSE420-N, whose Nde I restriction enzyme site had been collapsed, was constructed by sequentially subjecting pSE420D (JP-A No. 2000-189170) to digestion with Nde I, blunting with the Klenow fragment, and self-ligation. Positions 260-263 CTAG of pSE420-N was replaced with AGCT through site-specific mutation to construct pSE420S having a Sac I site at this site. The pSE420S was digested simultaneously with Cla I and Nco I, annealed with synthetic DNAs (SEQ ID NO: 1 and SEQ ID NO: 2) at this site, and ligated to construct a plasmid pSE420U (Fig. 1).

SEQ ID NO: 1:

CGATTAACTTTATTATTAAAAATTAAAGAGGTATATACATATGAAATATTTAATTAAGGA

GGAATAATC

SEQ ID NO: 2:

CATGGATTATTCCTCCTTAATTAAATATTTCATATGTATATACCTCTTTAATTTTTAATA

ATAAAGTTAAT

Cells were prepared by cultivating *Candida boidinii* strain DSM 70026 in a YEPD medium (2% Bactopeptone, 1% Bactoyeast extract, and 2% galactose, pH 6.0), from which chromosomal DNA was separated and purified according to the process as described in Meth. Cell Biol., 29, 39 (1975).

A sense primer CbDAO-A1 (SEQ ID NO: 3) and an antisense primer CbDAO-T1 (SEQ ID NO: 4) were synthesized for cloning based on the nucleotide sequence (Accession No. AB042032) of a D-amino acid oxidase gene derived from *Candida boidinii* strain TK 62 as described in Yeast, 16, 1217 (2000).

SEQ ID NO: 3: CACCATATGGGTGATCAAATTGTTGTTCTTGGTTC
SEQ ID NO: 4: CGACTTAAGTCTAGATTAAAGTTTAGCTTTAACTTTTTGGTTATCAACTAAAAG

A reaction mixture (50 μl) was subjected to a polymerase chain reaction (PCR) using the GeneAmp PCR

System 2400 (The Perkin-Elmer Corporation), in which the PCR was composed of 30 cycles of denaturation at 95°C for 30 seconds, annealing at 52°C for 30 seconds, and elongation at 72°C for 70 seconds, and the reaction mixture contained 50 ng of the chromosomal DNA purified from *Candida boidinii* strain DSM 70026 *(Candida boidinii* strain TK 62), 3.75 U PfuUltra DNA polymerase, buffer for PfuUltra DNA polymerase, 0.2 mM dNTP, and 20 pmol each of the sense primer CbDAO-A1 and the antisense primer CbDAO-T1. A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), and thus a DNA fragment was recovered.

The obtained DNA fragment was digested simultaneously with restriction enzymes Nde I and Afl II, treated with phenol-chloroform, and electrophoresed on an agarose gel. A target band was cut out and purified with the Sephaglas Band Prep Kit (Pharmacia) to recover a target DNA fragment. A plasmid pSE420U was digested simultaneously with restriction enzymes Nde I and Afl II, to which the target DNA fragment was ligated using T4 DNA ligase (TAKARA BIO INC.). *Escherichia coli* strain JM109 was transformed with the obtained plasmid. The transformant was cultivated on an ampicillin-containing LB medium plate, a colony-direct PCR was conducted using the sense primer CbDAO-A1 and the antisense primer CbDAO-T1, and the sizes of the inserted fragments were determined. A colony which was considered to have an inserted DNA fragment of 1.1 kb was cultivated in an ampicillin-containing LB medium, subjected to purification with the FlexiPrep Kit (Pharmacia), and thereby yielded a plasmid pSUCBDO1 (Fig. 2).

The nucleotide sequence of the inserted DNA in the purified plasmid pSUCBDO1 was analyzed by primer walking using the BigDye Terminator Cycle Sequencing FS Ready Reaction Kit (The Perkin-Elmer Corporation) and the ABI PRISMTM 310 (The Perkin-Elmer Corporation). The nucleotide sequence of the D-amino acid oxidase derived from *Candida boidinii* strain DSM 70026 which had been inserted into the plasmid was compared with the nucleotide sequence (Accession No. AB042032) of the D-amino acid oxidase gene derived from *Candida boidinii* TK 62 described in Yeast, 16, 1217 (2000). As a result, there occurred six base substitutions including three amino-acid substitutions, i.e., 77G→T (Cys27→Phe), 83C→A (Ala28→Asp), 198A→C, 243C→A (His81→Gln), 501A→G, and 525A→G in which the origin of an open reading frame (ORF) was defined as position 1. This verified that the plasmid pSUCBDO1 contains the D-amino acid oxidase gene derived from *Candida boidinii* strain DSM 70026.

Using the plasmid pSUCBDO1, a D-alanine oxidation activity was determined according to the above-mentioned enzymatic activity measuring method. The measured specific activity was 4.68 U/mg-protein.

PREPARATION EXAMPLE 2

Construction of Plasmid pSUTVDO1 Containing D-Amino Acid Oxidase Gene

Cells were prepared by cultivating *Trigonopsis variabilis* strain CBS 4095, from which chromosomal DNA was separated and subjected to purification by the procedure of Preparation Example 1.

A D-amino acid oxidase gene derived from *Trigonopsis variabilis* CBS 4095 (Accession No. Z50019) as described in PCT Japanese Translation Patent Publication No. 2001-506868 contains an intron in ORF. Accordingly, cloning of the second exon region was initially conducted. A sense primer TvDAO-e2A1 (SEQ ID NO: 5) and an antisense primer TvDAO-T2 (SEQ ID NO: 6) were synthesized for cloning from coding regions at the 5'- and 3'-ends of an intron region upstream from the second exon of the gene.

SEQ ID NO: 5: GACGCCGGCGTTGCAGGTTTAACTAC
SEQ ID NO: 6: CTCAAGCTTCTAGATTAAAGATTTGGACGAGTAAGAGCTC

A PCR was conducted under the same conditions as in Preparation Example 1, using the chromosomal DNA purified from *Trigonopsis variabilis* strain CBS 4095 as a template, and the primers TvDAO-e2A1 and TvDAO-T2 as primers. A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), and thereby a DNA fragment containing the second exon was recovered.

The obtained DNA fragment was digested with restriction enzyme Hind III, electrophoresed on an agarose gel, from which a target band was cut out and purified with the Sephaglas Band Prep Kit (Pharmacia). A plasmid vector pUC119 was digested simultaneously with restriction enzymes Hind III and Hinc II, to which the DNA fragment containing the second exon was ligated using T4 DNA ligase (TAKARA BIO INC.) to form a plasmid, and *Escherichia coli* strain JM109 was transformed with the plasmid.

The transformant was cultivated in an ampicillin-containing LB medium, subjected to purification with the FlexiPrep Kit (Pharmacia), and thereby yielded a plasmid pUCTVDOT (Fig. 3).

The nucleotide sequence of the inserted DNA in the purified plasmid pUCTVDOT was analyzed by primer walking under the same conditions as in Preparation Example 1. The identified nucleotide sequence was compared with the nucleotide sequence of the *Trigonopsis variabilis* CBS 4095-derived D-amino acid oxidase gene as described in PCT Japanese

Translation Patent Publication No. 2001-506868 (Accession No. Z50019) to find that one-base substitution of 1002G→A occurred in which the origin of the primer ORF was defined as position 1. This verified that the plasmid pUCTVDOT contains the second exon region of the D-amino acid oxidase gene derived from *Trigonopsis variabilis* strain CBS 4095.

The first exon region of the D-amino acid oxidase gene contained in chromosomal DNA of *Trigonopsis variabilis* strain CBS 4095 was synthesized as TvDAO-e5 (SEQ ID NO: 7) and TvDAO-e3 (SEQ ID NO: 8).

SEQ ID NO: 7: CAGCCATGGCTAAAATCGTTGTTATTGGTG
SEQ ID NO: 8: CCGGCACCAATAACAACGATTTTAGCCATGGCTG

A reaction mixture (50 μL) containing 1.25 μmol each of TvDAO-e3 and TvDAO-e5, and 0.1 mM EDTA in 10 mM Tris-HCl buffer (pH 8.0) was denatured at 94°C for one minute, annealed at 61°C for one minute, digested with restriction enzyme Nco I, treated with phenol-chloroform, electrophoresed on an agarose gel, from which a target band was cut out and purified with the Sephaglas Band Prep Kit (Pharmacia), and thereby yielded a DNA fragment containing the second exon. The plasmid pUCTVDOT was digested simultaneously with restriction enzymes NgoM IV and Hind III, electrophoresed on an agarose gel, from which a target band was cut out, purified with the Sephaglas Band Prep Kit (Pharmacia), and thereby yielded a DNA fragment containing the first exon.
The plasmid pSE420U described in Preparation Example 1 was digested simultaneously with restriction enzymes Nco I and Hind III, to which the DNA fragments containing the first exon and the second exon, respectively, were ligated using T4 DNA ligase (TAKARA BIO INC.). Thus, a plasmid pSUTVD01 containing, as integrated therein, the D-amino acid oxidase gene derived from *Trigonopsis variabilis* strain CBS 4095 was constructed (Fig. 4).

A D-alanine oxidation activity by the plasmid pSUTVDO1 was determined according to the above-mentioned enzymatic activity measuring method. The measured specific activity was 5.26 U/mg-protein.

PREPARATION EXAMPLE 3

Construction of Plasmid pETECDD1 Containing D-Amino Acid Dehydrogenase Gene

Cells were prepared by cultivating *Escherichia coli* strain JM109 in an LB medium, from which chromosomal DNA was separated and purified according to the method as described in Nucleic Acids Res., 8, 4321 (1980).
A sense primer EcDadA-A1 (SEQ ID NO: 9) and an antisense primer EcDadA-T1 (SEQ ID NO: 10) were synthesized for cloning based on the D-amino acid dehydrogenase gene derived from *Escherichia coli* strain K-12 (Accession No. L02948) as described in J. Bacteriol., 176, 1500 (1994).

SEQ ID NO: 9: TGGCCATGGCTCGTGTTGTAATTCTGGGAAGTGGTGTG
SEQ ID NO: 10: CAGTCTAGATTAAGAATGTGCACCATGTAAATGGCCC

A PCR was conducted under the same conditions as in Preparation Example 1, using the chromosomal DNA purified from *Escherichia coli* strain K-12 as a template, and the primers EcDadA-A1 and EcDadA-T1 as primers. A part of the resulting PCR reaction mixture was analyzed by electrophoresis on an agarose gel to detect a highly specific band of about 1.3 kbp. This band was cut out, purified with the GFX PCR DNA and Gel Band Purification Kit (Pharmacia), and a target DNA fragment was thereby recovered.
A plasmid vector pUC118 was digested with restriction enzyme Hinc II, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pUCECDD1 (Fig. 5).
The nucleotide sequence of the inserted DNA in the purified plasmid pUCECDD1 was analyzed by primer walking under the same conditions as in Preparation Example 1. The identified nucleotide sequence was found to completely agree with the nucleotide sequence of the D-amino acid dehydrogenase gene derived from *Escherichia coli* strain K-12 (Accession No. L02948) as described in J. Bacteriol., 176, 1500 (1994). This verified that the plasmid pUCECDD1 contains the D-amino acid dehydrogenase gene derived from *Escherichia coli* strain K-12.

The obtained pUCECDD1 was digested simultaneously with restriction enzymes Nco I and EcoR I, electrophoresed on an agarose gel, from which a target band was cut out and purified with the Sephaglas Band Prep Kit (Pharmacia), and thereby yielded a target DNA fragment.
A plasmid vector pET-21d was digested simultaneously with restriction enzymes Nco I and EcoR I, to which the target DNA fragment was ligated using T4 DNA ligase (TAKARA BIO INC.), and *Escherichia coli* strain BL21 (DB3)pLysS was transformed with the obtained plasmid. By purifying from the transformant by the procedure of Preparation Example 1, a plasmid pETECDD1 was obtained (Fig. 6). This plasmid contained, as integrated therein, the D-amino acid dehydrogenase gene derived from *Escherichia coli* strain K-12.

The *Escherichia coli* strain BL21(DB3)pLysS transformed with the purified plasmid pETECDD1 was cultivated

in an LB medium containing ampicillin and chloramphenicol (25μg/mL) at 28°C overnight and then combined with 0.1 mM IPTG to induce gene expression, followed by cultivation at 30°C for further four hours. Cells were collected, suspended in a 50 mM potassium phosphate buffer (pH 8.0) containing 0.02% 2-mercaptoethanol, crushed with the Closed System Ultrasonic Cell Disrupter UCD-200TM (COSMO BIO Co., Ltd.), separated by centrifugation, and a supernatant (cell-free extract) was recovered. A D-alanine oxidation activity by the cell-free extract was measured according to the above-mentioned enzymatic activity measuring method. The measured specific activity was 49.2 mU/mg-protein.

PREPARATION EXAMPLE 4

Construction of Plasmid pSU-MF26 Containing Formate Dehydrogenase Mutant Gene

An expression plasmid pSE-MF26 containing a mutant gene of the formate dehydrogenase gene derived from *Mycobacterium vaccae* (JP-A No. 2003-199595) was digested simultaneously with restriction enzymes Nco I and Xba I, treated with phenol-chloroform, electrophoresed on an agarose gel, from which a target band was cut out and purified with the Sephaglas Band Prep Kit (Pharmacia). The plasmid pSE420U (Preparation Example 1) was digested with these restriction enzymes Nco I and Xba I and ligated to the above-obtained DNA fragment using T4 ligase (TAKARA BIO INC.), and thereby yielded a plasmid. *Escherichia coli* strain JM109 was transformed with the obtained plasmid, cultivated in an ampicillin-containing LB medium, from which a transformant was selected. By purifying from the selected transformant, a plasmid pSU-MF26 was obtained (Fig. 7). This plasmid contains the formate dehydrogenase mutant gene derived from *Mycobacterium vaccae.*

PREPARATION EXAMPLE 5

Construction of Plasmid pSF-GSA2 Containing Two Genes (L-Alanine Dehydrogenase and Formate Dehydrogenase)

A sense primer BstAlaDH-A1 (SEQ ID NO: 11) and an antisense primer BstAlaDH-T1 (SEQ ID NO: 12) were synthesized for cloning based on the nucleotide sequence (Accession No. M33299) of an L-alanine dehydrogenase gene derived from *Geobacillus stearothermophilus* strain IFO 12550 as described in Biochemistry, 29, 1009 (1990).

SEQ ID NO: 11: GAGGAATTCTGTCATGAAAATTGGTATTCCAAAAGAAATCAAAAACAATG
SEQ ID NO: 12: CTGAAGCTTCTAGATTATCCATGTAACAACGAATGAACATCTG

A PCR was conducted by the procedure of Preparation Example 1, using a plasmid pICD301 as a template, and the sense primer BstAlaDH-A1 and the antisense primer BstAlaDH-T1 as primers, in which the plasmid pICD301 was a plasmid expressing a leucine dehydrogenase derived from *Geobacillus stearothermophilus* strain IFO 12250 described in Biochemistry, 29, 1009 (1990). A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), digested simultaneously with restriction enzymes EcoR I and Hind III, treated with phenol-chloroform, electrophoresed on an agarose gel, from which a target band was cut out and purified with the Sephaglas Band Prep Kit (Pharmacia), and thereby yielded a target DNA fragment.
The plasmid pSU-MF26 obtained in Preparation Example 4 was digested simultaneously with restriction enzymes EcoR I and Hind III, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSF-GSA2 (Fig. 8).
The nucleotide sequence of the inserted DNA in the purified plasmid pSF-GSA2 was analyzed by primer walking under the same conditions as in Preparation Example 1. The identified nucleotide sequence was compared with the nucleotide sequence of *Geobacillus stearothermophilus* strain IFO 12550 as described in Biochemistry, 29, 1009 (1990) to find that there occurred substitutions of 20 bases, deletions of 3 bases, and insertions of 3 bases. Along with this, there occurred substitutions of 16 amino acids, deletion of 1 amino acid, and insertion of 1 amino acid. The obtained nucleotide sequence is shown as SEQ ID NO: 13. This verified that the plasmid pSF-GSA2 contains the L-alanine dehydrogenase gene derived from *Geobacillus stearothermophilus* strain IFO 12550 and the formate dehydrogenase mutant gene derived from *Mycobacterium vaccae.*
Using the plasmid pSF-GSA2, an L-alanine oxidation activity and a formic acid oxidation activity were measured according to the above-mentioned enzymatic activity measuring methods. The measured specific activities were 2.40 U/mg-protein and 0.74 U/mg-protein, respectively.

PREPARATION EXAMPLE 6

Construction of Plasmid pSF-BTA1 Containing Two Genes (L-Alanine Dehydrogenase and Formate Dehydrogenase)

A sense primer BthAlaDH-A1 (SEQ ID NO: 14) and an antisense primer BthAlaDH-T1 (SEQ ID NO: 15) were synthesized for cloning based on the nucleotide sequence of the alanine dehydrogenase gene derived from *Bacillus thermocatenulatus* strain DSM 730 as described in Biochimie, 71, 559 (1989).

SEQ ID NO: 14: GAGGAATTCTATTCATGATTATTGGTGTGCCAAAGGAA
SEQ ID NO: 15: CAGAAGCTTCTAGATTAATTAGCAGCCAACGTTTTCC

A PCR was conducted by the procedure of Preparation Example 1, using the plasmid pKUAD103 as a template, and the sense primer BthAlaDH-A1 and the antisense primer BthAlaDH-T1 as primers. A part of the resulting PCR reaction mixture was analyzed by electrophoresis on an agarose gel to detect a highly specific band of about 1.2 kbp. This band was cut out, purified with the GFX PCR DNA and Gel Band Purification Kit (Pharmacia), digested simultaneously with restriction enzymes EcoR I and Hind III, treated with phenol-chloroform, electrophoresed on an agarose gel, from which a target band was cut out and purified with the Sephaglas Band Prep Kit (Pharmacia), and thereby yielded a target DNA fragment.

The plasmid pSE-MF26 obtained in Preparation Example 4 was digested simultaneously with restriction enzymes EcoR I and Hind III, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSF-BTA1 (Fig. 9).

The nucleotide sequence of the inserted DNA in the purified plasmid pSF-BTA1 was analyzed by primer walking. The identified nucleotide sequence was found to completely agree with the nucleotide sequence of the L-alanine dehydrogenase gene derived from *Bacillus thermocatenulatus* strain DSM 730 as described in Biochimie, 71, 559 (1989). This verified that the plasmid pSF-BTA1 contains the L-alanine dehydrogenase gene derived from *Bacillus thermocatenulatus* strain DSM 730 and the formate dehydrogenase mutant gene derived from *Mycobacterium vaccae.*

A cell-free extract was prepared using the plasmid pSF-BTA1, and the L-alanine oxidation activity and the formic acid oxidation activity thereof were measured according to the enzymatic activity measuring methods, except for carrying out cultivation at 25°C overnight. The measured specific activities were 3.38 U/mg-protein and 1.24 U/mg-protein, respectively.

PREPARATION EXAMPLE 7

Construction of Plasmid pSFBPAD1 Containing Two Genes (L-Alanine Dehydrogenase and Formate Dehydrogenase)

Cells were prepared by cultivating *Bacillus sphaericus* strain IFO 3525 in an LB medium, from which chromosomal DNA was separated and purified using the Genome tip 100 (Qiagen).

A sense primer BspAlaDH-A1 (SEQ ID NO: 16) and an antisense primer BspAlaDH-T1 (SEQ ID NO: 17) were synthesized based on the nucleotide sequence (Accession No. M33298) of an alanine dehydrogenase gene derived from *Bacillus sphaericus* strain IFO 3525 as described in Biochemistry, 29, 1009 (1990).

SEQ ID NO: 16: GTGGAATTCTATCATGAAAATTGGTATTCCAAAGGAAATTAAAAACAACG
SEQ ID NO: 17: CTGAAGCTTCTAGATTATTGGATTAATTCATCCACATTCACATATGG

A PCR was conducted under the same conditions as in Preparation Example 1, using the chromosomal DNA purified from *Bacillus sphaericus* strain IFO 3525 as a template, and the primers BspAlaDH-A1 and BspAlaDH-T1 as primers. A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), digested simultaneously with restriction enzymes EcoR I and Hind III, treated with phenol-chloroform, electrophoresed on an agarose gel, from which a target band was cut out, purified with the Sephaglas Band Prep Kit (Pharmacia), and thereby yielded a target DNA fragment.

The plasmid pSU-MF26 obtained in Preparation Example 4 was digested simultaneously with restriction enzymes EcoR I and Hind III, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSFBPAD1 (Fig. 10).

The nucleotide sequence of the inserted DNA in the purified plasmid pSFBPAD1 was analyzed by primer walking under the same conditions as in Preparation Example 1. The identified nucleotide sequence was compared with the nucleotide

sequence (Accession No. M33298) of *Bacillus sphaericus* strain IFO 3525 as described in Biochemistry, 29, 1009 (1990) to find that there occurred substitutions of 6 bases of 99GT→TG, 119G→C, 150G→T, 537G→C, and 888T→C; deletions of 6 bases of 96G, 103G, 104C, 169G, 297C, and 505C; and insertions of 3 bases of 186CA→CAA, 302CT→CGT, and 499CA→CAA, in which the origin of ORF was defined as position 1. The nucleotide sequence is shown as SEQ ID NO: 18. This verified that the plasmid pSFBPAD1 contains the L-alanine dehydrogenase gene derived from *Bacillus sphaericus* strain IFO 3525 and the formate dehydrogenase mutant gene derived from *Mycobacterium vaccae.*

A cell-free extract was prepared using the plasmid pSFBPAD1, and the L-alanine oxidation activity and the formic acid oxidation activity thereof were measured according to the enzymatic activity measuring methods, except for carrying out cultivation at 25°C overnight. The measured specific activities were 8.44 U/mg-protein and 0.83 U/mg-protein, respectively.

PREPARATION EXAMPLE 8

Construction of Plasmid pSF-SAD1 Containing Two Genes (L-Alanine Dehydrogenase and Formate Dehydrogenase)

A sense primer SheAlaDH-A2 (SEQ ID NO: 19) and an antisense primer SheAlaDH-T2 (SEQ ID NO: 20) were synthesized based on the nucleotide sequence (Accession No. AF070715) of a plasmid pSheAlaDH2 having an L-alanine dehydrogenase gene derived from *Shewanella* sp. strain Ac10 as described in Appl. Environ. Mictobiol., 65, 4014 (1999).

SEQ ID NO: 19: AGAGAATTCTATCATGATTATTGGTGTTCCAACAGAAATC
SEQ ID NO: 20: GACAAGCTTCTAGATTAAGCAAGTAGGCTTTTTGGTTCAG

A PCR was conducted by the procedure of Preparation Example 1, using the plasmid pSheAlaDH2 as a template, and the sense primer SheAlaDH-A2 and the antisense primer SheAlaDH-T2 as primers. A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), digested simultaneously with restriction enzymes EcoR I and Hind III, treated with phenol-chloroform, electrophoresed on an agarose gel, from which a target band was cut out, purified with the Sephaglas Band Prep Kit (Pharmacia), and thereby yielded a target DNA fragment. The plasmid pSE-MF26 prepared in Preparation Example 4 was digested simultaneously with restriction enzymes EcoR I and Hind III, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSF-SAD1 (Fig. 11).

The nucleotide sequence of the inserted DNA in the purified plasmid pSF-SAD1 was analyzed by primer walking. The identified nucleotide sequence was found to completely agree with the nucleotide sequence described in Appl. Environ. Mictobiol., 65, 4014 (1999). This verified that the plasmid pSF-SAD1 contains the L-alanine dehydrogenase gene derived from *Shewanella sp.* strain Ac10 and the formate dehydrogenase mutant gene derived from *Mycobacterium vaccae.*

A cell-free extract was prepared using the plasmid pSF-SAD1, and the L-alanine oxidation activity and the formic acid oxidation activity thereof were measured according to the enzymatic activity measuring methods, except for carrying out cultivation at 25°C overnight. The measured specific activities were 3.50 U/mg-protein and 1.02 U/mg-protein, respectively.

PREPARATION EXAMPLE 9

Construction of Plasmid pFGSLED1 Containing Two Genes (L-Leucine Dehydrogenase and Formate Dehydrogenase) Cells were prepared by cultivating *Geobacillus stearothermophilus* strain IFO 12550 in an LB medium, from which chromosomal DNA was separated and purified using the Genome tip 100 (Qiagen).

A sense primer GSTLEUDH-A2 (SEQ ID NO: 21) and an antisense primer GSTLEUDH-T2 (SEQ ID NO: 22) were synthesized based on the nucleotide sequence (Accession No. M22977) of an L-leucine dehydrogenase gene derived from *Geobacillus stearothermophilus* strain IFO 12550 as described in Biochemistry, 27, 9056 (1988).

SEQ ID NO: 21: GTCTCTAGAGGAATTCTACCATGGAATTGTTCAAATATATGGAAACTTACGATTATGAGC
SEQ ID NO: 22: CTGAAGCTTCTAGATTATATTGCCGAAGCACCTGCC

A PCR was conducted by the procedure of Preparation Example 1, using the chromosomal DNA derived from *Geobacillus stearothermophilus* strain IFO 12550 as a template, and the sense primer GSTLEUDH-A2 and the antisense primer GSTLEUDH-T2 as primers. A part of the resulting PCR reaction mixture was analyzed by electrophoresis on an agarose

gel to detect a highly specific band of about 1.3 kbp. This band was cut out, purified with the GFX PCR DNA and Gel Band Purification Kit (Pharmacia), digested simultaneously with restriction enzymes Xba I and Not I, treated with phenol-chloroform, electrophoresed on an agarose gel, from which a target band was cut out, purified with the Sephaglas Band Prep Kit (Pharmacia), and thereby yielded a target DNA fragment. A part of the residual PCR reaction mixture was digested simultaneously with restriction enzymes Not I and Hind III, purified by the above procedure, and thereby yielded a DNA fragment.
A plasmid pSE420D (JP-A No. 2000-189170) was digested simultaneously with restriction enzymes Xba I and Hind III, to which the above obtained two DNA fragments were ligated using T4 DNA ligase (TAKARA BIO INC.). *Escherichia coli* strain JM109 was transformed with the obtained plasmid, subjected to purification by the procedure of Preparation Example 1, and thereby yielded a plasmid pEGSLED2 (Fig. 12).
The nucleotide sequence of the inserted DNA in the purified plasmid was analyzed by primer walking under the same conditions as in Preparation Example 1. The identified nucleotide sequence was compared with the registered sequence (M22977) as described in Biochemistry, 27, 9056 (1988), to find that there occurred an insertion of a noncoding sequence of 186 bp at the 3'-end of the registered sequence. The nucleotide sequence is shown as SEQ ID NO: 23. This verified that the plasmid pEGSLED2 contains the L-leucine dehydrogenase gene derived from *Geobacillus stearothermophilus* strain IFO 12550.
A sense primer GstLeuDH-SalI (SEQ ID NO: 24) and an antisense primer GstLeuDH-TAA1 (SEQ ID NO: 25) were synthesized for cloning based on the nucleotide sequences of an intermediate region and the 3'-end of the L-leucine dehydrogenase gene derived from *Geobacillus stearothermophilus* strain IFO 12550.

SEQ ID NO: 24: GCGGTCGACCCGAACGAC
SEQ ID NO: 25: CTGAAGCTTCTAGATTAACGTGCACGACGGCGGCTTAA

A PCR was conducted by the procedure of Preparation Example 1, using the plasmid pLGSLED2 as a template, and the sense primer GstLeuDH-SalI and the antisense primer GstLeuDH-TAA1 as primers. A part of the resulting PCR reaction mixture was analyzed by electrophoresis on an agarose gel to detect a highly specific band of about 0.7 kbp. This band was cut out, purified with the GFX PCR DNA and Gel Band Purification Kit (Pharmacia), digested simultaneously with restriction enzymes Sal I and Hind III, treated with phenol-chloroform, electrophoresed on an agarose gel, from which a target band was cut out, purified with the Sephaglas Band Prep Kit (Pharmacia), and thereby yielded a target DNA fragment. Separately, the plasmid pLGSLED2 was digested simultaneously with restriction enzymes Xba I and Sal I, purified by the above procedure, and thereby yielded a DNA fragment.
The plasmid pSU-MF26 obtained in Preparation Example 4 was digested simultaneously with restriction enzymes Xba I and Hind III, to which the two DNA fragments were ligated using T4 DNA ligase (TAKARA BIO INC.). *Escherichia coli* strain JM109 was transformed with the obtained plasmid, subjected to purification by the procedure of Preparation Example 1, and thereby yielded a plasmid pFGSLED1 (Fig. 13).
The nucleotide sequence of the inserted DNA in the purified plasmid pFGSLED1 was analyzed by primer walking under the same conditions as in Preparation Example 1, to verify that the plasmid pFGSLED1 contains the L-leucine dehydrogenase gene derived from *Geobacillus stearothermophilus* IFO 12550 and composed of the nucleotide sequence of SEQ ID NO: 23 and the formate dehydrogenase mutant gene derived from *Mycobacterium vaccae.*
A cell-free extract was prepared using the plasmid pFGSLED1, and the L-leucine oxidation activity and the formic acid oxidation activity thereof were measured according to the enzymatic activity measuring methods, except for carrying out cultivation at 24°C overnight. The measured specific activities were 4.03 U/mg-protein and 0.63 U/mg-protein, respectively.

## PREPARATION EXAMPLE 10

### Construction of Plasmid pSFBPLD1 Containing Two Genes (L-Leucine Dehydrogenase and Formate Dehydrogenase)

A sense primer BspLeuDH-A1 (SEQ ID NO: 26) and an antisense primer BspLeuDH-T1 (SEQ ID NO: 27) were synthesized based on the nucleotide sequence (Accession No. AB103119) of the L-leucine dehydrogenase gene derived from *Bacillus sphaericus* described in DDBJ/EMBL/GenBank database.

SEQ ID NO: 26: GTGGAATTCTACCATGGAAATCTTCAAGTATATGGAAAAGTATG
SEQ ID NO: 27: CTCCTTAAGTCTAGATTAACGACCGTTCAAAATGTTTTTTTCATTTTTTAAGAACTG

A PCR was conducted by the procedure of Preparation Example 1, using the chromosomal DNA derived from *Bacillus sphaericus* strain IFO 3525 and prepared in Preparation Example 7 as a template, and the sense primer BspLeuDH-A1 and the antisense primer BspLeuDH-T1 as primers. A part of the resulting PCR reaction mixture was analyzed by

electrophoresis on an agarose gel to detect a specific band of about 1.1 kbp. This band was cut out, purified with the GFX PCR DNA and Gel Band Purification Kit (Pharmacia), digested simultaneously with restriction enzymes EcoR I and Afl II, treated with phenol-chloroform, electrophoresed on an agarose gel, from which a target band was cut out, purified with the Sephaglas Band Prep Kit (Pharmacia), and thereby yielded a target DNA fragment.

The plasmid pSU-MF26 obtained in Preparation Example 4 was digested simultaneously with the restrictions enzymes, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSFBPLD1 (Fig. 14).

The nucleotide sequence of the inserted DNA in the purified plasmid was analyzed by primer walking under the same conditions as in Preparation Example 1. The identified nucleotide sequence was found to completely agree with the nucleotide sequence (Accession No. AB103119) of the L-leucine dehydrogenase gene derived from *Bacillus sphaericus* as described in DDBJ/EMBL/GenBank database. This verified that the plasmid pSFBPLD1 contains the L-leucine dehydrogenase gene derived from *Bacillus sphaericus* strain IFO 3525.

A cell-free extract was prepared using the plasmid pSFBPLD1, and the L-leucine oxidation activity and the formic acid oxidation activity thereof were measured according to the enzymatic activity measuring methods, except for carrying out cultivation at 24°C overnight. The measured specific activities were 5.76 U/mg-protein and 0.54 U/mg-protein, respectively.

## PREPARATION EXAMPLE 11

### Construction of Plasmid pSF-TIP2 Containing Two Genes (Phenylalanine Dehydrogenase and Formate Dehydrogenase)

Cells were prepared by cultivating *Thermoactinomyces intermedius* strain IFO 14230 in an M-64 medium at 55°C, from which chromosomal DNA was purified according to the method described in Methods Enzymol., 152, 116 (1987). The M-64-medium contains 1% peptone, 0.25% yeast extract, 0.2% meat extract, 0.2% glycerol, 0.2% sodium chloride, 0.2% dipotassium hydrogen phosphate, 0.2% potassium dihydrogen phosphate, 0.01% magnesium sulfate heptahydrate, and a trace amount of biotin (2 μL of a 20 mg/100 mL solution per 1 liter of the medium) (pH 7.2).

A sense primer TIP-ATG1 (SEQ ID NO: 28) and an antisense primer TIP-TAA1 (SEQ ID NO: 29) were synthesized based on the nucleotide sequence (Accession No. D00631) of the phenylalanine dehydrogenase gene derived from *Thermoactinomyces intermedius* strain IFO 14230 as described in J. Biochem., 109, 371 (1991).

SEQ ID NO: 28: CAGGAATTCTATAATGCGTGATGTATTTGAAATGATGGAC
SEQ ID NO: 29: CTGAAGCTTCTAGATTAACGACGTGCACTATTACGGGGATCCTCCAA

A PCR was conducted by the procedure of Preparation Example 1, using the chromosomal DNA derived from *Thermoactinomyces intermedius* strain IFO 14230 as a template, and the sense primer TIP-ATG1 and the antisense primer TIP-TAA1 as primers. A part of the resulting PCR reaction mixture was analyzed by electrophoresis on an agarose gel to detect a specific band of about 1.1 kbp. This band was cut out, precipitated with phenol-chloroform, precipitated with ethanol, from which a DNA fragment was recovered, digested simultaneously with restriction enzymes EcoR I and Hind III, treated with phenol-chloroform, electrophoresed on an agarose gel. A target band was cut out therefrom, purified with the Sephaglas Band Prep Kit (Pharmacia), and thereby yielded a target DNA fragment.

The plasmid pSE420U prepared in Preparation Example 1 was digested simultaneously with restriction enzymes EcoR I and Hind III, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSU-TIP2 (Fig. 15).

The nucleotide sequence of the inserted DNA in the purified plasmid pSU-TIP2 was analyzed by primer walking under the same conditions as in Preparation Example 1. The identified nucleotide sequence was compared with the nucleotide sequence (Accession No. D00631) of the L-phenylalanine dehydrogenase gene derived from *Thermoactinomyces intermedius* strain IFO 14230 as described in J. Biochem., 109, 371 (1991), to find that there occurred base substitutions including two amino acid substitutions of 431AG→GC (Lys144→Ser) and 846T→G (Cys282→Trp), in which the origin of ORF was defined as position 1. This verified that the plasmid pSU-TIP2 contains the L-phenylalanine dehydrogenase gene derived from *Thermoactinomyces intermedius* strain IFO 14230.

The plasmid pSE-MF26 prepared in Preparation Example 4 was digested simultaneously with restriction enzymes Nco I and EcoR I, treated with phenol-chloroform, electrophoresed on an agarose gel, from which a target band was cut out, purified with the Sephaglas Band Prep Kit (Pharmacia), and thereby yielded a target DNA fragment. The plasmid pSU-TIP2 was digested simultaneously with restriction enzymes Nco I and EcoR I, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid

pSF-TIP2 (Fig. 16). This plasmid contains the L-phenylalanine dehydrogenase derived from *Thermoactinomyces intermedius* strain IFO 14230 and the formate dehydrogenase gene derived from *Mycobacterium vaccae.*

A cell-free extract was prepared using the plasmid pSF-TIP2, and the L-phenylalanine oxidation activity and the formic acid oxidation activity thereof were measured according to the enzymatic activity measuring methods, except for carrying out cultivation at 24°C overnight. The measured specific activities were 9.56 U/mg-protein and 0.76 U/mg-protein, respectively.

PREPARATION EXAMPLE 12

Construction of Plasmid pSE-ECB1 Containing Branched-Chain-Amino-Acid Transaminase Gene

A sense primer EcBCA-A1 (SEQ ID NO: 30) and an antisense primer EcBCA-T1 (SEQ ID NO: 31) were synthesized based on the nucleotide sequence (Accession No. X02413) of a branched-chain-amino-acid transaminase gene derived from *Escherichia coli* strain K-12 as described in J. Biochem., 97, 993 (1985).

SEQ ID NO: 30: CTGTCATGACTACTAAGAAAGCTGATTACATTTGGTTCAATGG
SEQ ID NO: 31: GTCTCTAGATTATTGATTAACTTGATCTAACCAGCCCCA

A PCR was conducted under the same conditions as in Preparation Example 1, using the chromosomal DNA derived from *Escherichia coli* strain JM109 and prepared in Preparation Example 3 as a template, and the sense primer EcBCA-A1 and the antisense primer EcBCA-T1 as primers. A part of the resulting PCR reaction mixture was analyzed by electrophoresis on an agarose gel to detect a highly specific band. This band was cut out, purified with the GFX PCR DNA and Gel Band Purification Kit (Pharmacia), digested simultaneously with restriction enzymes BspH I and Xba I, treated with phenol-chloroform, electrophoresed on an agarose gel, from which a target band was cut out, purified with the Sephaglas Band Prep Kit (Pharmacia), and thereby yielded a target DNA fragment.
A plasmid pSE420D (JP-A No. 2000-189170) was digested simultaneously with restriction enzymes Nco I and Xba I, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSE-ECB1 (Fig. 17).
The nucleotide sequence of the inserted DNA in the purified plasmid pSFBPAD1 was analyzed by primer walking under the same conditions as in Preparation Example 1. The identified nucleotide sequence was found to completely agree with the nucleotide sequence (Accession No. X02413) of the branched-chain-amino-acid transaminase gene derived from *Escherichia coli* strain K-12 as described in J. Biochem., 97, 993 (1985). This verified that the plasmid pSE-ECB1 contains the branched-chain-amino-acid transaminase gene derived from *Escherichia coli* strain K-12.

A cell-free extract was prepared using the plasmid pSE-ECB1, and the transamination activity thereof was measured according to the above-mentioned enzymatic activity measuring method, except for carrying out cultivation at 24°C overnight. The measured specific activity was 1.44 U/mg-protein.

PREPARATION EXAMPLE 13

Construction of Plasmid pSE-BSB1 Containing Branched-Chain-Amino-Acid Transaminase Gene

Cells were prepared by cultivating *Bacillus subtilis* DB 104 strain in an LB medium, from which chromosomal DNA was separated and purified according to the method described in Nucleic Acids Res., 8, 4321 (1980).
A primer BsBCA-A1 (SEQ ID NO: 32) and an antisense primer BsBCA-T1 (SEQ ID NO: 33) were synthesized based on the nucleotide sequence (Z49992) of a branched-chain-amino-acid transaminase derived from *Bacillus subtilis* as described in J. Bacteriol., 179, 496 (1997).

SEQ ID NO: 32: TGATCATGACTAAACAAACAATTCGCGTTGA
SEQ ID NO: 33: GCTTCTAGATTATTTACTTTCAGTCAGCGCTGCGA

A PCR was conducted by the procedure of Preparation Example 1, using the chromosomal DNA derived from *Bacillus subtilis* DB 104 strain as a template, and the sense primer BsBCA-A1 and the antisense primer BsBCA-T1 as primers. A part of the resulting PCR reaction mixture was analyzed by electrophoresis on an agarose gel to detect a highly specific band. This band was cut out, purified with the GFX PCR DNA and Gel Band Purification Kit (Pharmacia), digested simultaneously with restriction enzymes BspH I and Xba I, treated with phenol-chloroform, electrophoresed on an agarose gel, from which a target band was cut out, purified with the Sephaglas Band Prep Kit (Pharmacia), and thereby yielded a target DNA fragment.

A plasmid pSE420D (JP-A No. 2000-189170) was digested simultaneously with restriction enzymes Nco I and Xba I, to which the target DNA fragment was ligated using T4 DNA ligase (TAKARA BIO INC.), and *Escherichia coli* strain JM109 was transformed therewith. *Escherichia coli* strain JM109 was transformed with the obtained plasmid, subjected to purification by the procedure of Preparation Example 1, and thereby yielded a plasmid pSE-BSB1 (Fig. 18).
The nucleotide sequence of the inserted DNA in the purified plasmid was analyzed by primer walking under the same conditions as in Preparation Example 1. The identified nucleotide sequence was compared with the nucleotide sequence (Z49992) of the branched-chain-amino-acid transaminase derived from *Bacillus subtilis* as described in J. Bacteriol., 179, 496 (1997), to find that the former nucleotide sequence contains two base substitutions including one amino acid substitution of 177A→G and 178A→G (Thr60→Ala), in which the origin of ORF was defined as position 1. This verified that the plasmid pSE-BSB1 contains the branched-chain-amino-acid transaminase gene derived from *Bacillus subtilis.*

A cell-free extract was prepared using the plasmid pSE-BSB1 according to the above-mentioned enzymatic activity measuring method, except for carrying out cultivation at 24°C overnight, and the transamination activity thereof was measured according to the above-mentioned enzymatic activity measuring method. The measured specific activity was 0.30 U/mg-protein.

PREPARATION EXAMPLE 14

Production of 2-Oxopentanoic Acid from DL-Norvaline

*Escherichia coli* strain HB101 was transformed with the plasmid pSUCBDO1 prepared in Preparation Example 1, and the transformant was cultivated in a 2x YT medium (2% Bactotriptone, 1% Bactoyeast extract, 1% sodium chloride, pH 7.2) at 33°C overnight. The medium was combined with 0.1 mM IPTG to induce the expression of the D-amino acid oxidase gene, cells were collected therefrom, and thereby yielded *Escherichia coli* expressing a D-amino acid oxidase.
DL-Norvaline (enantiomeric mixture of norvaline) was placed in three reactors to final concentrations of the substrate of 1 percent by weight (85 mM), 4 percent by weight (341 mM), and 10 percent by weight (850 mM), respectively. To the substrate in the three reactors, 400 mM potassium phosphate buffer (pH 8.0) and the cells prepared from 10 g of the culture were added, to yield reaction mixtures each in a total weight of 10 g, followed by reactions with shaking at 30°C overnight. Samples (each 0.1 g) were collected from the reaction mixtures, and the optical purities and amounts of norvaline contained in the reaction mixtures were analyzed under the after-mentioned norvaline analytical conditions 1. The results are shown in Table 1.

PREPARATION EXAMPLE 15

*Escherichia coli* expressing a D-amino acid oxidase was prepared by the procedure of Preparation Example 14, except for using, as a plasmid, pSUTVDO1 prepared in Preparation Example 2 instead of pSUCBDO1 prepared in Preparation Example 1. Using obtained *Escherichia coli,* reactions were conducted, and the optical purities and amounts of norvaline contained in the reaction mixtures were analyzed by the procedure of Preparation Example 14. The results are shown in Table 1.

PREPARATION EXAMPLE 16

*Escherichia coli* strain BL21(DE3)pLysS was transformed with the plasmid pETECDD1 prepared in Preparation Example 3, and the transformant was cultivated in a 2x YT medium (2% Bactotriptone, 1% Bactoyeast extract, and 1% sodium chloride, pH 7.2) at 33°C overnight. The medium was combined with 0.1 mM IPTG to induce the expression of D-amino acid dehydrogenase gene, cells were collected therefrom, and thereby yielded *Escherichia coli* expressing a D-amino acid dehydrogenase.
DL-Norvaline (enantiomeric mixture of norvaline) was placed in two reactors to final concentrations of the substrate of 1 percent by weight (85 mM) and 4 percent by weight (341 mM), respectively. To the substrate in the two reactors, 400 mM potassium phosphate buffer (pH 8.0) and cells prepared from 10 g of the culture were added, to yield reaction mixtures each in a total weight of 10 g, followed by reactions with shaking at 30°C overnight. Samples (each 0.1 g) were collected from the reaction mixtures, and the optical purities and amounts of norvaline contained in the reaction mixtures were analyzed under the norvaline analytical conditions 1. The results are shown in Table 1.

PREPARATION EXAMPLE 17

Production of L-Norvaline from 2-Oxopentanoic Acid (Using L-Amino Acid Dehydrogenase)

*Escherichia coli* strain HB101 was transformed with the plasmid pSF-GSA2 prepared in Preparation Example

5, and the transformant was cultivated in the 2x YT medium (as with Preparation Example 14) at 33°C overnight. The medium was combined with 0.1 mM IPTG to induce the expression of the L-alanine dehydrogenase gene, cells were collected therefrom, and thereby yielded *Escherichia coli* coexpressing an L-alanine dehydrogenase and a formate dehydrogenase.

In three reactors were placed sodium salt of 2-oxopentanoic acid to final concentrations of 2 percent by weight (145 mM), 4 percent by weight (290 mM), and 10 percent by weight (724 mM), respectively, and sodium formate to final concentrations of 174 mM, 348 mM, and 869 mM, respectively. To these substances in the three reactors, were added 400 mM potassium phosphate buffer (pH 8.0) and the cells prepared from 10 g of the culture respectively, to yield reaction mixtures each in a total weight of 10 g, followed by reactions with shaking at 30°C overnight. Samples (each 0.1 g) were collected from the reaction mixtures, and the optical purities and amounts of norvaline contained in the reaction mixtures were analyzed under the norvaline analytical conditions 1. The results are shown in Table 2.

PREPARATION EXAMPLE 18

*Escherichia coli* coexpressing an L-alanine dehydrogenase and a formate dehydrogenase was prepared by the procedure of Preparation Example 17, except for using, as a plasmid, pSF-BTA1 prepared in Preparation Example 6 instead of pSF-GSA2 prepared in Preparation Example 5. Using obtained *Escherichia coli,* reactions were conducted, and the optical purities and amounts of norvaline contained in the reaction mixtures were determined by the procedure of Preparation Example 17. The results are shown in Table 2.

PREPARATION EXAMPLE 19

*Escherichia coli* coexpressing an L-alanine dehydrogenase and a formate dehydrogenase was prepared by the procedure of Preparation Example 17, except for using, as a plasmid, pSFBPAD1 prepared in Preparation Example 7 instead of pSF-GSA2 prepared in Preparation Example 5. Using obtained *Escherichia coli,* reactions were conducted, and the optical purities and amounts of norvaline contained in the reaction mixtures were determined by the procedure of Preparation Example 17. The results are shown in Table 2.

PREPARATION EXAMPLE 20

*Escherichia coli* coexpressing an L-alanine dehydrogenase and a formate dehydrogenase was prepared by the procedure of Preparation Example 17, except for using, as a plasmid, pSF-SAD1 prepared in Preparation Example 8 instead of pSF-GSA2 prepared in Preparation Example 5. Using obtained *Escherichia coli,* reactions were conducted, and the optical purities and amounts of norvaline contained in the reaction mixtures were determined by the procedure of Preparation Example 17. The results are shown in Table 2.

PREPARATION EXAMPLE 21

*Escherichia coli* coexpressing an L-leucine dehydrogenase and a formate dehydrogenase was prepared by the procedure of Preparation Example 17, except for using, as a plasmid, pFGSLED1 prepared in Preparation Example 9 instead of pSF-GSA2 prepared in Preparation Example 5. Using obtained *Escherichia coli,* reactions were conducted, and the optical purities and amounts of norvaline contained in the reaction mixtures were determined by the procedure of Preparation Example 17. The results are shown in Table 2.

PREPARATION EXAMPLE 22

*Escherichia coli* coexpressing an L-leucine dehydrogenase and a formate dehydrogenase was prepared by the procedure of Preparation Example 17, except for using, as a plasmid, pSFBPLD1 prepared in Preparation Example 10 instead of pSF-GSA2 prepared in Preparation Example 5. Using obtained *Escherichia coli,* reactions were conducted, and the optical purities and amounts of norvaline contained in the reaction mixtures were determined by the procedure of Preparation Example 17. The results are shown in Table 2.

PREPARATION EXAMPLE 23

*Escherichia coli* coexpressing an L-amino acid dehydrogenase and a formate dehydrogenase was prepared by the procedure of Preparation Example 17, except for using, as a plasmid, pSF-TIP2 prepared in Preparation Example 11 instead of pSF-GSA2 prepared in Preparation Example 5. Using obtained *Escherichia coli,* reactions were conducted, and the optical purities and amounts of norvaline contained in the reaction mixtures were determined by the procedure

of Preparation Example 17. The results are shown in Table 2.

PREPARATION EXAMPLE 24

Production of L-Norvaline from 2-Oxopentanoic Acid (Using L-Amino Acid Transaminase)

*Escherichia coli* expressing a branched-chain-amino-acid transaminase was prepared by the procedure of Preparation Example 17, except for using, as a plasmid, pSE-ECB1 prepared in Preparation Example 12 instead of pSF-GSA2 prepared in Preparation Example 5.

In two reactors were placed sodium salt of 2-oxopentanoic acid (sodium 2-oxopentanoate) to final concentrations of 2 percent by weight (145 mM) and 4 percent by weight (290 mM), respectively, and sodium L-glutamate as an amino donor to final concentrations of 290 mM and 580 mM, respectively. To these substances in the two reactors, was added 400 mM potassium phosphate buffer (pH 8.0), the pH was adjusted to 8.0 with 5 M aqueous sodium hydroxide solution, and cells prepared from 10 g of the culture were added, to yield reaction mixtures each in a total weight of 10 g, followed by reactions with shaking at 30°C overnight. Samples (each 0.1 g) were collected from the reaction mixtures, and the optical purities and amounts of norvaline contained in the reaction mixtures were analyzed under the norvaline analytical conditions 1. The results are shown in Table 3.

PREPARATION EXAMPLE 25

*Escherichia coli* expressing a branched-chain-amino-acid transaminase prepared in Preparation Example 24 was used in the following reactions. Specifically, reactions were conducted by the procedure of Preparation Example 24, except for using, as an amino donor, L-aspartic acid instead of sodium L-glutamate, and the optical purities and amounts of norvaline contained in the reaction mixtures were determined. The results are shown in Table 3.

PREPARATION EXAMPLE 26

*Escherichia coli* expressing a branched-chain-amino-acid transaminase was prepared by the procedure of Preparation Example 17, except for using, as a plasmid, pSE-BSB1 prepared in Preparation Example 13 instead of pSF-GSA2 prepared in Preparation Example 5. Using obtained *Escherichia coli,* reactions were conducted, and optical purities and amounts of norvaline contained in the reaction mixtures were determined by the procedure of Preparation Example 24. The results are shown in Table 3.

PREPARATION EXAMPLE 27

*Escherichia coli* expressing a branched-chain-amino-acid transaminase prepared in Preparation Example 26 was used in the following reactions. Specifically, reactions were conducted by the procedure of Preparation Example 26, except for using, as an amino donor, L-aspartic acid instead of sodium L-glutamate, and the optical purities and amounts of norvaline contained in the reaction mixtures were determined. The results are shown in Table 3.

PREPARATION EXAMPLE 28

Production of L-Norvaline Using Cell Mixture

*Escherichia coli* strain HB101 was transformed with the plasmid pSUCBDO1 prepared in Preparation Example 1, and the transformant was cultivated in 50 ml of the 2x YT medium (as with Preparation Example 14) at 33°C overnight. The medium was combined with 0.1 mM IPTG to induce gene expression, cells were collected therefrom, and thereby yielded *Escherichia coli* expressing a D-amino acid oxidase.

Separately, *Escherichia coli* strain HB101 was transformed with the plasmid pSF-TIP2 prepared in Preparation Example 11, and the transformant was cultivated in 50 ml of the 2x YT medium (as with Preparation Example 14) at 33°C overnight. The medium was combined with 0.1 mM IPTG to induce gene expression, cells were collected therefrom, and thereby yielded *Escherichia coli* coexpressing an L-phenylalanine dehydrogenase and a formate dehydrogenase.

The cells prepared from 5 g each of the two cultures were added to a reaction mixture containing 4 percent by weight DL-norvaline, 0.20 M sodium formate, and 0.75 M ammonia buffer (pH 8.0) to a total weight of 10 g, followed by a reaction at 30°C with shaking under aeration conditions. Samples (each 0.1 g) were collected from the reaction mixture after 20 hours and 45 hours from the beginning of the reaction, and the optical purities and amounts of norvaline contained in the reaction mixtures were analyzed under the norvaline analytical conditions 1. The results are shown in Table 4.

PREPARATION EXAMPLE 29

A reaction was conducted by the procedure of Preparation Example 28, except for using, as a reaction mixture, one containing 10 percent by weight DL-norvaline, and 0.51 M sodium formate in 0.75 M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 4.

PREPARATION EXAMPLE 30

Production of L-Norvaline Using Cell Mixture

*Escherichia coli* strain HB101 was transformed with the plasmid pSUCBDO1 prepared in Preparation Example 1, and the transformant was cultivated in 50 ml of the 2x YT medium (as with Preparation Example 14) at 33°C overnight. The medium was combined with 0.1 mM IPTG to induce gene expression, cells were collected therefrom, and thereby yielded *Escherichia coli* expressing a D-amino acid oxidase.
Separately, *Escherichia coli* strain HB101 was transformed with the plasmid pSF-GSA2 prepared in Preparation Example 5, and the transformant was cultivated in 50 ml of the 2x YT medium (as with Preparation Example 14) at 33°C overnight. The medium was combined with 0.1 mM IPTG to induce gene expression, cells were collected therefrom, and thereby yielded *Escherichia coli* coexpressing an L-alanine dehydrogenase and a formate dehydrogenase.
The cells prepared from 5 g each of the two cultures were added to a reaction mixture containing 4 percent by weight DL-norvaline, 0.20 M sodium formate, and 0.75 M ammonia buffer (pH 8.0) to a total weight of 10 g, followed by a reaction at 30°C with shaking under aeration conditions. Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 4.

PREPARATION EXAMPLE 31

A reaction was conducted by the procedure of Preparation Example 30, except for using, as a reaction mixture, one containing 10 percent by weight DL-norvaline and 0.51 M sodium formate in 0.75 M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 4.

PREPARATION EXAMPLE 32

Construction of Vector pSE420Q Capable of Expressing Four Genes

The plasmid pSE420U prepared in Preparation Example 1 was digested simultaneously with SpeI, and synthetic DNAs (SEQ ID NO: 35 and SEQ ID NO: 36) were annealed and ligated to this site, to thereby construct a plasmid pSE420Q.

SEQ ID NO: 35: CTAGTAGAGGTACCTATATATGCATGTGCACGC
SEQ ID NO: 36: TTAAGCGTGCACATGCATATATAGGTACCTCTA

PREPARATION EXAMPLE 33

Construction of Plasmid pSQECKE1 Containing Catalase Gene katE Derived from *Escherichia coli*

A sense primer EckatE-A1 (SEQ ID NO: 37) and an antisense primer EckatE-T1 (SEQ ID NO: 38) were synthesized for cloning based on the nucleotide sequence (Accession No. NC_000913 REGION: 1811891..1814152) of the catalase gene katE derived from *Escherichia coli* strain K-12.

SEQ ID NO: 37: gacggtacctatacATGTCTCAACATAACGAAAAGAACCCACA
SEQ ID NO: 38: tcgaagcttaagaTTATGCAGGAATTTTGTCAATCTTAGGAATGC

A PCR was conducted by the procedure of Preparation Example 1, using the chromosomal DNA derived from *Escherichia coli* strain K-12 as a template, and the sense primer EckatE-A1 (SEQ ID NO: 37) and the antisense primer EckatE-T1

(SEQ ID NO: 38) as primers. Thus, a DNA fragment composed of the nucleotide sequence of SEQ ID NO: 39 was amplified. The obtained DNA fragment was digested simultaneously with restriction enzymes KpnI and HindIII, and ligated to the plasmid pSE420Q prepared in Preparation Example 32 which had been digested simultaneously with these restriction enzymes, to thereby construct pSQECKE1.
The catalase activity by the plasmid pSQECKE1 was determined according to the above-mentioned enzymatic activity measuring method. The measured specific activity was 260 U/m g-protein.
The *Escherichia* coli-derived catalase EckatE contained in the plasmid pSQECKE1 has been reported to have a higher catalase activity and a higher stability, and a lower peroxidase activity than the *Escherichia* coli-derived catalase EckatG contained in the plasmid pSQECKG1 prepared in Preparation Example 34. These properties are more suitable for the objects of the present invention, and the plasmid pSQECKE1 expressing EckatE was used in the following experiments.

PREPARATION EXAMPLE 34

Construction of Plasmid pSQECKG1 Containing Catalase Gene katG Derived from *Escherichia coli*

A sense primer EckatG-A1 (SEQ ID NO: 40) and an antisense primer EckatG-T1 (SEQ ID NO: 41) were synthesized for cloning based on the nucleotide sequence (Accession NC_000913 REGION: 4131858..4134038) of the catalase gene katG derived from *Escherichia coli* strain K-12.

SEQ ID NO: 40: gacggtaccatatcATGAGTACTTCAGACGATATCCATAACAC
SEQ ID NO: 41: gacaagcttaagaTTAAAGCAGATCAAAACGGTCGAGG

A PCR was conducted by the procedure of Preparation Example 1, using the chromosomal DNA derived from *Escherichia coli* strain K-12 as a template, and the sense primer EckatG-A1 (SEQ ID NO: 40) and the antisense primer EckatG-T1 (SEQ ID NO: 41) as primers. Thus, a DNA fragment composed of the nucleotide sequence of SEQ ID NO: 42 was amplified. The obtained DNA fragment was digested simultaneously with restriction enzymes KpnI and HindIII, ligated to pSE420Q which had been prepared in Preparation Example 32 and digested simultaneously with these restriction enzymes, to construct pSQECKG1.
The catalase activity by the plasmid pSQECKG1 was determined according to the above-mentioned enzymatic activity measuring method. The measured specific activity was 108 U/mg-protein.

PREPARATION EXAMPLE 35

Construction of Plasmid pSQTIP2 Containing Two Genes (Phenylalanine Dehydrogenase and Catalase)

A sense primer TIP-ATG4 (SEQ ID NO: 43) and an antisense primer TIP-TAA4 (SEQ ID NO: 44) were synthesized based on the nucleotide sequence (Accession No. D00631) of a phenylalanine dehydrogenase gene derived from *Thermoactinomyces intermedius* strain IFO 14230 as described in J. Biochem., 109, 371 (1991).

SEQ ID NO: 43: CAGGAATTCTATAATGCGTGATGTATTTGAAATGATGGAC
SEQ ID NO: 44: gtcaggtacctcTTAACGACGTGCACTATTACGG

A PCR was conducted by the procedure of Preparation Example 1, using the phenylalanine dehydrogenase-expressing plasmid pSU-TIP2 prepared in Preparation Example 11 as a template, and the sense primer TIP-ATG4 and the antisense primer TIP-TAA4 as primers, to amplify a DNA fragment. The obtained DNA fragment was digested simultaneously with restriction enzymes KbaI and KpnI, ligated to pSQECKE1 which had been prepared in Preparation Example 33 and digested simultaneously with these restrictions enzymes, and thereby yielded a plasmid pSQTIP4. The plasmid pSQTIP4 contains the L-phenylalanine dehydrogenase derived from *Thermoactinomyces intermedius* strain IFO 14230 and the catalase gene katE derived from *Escherichia coli* strain K-12.

EXAMPLE 1

Construction of Plasmid pSFTPCO1 Containing Three Genes

An antisense primer CbDAO-T2 (SEQ ID NO: 34) corresponding to the D-amino acid oxidase gene derived from *Candida boidinii* strain DSM 70026 was synthesized.

SEQ ID NO: 34: GTCTTAATTAAAGTTTAGCTTTAACTTTTTGGTTATCAACTAA

A PCR was conducted by the procedure of Preparation Example 11 using the plasmid pSUCBDO1 prepared in Preparation Example 11 as a template, and the sense primer CbDAO-A1 (SEQ ID NO: 3) and the antisense primer CbDAO-T1 (SEQ ID NO: 4) used in Preparation Example 11 as primers. A part of the resulting PCR reaction mixture was analyzed by electrophoresis on an agarose gel to detect a specific band of about 1.1 kbp. This band was cut out, purified with the GFX PCR DNA and Gel Band Purification Kit (Pharmacia), digested simultaneously with restriction enzymes Nde I and Pac I, treated with phenol-chloroform, electrophoresed on an agarose gel, from which a target band was cut out, purified with the Sephaglas Band Prep Kit (Pharmacia), and thereby yielded a target DNA fragment.

The plasmid pSF-TIP2 prepared in Preparation Example 11 was digested simultaneously with restriction enzymes Nde I and Pac I, treated with phosphatase, and subsequently treated with phenol-chloroform, to which the target DNA fragment was ligated using T4 DNA ligase (TAKARA BIO INC.). *Escherichia coli* strain JM109 was transformed with the obtained plasmid, subjected to purification by the procedure of Preparation Example 1, and thereby yielded a plasmid pSFTPCO1 (Fig. 19).

The nucleotide sequence of the inserted DNA in the purified plasmid was analyzed by primer walking under the same conditions as in Preparation Example 1 to verify that the plasmid contains the D-alanine oxidase derived from *Candida boidinii,* the L-phenylalanine dehydrogenase derived from *Thermoactinomyces intermedius,* and the formate dehydrogenase derived from *Mycobacterium vaccae.*

Using the plasmid pSFTPCO1, a cell-free extract was prepared and the respective enzymatic activities were determined according to the enzymatic activity measuring methods except for carrying out cultivation at 26°C overnight, to find that the sample had, in terms of specific activities, a D-alanine oxidation activity of 2.93 U/mg-protein, an L-phenylalanine oxidation activity of 7.03 U/mg-protein, and a formic acid oxidation activity of 0.71 U/mg-protein, respectively.

EXAMPLE 2

Construction of Plasmid pSFGACO1 Containing Three Genes

The DNA fragment containing the D-amino acid oxidase gene derived from *Candida boidinii* prepared in the PCR in Preparation Example 1, and the plasmid pSF-GSA2 prepared in Preparation Example 5 were respectively digested simultaneously with restriction enzymes Nde I and Pac I, treated with phosphatase, subsequently treated with phenol-chloroform, ligated to each other using T4 DNA ligase (TAKARA BIO INC.), and *Escherichia coli* strain JM109 was transformed therewith. The transformant having the target DNA fragment inserted thereinto was cultivated in a liquid LB medium containing ampicillin, subjected to purification with the FlexiPrep Kit (Pharmacia), and thereby yielded a plasmid pSFGACO1 (Fig. 20) containing and expressing three genes of the D-amino acid oxidase derived from *Candida boidinii,* the alanine dehydrogenase derived from *Geobacillus stearothermophilus,* and the formate dehydrogenase derived from *Mycobacterium vaccae.* The nucleotide sequences of the inserted DNAs in the purified plasmid pSFGACO1 were identified by primer walking.

Using the plasmid pSFGACO1, a cell-free extract was prepared and the respective enzymatic activities were determined according to the enzymatic activity measuring methods, except for carrying out cultivation at 26°C overnight, to find that the sample had, in terms of specific activities, a D-alanine oxidation activity of 2.42 U/mg-protein, an L-alanine oxidation activity of 2.63 U/mg-protein, and a formic acid oxidation activity of 0.79 U/mg-protein, respectively.

EXAMPLE 3

Production of L-Norvaline Using Plasmid pSFTPCO1 Containing Three Genes

*Escherichia coli* strain HB101 was transformed with the three-gene-containing plasmid pSFTPCO1 constructed in Example 1, and the transformant was cultivated in 50 ml of the 2x YT medium (as with Preparation Example 14) at 33°C overnight. The medium was combined with 0.1 mM IPTG to induce gene expression, cells were collected therefrom, and thereby yielded *Escherichia coli* expressing three genes.

Cells prepared from 10 g of the culture were added to a reaction mixture containing 4 percent by weight DL-norvaline and 0.20 M sodium formate in 0.17 M ammonia buffer (pH 8.0) to a total weight of 10 g, followed by a reaction at 30°C with shaking under aeration conditions. Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 4

A reaction was conducted by the procedure of Example 3, except for using, as a reaction mixture, one containing

10 percent by weight DL-norvaline and 0.51 M sodium formate in 0.43M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 5

A reaction was conducted by the procedure of Example 3, except for using, as a reaction mixture, one containing 12 percent by weight DL-norvaline and 0.61 M sodium formate in 0.51 M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 6

A reaction was conducted by the procedure of Example 3, except for using, as a three-gene-containing plasmid, the three-gene-containing plasmid pSFGACO1 constructed in Example 2 instead of pSFTPCO1. Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 7

A reaction was conducted by the procedure of Example 6, except for using, as a reaction mixture, one containing 10 percent by weight DL-norvaline and 0.51 M sodium formate in 0.21 M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 8

A reaction was conducted by the procedure of Example 6, except for using, as a reaction mixture, one containing 12 percent by weight DL-norvaline and 0.61 M sodium formate in 0.26 M ammonia buffer (pH 8.0). Samples (each 0.1 g) were collected from the reaction mixture after 20 hours and 45 hours from the beginning of the reaction, and the optical purities and amounts of norvaline contained in the reaction mixture samples were analyzed under the norvaline analytical conditions 1. The results are shown in Table 5.

EXAMPLE 9

Production of L-Norvaline Using Plasmid pSFTPCO1 Containing Three Genes

*Escherichia coli* strain HB101 was transformed with the three-gene-expressing plasmid pSFTPC01 constructed in Example 1, and the transformant was cultivated in 400 ml of the 2x YT medium (as with Preparation Example 14) at 33°C overnight. The medium was combined with 0.1 mM IPTG to induce gene expression, cells were collected therefrom, and thereby yielded *Escherichia coli* expressing three genes.
The obtained *Escherichia coli* was added to a reaction mixture containing 12 percent by weight (1.02 M) DL-norvaline and 2.8% (0.61 M) sodium formate in 1.1% (0.62 M) ammonia buffer (pH 8.0) up to 400 g, and reacted with stirring under aeration conditions at 30°C for 24 hours. A sample (0.1 g) was collected from the reaction mixture, and the optical purity and amount of norvaline contained in the reaction mixture sample were analyzed under the norvaline analytical conditions 1, to find that the reaction mixture has a L-norvaline concentration of 9.1 percent by weight (0.779 M) and an optical purity of 100% while no D-norvaline was detected.

EXAMPLE 10

A part of the reaction mixture obtained in Example 9 was purified in the following manner.
To 30.5 g of the reaction mixture obtained in Example 9 was added 1.7 g of concentrated sulfuric acid to adjust to pH 2.5, followed by heating and stirring at 30°C for 17 hours. The resulting acidic suspension was separated by centrifugation to remove cells, and the supernatant was deproteinized by adding 50 ml of methanol to precipitate a solid and removing

the precipitated solid by filtration. The filtrate was combined with an aqueous ammonia solution to adjust to pH 6, and further combined with 60 ml of acetone to precipitate solids. The precipitated solids were collected by filtration, dried, and thereby yielded 2.42 g of a white solid containing purified norvaline. The solid was subjected to 1H-NMR analysis using maleic acid as an internal standard and found to have a chemical purity of 95% and an optical purity determined under the after-mentioned norvaline analytical conditions 2 of 100% (L-form).

EXAMPLE 11

A part of the reaction mixture obtained in Example 9 was purified in the following manner.
To 9.1 g of the reaction mixture obtained in Example 9 was added 4.5 ml of water, followed by heating and stirring at 60°C for 2.5 hours. The resulting suspension was separated by centrifugation to remove cells, and deproteinized by centrifugation using an ultrafiltration membrane of a cut-off molecular weight of 10000 (CentriCon YM10, Millipore Corporation) at 2000x g at 30°C. Next, the filtrate was concentrated under reduced pressure until solids were precipitated. The concentrate was combined with 0.1 g of formic acid to adjust to pH 6.2 and further combined with 14.8 mL of acetone to precipitate solids. The solids were collected by filtration, dried, and thereby yielded 0.433 g of a white solid containing purified norvaline. The solid was subjected to 1H-NMR analysis using maleic acid as an internal standard and found to have a chemical purity of 95% and an optical purity determined under the norvaline analytical conditions 2 of 100% (L-form).

EXAMPLE 12

Construction of Plasmid pSFCPC01 Containing Four Genes (D-Amino Acid Oxidase, Formate Dehydrogenase, Phenylalanine Dehydrogenase, and Catalase)

The three-gene-expressing plasmid pSFTP01 prepared in Example 1 was digested simultaneously with restriction enzymes NdeI and XbaI and thereby yielded a DNA fragment containing D-alanine oxidase and formate dehydrogenase genes. Separately, the two-gene-expressing plasmid pSQTIP4 prepared in Preparation Example 35 was digested simultaneously with these restriction enzymes, to which the DNA fragment containing the D-alanine oxidase and formate dehydrogenase genes was ligated, and thereby yielded a plasmid pSFCPC01. The plasmid pSFCPC01 coexpresses four genes including the D-alanine oxidase derived from *Candida boidini,* formate dehydrogenase derived from *Mycobacterium vaccae,* L-phenylalanine dehydrogenase derived from *Thermoactinomyces intermedius* strain IFO 14230, and catalase gene katE derived from *Escherichia coli* strain K-12.
Using the four-gene-expressing plasmid pSFCPCO1, a cell-free extract was prepared and the activities of the corresponding enzymes were measured according to the methods as described in "Measurement of Enzymatic Activity" to find that this has a D-alanine oxidase activity of 2.40 U/mg-protein, a formate dehydrogenase activity of 0.63 U/mg-protein, an L-phenylalanine dehydrogenase activity of 5.30 U/mg-protein, and a catalase activity of 399 U/mg-protein. These verified that all the enzymes are expressed with sufficient activities.

EXAMPLE 13

Production of L-Norvaline Using Plasmid pSFTPCO1 Containing Three Genes

*Escherichia coli* strain HB101 was transformed with the three-gene-containing plasmid pSFTPCO1 constructed in Example 12, and the transformant was cultivated in the 2x YT medium (as with Preparation Example 14) at 33°C overnight. The medium was combined with 0.1 mM IPTG to induce gene expression, cells were collected therefrom, and thereby yielded *Escherichia coli* expressing three genes.
Cells prepared from 5 g of the culture were added to a reaction mixture (pH 8.0) containing 8 percent by weight DL-norvaline and 0.6 equivalent of ammonium formate relative to norvaline, to a total weight of 10 g, followed by a reaction with shaking under aeration conditions at 30°C for 44 hours. A sample (0.1g) was collected from the reaction mixture, and the optical purity and amount of norvaline contained in the reaction mixture was determined under the norvaline analytical conditions 1 to find that L-norvaline was obtained with an optical purity of 100% e.e. in a yield of 65.9%.
The reaction mixture after the completion of the reaction was analyzed by high-performance liquid chromatography to detect no α-ketovaleric acid.

EXAMPLE 14

Production of L-Norvaline Using Plasmid pSFCPC01 Containing Four Genes

A reaction was conducted, and the optical purity and amount of norvaline were determined by the procedure of Example 13, except for using the four-gene-containing plasmid pSFCPCO1 prepared in Example 12 instead of the three-gene-containing plasmid pSFTPCO1. As a result, L-norvaline was obtained with an optical purity of 100% e.e. in a yield of 76.6%.
The concentration of α-ketovaleric acid remained after the completion of the reaction was measured by the procedure of Example 13, to detect 21.3 percent by mole of α-ketovaleric acid relative to the starting material DL-norvaline. This result shows that a reductive amination reaction of the keto acid for forming the L-amino acid from the keto acid did not fully proceed and the α-keto acid remained in the reduction step subsequent to the oxidation step of forming the keto acid from the D-amino acid.

EXAMPLE 15

Process Using Different Conditions in Oxidation Step and Reduction Step

*Escherichia coli* strain HB101 was transformed with the four-gene-containing plasmid pSFCPC01 prepared in Example 12, and the transformant was cultivated in the 2x YT medium (as with Preparation Example 14) at 33°C overnight. The medium was combined with 0.1 mM IPTG to induce gene expression, cells were collected therefrom, and thereby yielded *Escherichia coli* expressing four genes.
The cells prepared from 132 g of the culture were combined with 8 percent by weight of DL-norvaline, 0.6 equivalent of ammonium formate relative to norvaline, and 0.02% ADEKANOL (antifoaming agent; Asahi Denka Kogyo K.K.), and the mixture was adjusted to pH 8.0 with sulfuric acid, and thereby yielded a reaction mixture. In a 1-liter mini jar was placed 400 ml of the reaction mixture, and, as an oxidation step, a reaction was conducted under conditions of an air aeration rate of 0.5 vvm and a stirring rate of 500 rpm at 30°C for 43 hours. A sample (0.1 g) was collected from the reaction mixture after the completion of the oxidation step, and the optical purity and amount of norvaline contained in the reaction mixture were determined under the norvaline analytical conditions 1, to find that L-norvaline was obtained with an optical purity of 98.3% e.e. in a yield of 64.4%.
Next, as a reduction step, the aeration of the oxygen-containing gas was stopped, 0.37 equivalent of ammonium formate was added relative to the starting material DL-norvaline (cells prepared from 132 g of the culture), and a reaction was conducted under conditions of a stirring rate of 300 rpm and a pH of the reaction mixture of 8.0 at 30°C for 62 hours. A sample (0.1 g) was collected from the reaction mixture after the completion of the reaction, and the optical purity and amount of norvaline contained in the reaction mixture were determined under after-mentioned norvaline analytical conditions 1, to find that L-norvaline was obtained with an optical purity of 100% e.e. in a yield of 86.6%.
The reaction mixture after the completion of the reaction was analyzed by high-performance liquid chromatography to find that α-ketovaleric acid remained slightly (about 4 percent by weight on the basis of the starting material DL-norvaline).

EXAMPLE 16

Process of Controlling pH with Formic Acid in Oxidation Step and Supplementing Ammonium Formate in Reduction Step

An oxidation step and a reduction step were carried out by the procedure of Example 15, except for using formic acid instead of sulfuric acid for the pH control of the reaction mixture in the oxidation step. As a result, L-norvaline was obtained with an optical purity of 100% e.e. in a yield of 92.8%.
The reaction mixture after the completion of the reaction was analyzed by high-performance liquid chromatography to detect no α-ketovaleric acid.

EXAMPLE 17

Process of Controlling pH with Formic Acid in Oxidation Step Without Supplementation of Ammonium Formate in Reduction Step

An oxidation step and a reduction step were carried out by the procedure of Example 15, except for using formic acid instead of sulfuric acid for controlling the pH of the reaction mixture in the oxidation step, and for supplementing no ammonium formate in the reduction step. As a result, L-norvaline was obtained with an optical purity of 100% e.e. in a yield of 93.3%.
The concentration of α-ketovaleric acid remained after the completion of the reaction was determined by the procedure

of Example 13, to detect no α-ketovaleric acid.

These results demonstrate as follows. When sulfuric acid is used for pH control in the oxidation step, α-keto acid remains after the completion of the reaction, and a reductive amination reaction does not sufficiently proceed in the reduction step (Example 15). In contrast, when formic acid is used for pH control in the oxidation step, the reduction step fully proceeds, and there remains no α-keto acid after the completion of the reaction (Examples 16 and 17). In this case, the yield does not vary regardless of whether ammonium formate is supplemented in the reduction step (Example 16) or not (Example 17). This demonstrates that, when formic acid is wasted in the oxidation step, formic acid can be efficiently supplemented by using formic acid for pH control.

REFERENTIAL EXAMPLE 1

*Escherichia coli* strain HB101 was transformed with the four-gene-containing plasmid pSFCPC01 prepared in Example 12, and the transformant was cultivated in the 2x YT medium (as with Preparation Example 14) at 33°C overnight. The medium was combined with 0.1 mM IPTG to induce gene expression, cells were collected therefrom, and thereby yielded *Escherichia coli* expressing four genes.

A series of reaction mixtures was prepared by mixing cells prepared from 132 g of the culture, 8 percent by weight of DL-norvaline, 0.02% ADEKANOL (antifoaming agent; produced by Asahi Denka Kogyo K.K.), and ammonium formate in amounts of 0 (not added), 0.2 equivalent, 0.4 equivalent, and 0.6 equivalent relative to the starting material DL-norvaline, and adjusting to pH 8.0 with formic acid. In 1-liter mini jars were placed 400 ml of the reaction mixtures, respectively, and reactions were conducted as an oxidation reaction under conditions of an air aeration rate of 0.5 vvm and a stirring rate of 500 rpm at 30°C. The time periods for completing the oxidation step under the respective conditions were 28 hours or more, 28 hours, 24 hours, and 28 hours, respectively. Samples (each 0.1 g) were collected from the reaction mixtures after the completion of the reactions, and the optical purities of norvaline contained in the reaction mixtures were analyzed under the norvaline analytical conditions 1. The results are shown in Table 6. These results showed that, when ammonium formate is not added at the beginning of reaction in the oxidation step, the optical purity is at most 49.3% e.e. even the reaction is conducted for more than 28 hours, and the reaction proceeds very slowly, as compared with the optical purity of 99% e.e. or more where ammonium formate is added in amounts of 0.2 equivalent or more relative to the starting material DL-norvaline. The optical purities of L-norvaline are sufficiently high with no difference among them at amounts of ammonium formate of 0.2 equivalent or more.

REFERENTIAL EXAMPLE 2

Oxidation steps were carried out by the procedure of Referential Example 1, except for using formic acid for pH control instead of ammonium formate, and for adjusting pH of the reaction mixture to 7.0, 7.5, 8.0, and 8.5, respectively. Samples (each 0.1 g) were colleted from the reaction mixtures after the completion of the oxidation step, and the optical purities of norvaline contained in the reaction mixtures were determined under after-mentioned norvaline analytical conditions 1. The results are shown in Table 7.

EXAMPLE 18 (Proposal of your company: EXAMPLE 13 as added; former REFERENTIAL EXAMPLE 3)

*Escherichia coli* strain HB101 was transformed with the four-gene-containing plasmid pSFCPC01 prepared in Example 12, and the transformant was cultivated in the 2x YT medium (as with Preparation Example 14) at 33°C overnight. The medium was combined with 0.1 mM IPTG to induce gene expression, cells were collected therefrom, and thereby yielded *Escherichia coli* expressing four genes.

A reaction mixture was prepared by mixing the cells prepared from 132 g of the culture with 8 percent by weight DL-norvaline, 0.6 equivalent of ammonium formate relative to norvaline, and 0.02% ADEKANOL (antifoaming agent; Asahi Denka Kogyo K.K.), and adjusting to pH 8.0 with sulfuric acid. In a 1-liter mini jar was placed 400 ml of the reaction mixture, and a reaction was conducted as an oxidation step under conditions of an air aeration rate of 0.5 vvm and a stirring rate of 500 rpm at 30°C for 43 hours. A sample (0.1 g) was collected from the reaction mixture after the completion of the oxidation step, and the optical purity and amount of norvaline contained in the reaction mixture were determined under the norvaline analytical conditions 1, to find that L-norvaline was obtained with an optical purity of 98.3% e.e. in a yield of 64.4%.

Next, the aeration of the oxygen-containing gas was stopped, pH of the reaction mixture was adjusted to 6.5, 7.0, 7.5, 8.0, 8.5, and 9.0, respectively, with formic acid, and reactions were conducted as a reduction step under conditions of a stirring rate of 300 rpm and a temperature of 30°C for 62 hours. Samples (each 0.1 g) were collected from the reaction mixtures after the completion of the reactions, and the optical purities and amounts of norvaline contained in the reaction mixtures were determined under the norvaline analytical conditions 1, to find that L-norvaline was obtained with an optical purity of 100% e.e. in a yield of 86.6%.

The reaction mixtures after the completion of the reactions were analyzed by high-performance liquid chromatography to find that α-ketovaleric acid remained slightly (about 4 percent by weight on the basis of the starting material DL-norvaline).

(Evaluation Test)

Norvaline Analytical Conditions 1

A sample reaction mixture (0.1 g) containing norvaline was suspended with 0.1 M hydrochloric acid, the suspension was centrifuged to yield a supernatant, and the supernatant was diluted with a 50% aqueous acetonitrile solution containing 0.4% triethylamine. The dilution (100 μL) was combined with 0.2% 2,3,4,6-tetra-O-acetyl-β-D-glucopyranosyl isothiocyanate (GITC) (100 μL), followed by a reaction at 40°C for 90 minutes. The resulting reaction mixture was analyzed by high-performance liquid chromatography under following conditions.

Column: Inertsil ODS-3 (4.6 mm in diameter x 250 mm) (GL Sciences Inc.)
Temperature: 25°C
Detection: UV 250 nm
Eluent: (10 mM potassium phosphate buffer (pH 2.5))/(methanol) = 45/55
Flow rate: 1 mL/min.

Under these conditions, 2,3,4,6-tetra-O-acetyl-β-D-glucopyranosyl-isothiocyanated L-norvaline and 2,3,4,6-tetra-O-acetyl-β-D-glucopyranosyl-isothiocyanated D-norvaline were detected at retention times of 8.8 minutes and 11.8 minutes, respectively.

Norvaline Analytical Conditions 2

A solid (5 mg) containing norvaline was dissolved in 1 mL of water and analyzed under the following conditions.

Column: CROWNPAK CR(+) (4 mm in diameter x 150 mm) (Daicel Chemical Industries, Ltd.)
Temperature: 40°C
Detection: UV 210 nm
Eluent: aqueous perchloric acid solution (pH 2.0)
Flow rate: 0.8 mL/minute

Under these conditions, L-norvaline and D-norvaline were detected at retention times of 2.5 minutes and 3.3 minutes, respectively.

2-Oxopentanoic Acid Analytical Conditions

A sample reaction mixture (0.1 g) upon the preparation of reaction mixture was suspended with 0.1 M hydrochloric acid, the suspension was centrifuged to yield a supernatant, and the supernatant was analyzed under the following conditions.

Column: Wakosil-II 5C18 HG (4.6 mm in diameter x 250 mm) (Wako Pure Chemical Industries, Ltd.)
Temperature: 40°C
Detection: UV 230 nm
Eluent: (0.1 percent by volume aqueous trifluoroacetic acid solution)/(acetonitrile) = 95/5
Flow rate: 1 mL/minute

Under these conditions, 2-oxopentanoic acid was detected at a retention time of 8.6 minutes.
[Table 1]

Table 1

| Preparation Example | D-Amino acid oxidase or dehydrogenase | Nva [% by weight] | Optical purity [% e.e. (L)] | Nva residual rate [%] |
|---|---|---|---|---|
| 14 | D-amino acid oxidase derived from *Candida boidinii* strain DSM 70026 | 1<br>4<br>10 | 100<br>100<br>39 | 57<br>60<br>82 |
| 15 | D-amino acid oxidase derived from *Trigonopsis variabilis* strain CBS 4095 | 1<br>4<br>10 | 100<br>100<br>24 | 59<br>49<br>91 |
| 16 | D-amino acid dehydrogenase derived from *Escherichia coli* strain K-12 | 1<br>4 | 100<br>57 | 54<br>68 |

[Table 2]

[Table 2]

| Preparation Example | L-Amino acid dehydrogenase | Substrate [% by weight] | Optical purity [% e.e. (L)] | Nva formation rate [%] |
|---|---|---|---|---|
| 17 | Alanine dehydrogenase derived from *Geobacillus stearothermophilus* strain IFO 12550 | 2<br>4<br>10 | 100<br>100<br>100 | 79<br>81<br>74 |
| 18 | Alanine dehydrogenase derived from *Bacillus thermocatenulatus* strain DSM 730 | 2<br>4<br>10 | 100<br>100<br>100 | 68<br>73<br>76 |
| 19 | Alanine dehydrogenase derived from *Bacillus sphaericus* strain IFO 3525 | 2<br>4<br>10 | 100<br>100<br>100 | 76<br>76<br>77 |
| 20 | Alanine dehydrogenase derived from *Shewanella sp.* strain Ac10 | 2<br>4<br>10 | 100<br>100<br>100 | 79<br>82<br>62 |
| 21 | Leucine dehydrogenase derived from *Geobacillus stearothermophilus* strain IFO 12550 | 2<br>4<br>10 | 100<br>100<br>100 | 90<br>89<br>32 |

(continued)

| Preparation Example | L-Amino acid dehydrogenase | Substrate [% by weight] | Optical purity [% e.e. (L)] | Nva formation rate [%] |
|---|---|---|---|---|
| 22 | Leucine dehydrogenase derived from *Bacillus sphaericus* strain IFO 3525 | 2<br>4<br>10 | 100<br>100<br>100 | 92<br>92<br>39 |
| 23 | Phenylalanine dehydrogenase derived from *Thermoactinomyces intermedius* | 2<br>4<br>10 | 100<br>100<br>100 | 100<br>88<br>73 |

[Table 3]

Table 3

| Preparation Example | Branched-chain-amino-acid transaminase | Amino donor | Substrate [% by weight] | Optical purity [% e.e. (L)] | Nva formation rate [%] |
|---|---|---|---|---|---|
| 23 | Branched-chain-amino-acid transaminase derived from *Escherichia coli* strain K-12 | Glutamic acid | 2<br>4 | 100<br>100 | 88<br>90 |
| 24 | | Aspartic acid | 2<br>4 | 100<br>100 | 44<br>35 |
| 25 | branched-chain-amino-acid transaminase derived from *Bacillus subtilis* | Glutamic acid | 2<br>4 | 100<br>100 | 70<br>70 |
| 26 | | Aspartic acid | 2<br>4 | 100<br>100 | 41<br>36 |

[Table 4]

Table 4

| Production Example | Reaction system | | | 20 hr later | | 45 hr later | |
|---|---|---|---|---|---|---|---|
| | Plasmid | Nva [% by weight] | Sodium formate [M] | Optical purity [%} | Nva residual rate [%] | Optical purity [%} | Nva residual rate [%] |
| 28 | pSUCBDO1/ pSF-TIP2 | 4 | 0.20 | 100 | 73 | - | - |
| 29 | | 10 | 0.51 | 48 | 79 | 100 | 77 |
| 30 | pSUCBDO1/ pSF-GSA2 | 4 | 0.20 | 100 | 44 | - | - |
| 31 | | 10 | 0.51 | 61 | 64 | 100 | 82 |

[Table 5]

Table 5

| Example | Reaction system | | | 20 hr later | | 45 hr later | |
|---|---|---|---|---|---|---|---|
| | Plasmid | Nva [% by weight] | Sodium formate [M] | Optical purity [%} | Nva residualrate [%] | Optical purity [%} | Nva residualrate [%] |
| 2 | pSFTPCO1 | 4 | 0.20 | 100 | 58 | - | - |
| 3 | | 10 | 0.51 | 61 | 79 | 100 | 72 |
| 4 | | 12 | 0.61 | 53 | 72 | 100 | 69 |
| 5 | pSFGACO1 | 4 | 0.20 | 100 | 51 | - | - |
| 6 | | 10 | 0.51 | 61 | 74 | 100 | 74 |
| 7 | | 12 | 0.61 | 42 | 93 | 100 | 68 |

[Table 6]

Table 6

| Sodium formate [equivalent] | Time for oxidization step | Optical purity [%] |
|---|---|---|
| 0 | more than 28 hr | 49.3 |
| 0.2 | 28 hr | more than 99 |
| 0.4 | 24 hr | more than 99 |
| 0.6 | 28 hr | more than 99 |

[Table 7]

Table 7

| pH [-] | Optical purity [%] |
|---|---|
| 7.0 | 16.5 |
| 7.5 | 55.8 |
| 8.0 | 99.5 |
| 8.5 | 92.3 |

Industrial Applicability

Processes for producing the L-form of an amino acid according to the present invention are useful typically as processes for efficiently producing the L-form of an amino acid in a single system using enzymatic reactions. According to these processes, L-amino acids can be obtained in high optical yields from inexpensive enantiomeric mixtures of amino acids. Among them, a process, in which a hydrogen peroxide catabolic enzyme is further expressed in a single host, can be carried out under mild reaction conditions to avoid decomposition of an intermediate keto acid and can thereby improve the productivity of the L-form of the amino acid.

**Claims**

1. A process for producing an L-form of an amino acid enzymatically from an enantiomeric mixture of the amino acid, the process comprising
the step of contacting a transformant or a treated product thereof with the enantiomeric mixture of the amino acid, the transformant comprising a single host and one or more recombinant vectors introduced into the single host, wherein the one or more recombinant vectors comprise one or more expression vectors and nucleotide sequences, the nucleotide sequences comprising :

    a nucleotide sequence encoding an enzyme capable of converting a D-form of the amino acid into a keto acid; and a nucleotide sequence encoding an enzyme capable of converting the keto acid into the L-form of the amino acid, and the nucleotide sequences being integrated into the one expression vector or the different expression vectors, respectively.

2. A process for producing an L-form of an amino acid, according

to claim 1, wherein
the transformant comprises the single host and the one or more recombinant vectors introduced into the single host, wherein the one or more recombinant vectors comprise one or more expression vectors and nucleotide sequences, the nucleotide sequences comprising :

the nucleotide sequence encoding the enzyme capable of converting the D-form of the amino acid into the keto acid;
the nucleotide sequence encoding the enzyme capable of converting the keto acid into the L-form of the amino acid; and
a nucleotide sequence encoding a coenzyme-regenerating enzyme, and the nucleotide sequences being integrated into the one expression vector or the different expression vectors, respectively.

**3.** A process for producing an L-form of an amino acid, according to one of claims 1 and 2, wherein
the transformant comprises the single host and the one or more recombinant vectors introduced into the single host, wherein the one or more recombinant vectors comprise one or more expression vectors and nucleotide sequences, the nucleotide sequences comprising :

the nucleotide sequence encoding the enzyme capable of converting the D-form of the amino acid into the keto acid;
the nucleotide sequence encoding the enzyme capable of converting the keto acid into the L-form of an amino acid; and
a nucleotide sequence encoding a hydrogen peroxide catabolic enzyme,
and the nucleotide sequences being integrated into the one expression vector or the different expression vectors, respectively.

**4.** A process for producing an L-form of an amino acid, according to any one of claims 1 to 3, wherein
the enzyme capable of converting the D-form of the amino acid into the keto acid is a D-amino acid oxidase and/or a D-amino acid dehydrogenase.

**5.** A process for producing an L-form of an amino acid, according to claim 4, wherein
the D-amino acid oxidase is derived from at least one microorganism selected from the group consisting of *Candida boidinii, Trigonopsis variabilis,* and *Schizosaccharomyces pombe.*

**6.** A process for producing an L-form of an amino acid, according to claim 4, wherein
the D-amino acid dehydrogenase is derived from *Escherichia coli.*

**7.** A process for producing an L-form of an amino acid, according to any one of claims 1 to 3, wherein
the enzyme capable of converting the keto acid into the L-form of the amino acid is an L-amino acid dehydrogenase and/or an L-amino acid transaminase.

**8.** A process for producing an L-form of an amino acid, according to claim 7, wherein
the L-amino acid dehydrogenase is derived from at least one microorganism selected from the group consisting of a phenylalanine dehydrogenase derived from *Thermoactinomyces intermedius,* an alanine dehydrogenase derived from *Geobacillus stearothermophilus,* an alanine dehydrogenase derived from *Bacillus thermocatenulatus,* an alanine dehydrogenase derived from *Bacillus sphearicus,* an alanine dehydrogenase derived from *Shewanella species,* a leucine dehydrogenase derived from *Geobacillus stearothermophilus,* and a leucine dehydrogenase derived from *Bacillus sphaericus.*

**9.** A process for producing an L-form of an amino acid, according to claim 2, wherein
the coenzyme-regenerating enzyme is a formate dehydrogenase.

**10.** A process for producing an L-form of an amino acid, according to claim 9, wherein
the formate dehydrogenase is derived from *Mycobacterium vaccae.*

**11.** A process for producing an L-form of an amino acid, according to claim 3, wherein
the hydrogen peroxide catabolic enzyme is a catalase.

**12.** A process for producing an L-form of an amino acid, according to claim 11, wherein the catalase is derived from *Escherichia coli.*

**13.** A process for producing an L-form of an amino acid, according to one of claims 2 and 3, wherein:

the enzyme capable of converting the D-form of the amino acid into the keto acid is a D-amino acid oxidase;
the enzyme capable of converting the keto acid into the L-form of the amino acid is an L-amino acid dehydrogenase;
the coenzyme-regenerating enzyme is a formate dehydrogenase; and
the hydrogen peroxide catabolic enzyme is a catalase.

**14.** A process for producing an L-form of an amino acid, according to claim 13, wherein:

the D-amino acid oxidase is derived from *Candida boidinii;*
the L-amino acid dehydrogenase is a phenylalanine dehydrogenase derived from *Thermoactinomyces intermedius;*
the formate dehydrogenase is derived from *Mycobacterium vaccae;* and
the catalase is derived from *Escherichia coli.*

**15.** A process for producing an L-form of an amino acid, according to any one of claims 1 to 14, wherein the L-form of the amino acid is L-norvaline.

**16.** A transformant comprising a single host and one or more recombinant vectors introduced into the single host,
wherein the one or more recombinant vectors comprises one or more expression vectors and nucleotide sequences, and
wherein the nucleotide sequences comprise:

a nucleotide sequence encoding an enzyme capable of converting the D-form of an amino acid into a keto acid;
a nucleotide sequence encoding an enzyme capable of converting a keto acid into the L-form of an amino acid;
a nucleotide sequence encoding a coenzyme-regenerating enzyme; and
a nucleotide sequence encoding a hydrogen peroxide catabolic enzyme,
and the nucleotide sequences are integrated into the one expression vector or the different expression vectors, respectively.

**17.** A transformant according to claim 16, wherein:

the enzyme capable of converting the D-form of the amino acid into the keto acid is a D-amino acid oxidase;
the enzyme capable of converting the keto acid into the L-form of the amino acid is an L-amino acid dehydrogenase;
the coenzyme-regenerating enzyme is a formate dehydrogenase; and
the hydrogen peroxide catabolic enzyme is a catalase.

**18.** A transformant according to claim 17, wherein:

the D-amino acid oxidase is derived from *Candida boidinii;*
the L-amino acid dehydrogenase is a phenylalanine dehydrogenase derived from *Thermoactinomyces intermedius;*
the formate dehydrogenase is derived from *Mycobacterium vaccae;* and
the catalase is derived from *Escherichia coli.*

**19.** A recombinant vector comprising a single expression vector and nucleotide sequences integrated into the single expression vector,
wherein the nucleotide sequences comprise:

a nucleotide sequence encoding an enzyme capable of converting the D-form of an amino acid into a keto acid;
a nucleotide sequence encoding an enzyme capable of converting a keto acid into the L-form of an amino acid;
a nucleotide sequence encoding a coenzyme-regenerating enzyme; and

a nucleotide sequence encoding a hydrogen peroxide catabolic enzyme.

**20.** A recombinant vector according to claim 19, wherein:

the enzyme capable of converting the D-form of the amino acid into the keto acid is a D-amino acid oxidase;
the enzyme capable of converting the keto acid into the L-form of the amino acid is an L-amino acid dehydrogenase;
the coenzyme-regenerating enzyme is a formate dehydrogenase; and
the hydrogen peroxide catabolic enzyme is a catalase.

**21.** A recombinant vector according to claim 20, wherein:

the D-amino acid oxidase is derived from *Candida boidinii;*
the L-amino acid dehydrogenase is a phenylalanine dehydrogenase derived from *Thermoactinomyces intermedius;*
the formate dehydrogenase is derived from *Mycobacterium vaccae;* and
the catalase is derived from *Escherichia coli.*

**22.** A process for producing an L-form of an amino acid enzymatically from an enantiomeric mixture of the amino acid, the process comprising
two or more different reaction steps including an oxidation step and a reduction step,
wherein the oxidation step mainly comprises oxidizing a D-form of the amino acid using a transformant or a treated product thereof, the transformant coexpressing at least an enzyme capable of oxidizing the D-form of the amino acid to a keto acid and a hydrogen peroxide catabolic enzyme, and
wherein the reduction step mainly comprises synthetically producing the L-form of the amino acid from the keto acid using another transformant or a treated product thereof, the transformant expressing an enzyme capable of producing the L-form of the amino acid from the keto acid and optionally coexpressing a coenzyme-regenerating enzyme according to necessity.

**23.** A process for producing an L-form of an amino acid, according to claim 22, wherein
the transformant of any one of claims 16 to 18 is used.

**24.** A process for producing an L-form of an amino acid, according to one of claims 22 and 23, wherein
the oxidation step and the reduction step are carried out under different reaction conditions.

**25.** A process for producing an L-form of an amino acid, according to claim 24, wherein:

the oxidation step is carried out under a condition with aeration of an oxygen-containing gas; and
the reduction step is carried out under a condition without aeration of an oxygen-containing gas or under a condition with aeration of an oxygen-containing gas at an aeration rate lower than that in the oxidation step.

**26.** A process for producing an L-form of an amino acid, according to claim 25, wherein:

in the oxidation step, an aeration rate of the oxygen-containing gas is 1 percent by volume per minute or more relative to a reaction mixture; and
in the reduction step, an aeration rate of the oxygen-containing gas is less than 10 percent by volume per minute relative to the reaction mixture.

**27.** A process for producing an L-form of an amino acid, according to claim 25, wherein:

in the oxidation step, an aeration rate of the oxygen-containing gas is 2 percent by volume per minute or more relative to a reaction mixture; and
in the reduction step, an aeration rate of the oxygen-containing gas is less than 2 percent by volume per minute relative to the reaction mixture.

**28.** A process for producing an L-form of an amino acid, according to claim 25, wherein:

in the oxidation step, an aeration rate of the oxygen-containing gas is 5 percent by volume per minute or more

relative to a reaction mixture; and
in the reduction step, an aeration rate of the oxygen-containing gas is less than 5 percent by volume per minute relative to the reaction mixture.

**29.** A process for producing an L-form of an amino acid, according to claim 25, wherein:

in the oxidation step, an aeration rate of the oxygen-containing gas is 1 percent by volume per minute or more relative to a reaction mixture; and
in the reduction step, substantially no aeration of the oxygen-containing gas is conducted.

**30.** A process for producing an L-form of an amino acid, according to claim 24, wherein
in the oxidation step, a reaction mixture in the reduction step is controlled to have a pH lower than that of the reaction mixture.

**31.** A process for producing an L-form of an amino acid, according to claim 30, wherein:

in the oxidation step, the reaction mixture is controlled to have a pH of 7.0 to 9.5; and
in the reduction step, the reaction mixture is controlled to have a pH falling within a range of 6.0 to 8.5 and being lower than the pH of the reaction mixture in the oxidation step.

**32.** A process for producing an L-form of an amino acid, according to claim 30, wherein:

in the oxidation step, the reaction mixture is controlled to have a pH of 7.5 to 9.0; and
in the reduction step, the reaction mixture is controlled to have a pH falling within a range of 6.5 to 8.0 and being lower than the pH of the reaction mixture in the oxidation step.

**33.** A process for producing an L-form of an amino acid, according to any one of claims 9, 10, 13 to 15, and 22 to 32, wherein
the pH control in a reaction for converting the D-form of the amino acid into the keto acid is carried out using formic acid or a salt thereof.

**34.** A process for producing an L-form of an amino acid, according to claim 24, wherein
the transformant of any one of claims 16 to 18 or a treated product thereof is supplemented to a reaction mixture before or after a switch from the oxidation step to the reduction step.

**35.** A process for producing an L-form of an amino acid, according to any one of claims 1 to 15 and 22 to 34, further comprising
the step of using a reaction mixture containing 0.2 equivalent or more of formate ion and/or ammonium ion relative to the material enantiomeric mixture of the amino acid.

Fig. 1

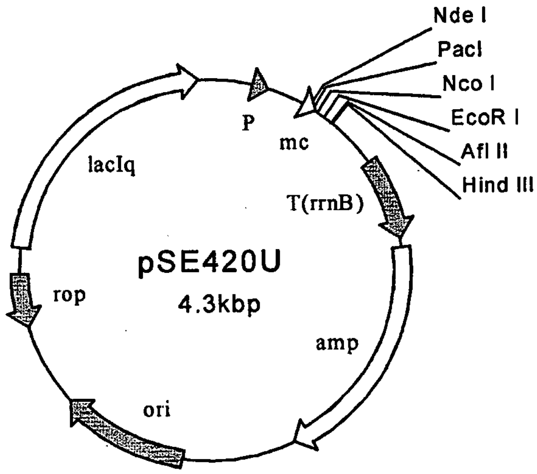


lac $I^q$, lac repressor; P(trc), trc promoter;

mc, multicloning site; T(rrnB), rrnB terminator;

amp, ampicillin resistance gene; ori, origin of replication;

rop, rop protein gene

Fig. 2

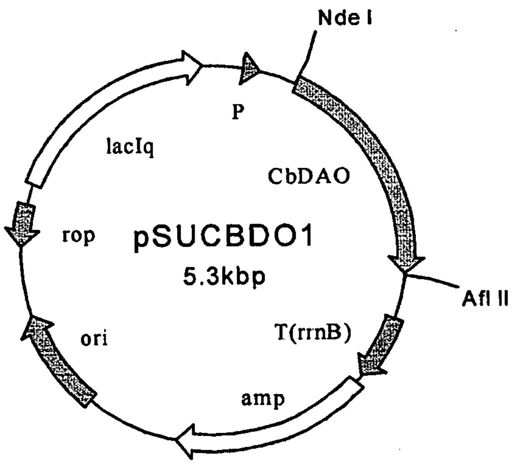


CbDAO, *Candida boidinii*-derived D-amino acid oxidase gene

Fig. 3

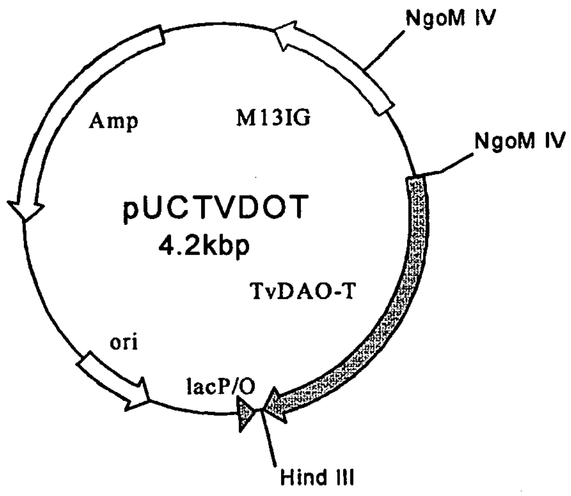


TvDAO-T, *Trigonopsis variabilis*-derived D-amino acid oxidase gene second exon

Fig. 4

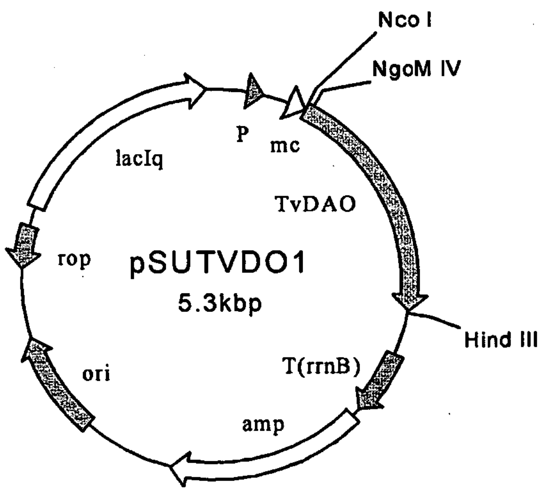


TvDAO, *Trigonopsis variabilis*-derived D-amino acid oxidase gene

Fig. 5

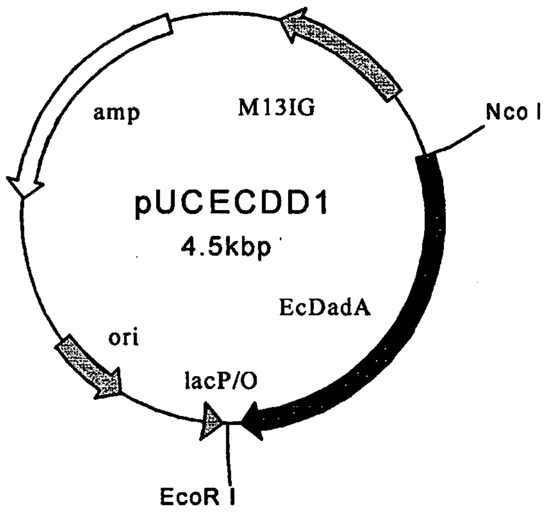


Fig. 6

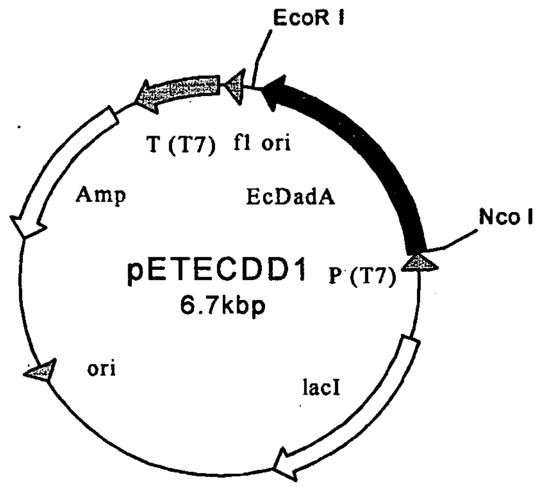


P (T7), T7 promoter; T (P7), T7 terminator;
f1 ori, f1 origin of replication

Fig. 7

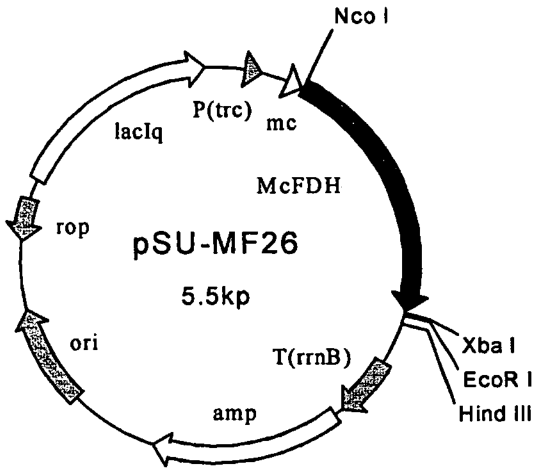


McFDH, *Mycobacterium vaccae*-derived formate dehydrogenase gene

Fig. 8

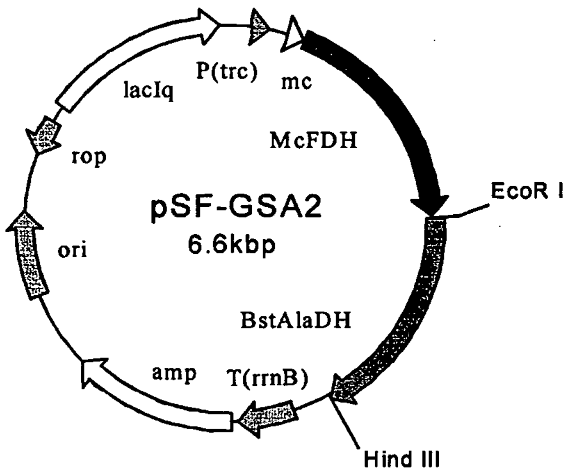


BstAlaDH, *Geobacillus stearothermophilus*-derived alanine dehydrogenase gene

Fig. 9

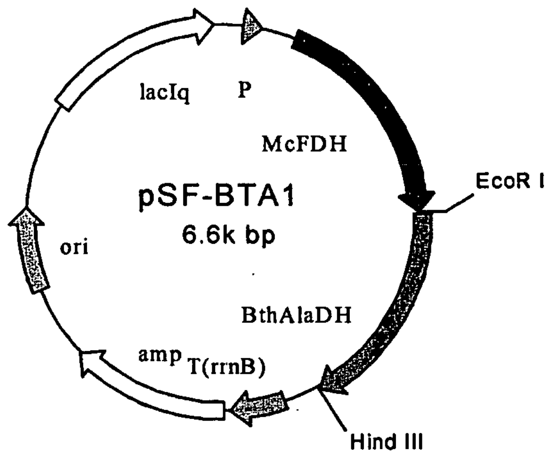


BthAlaDH, *Bacillus thermocatenulatus*-derived alanine dehydrogenase gene

Fig. 10

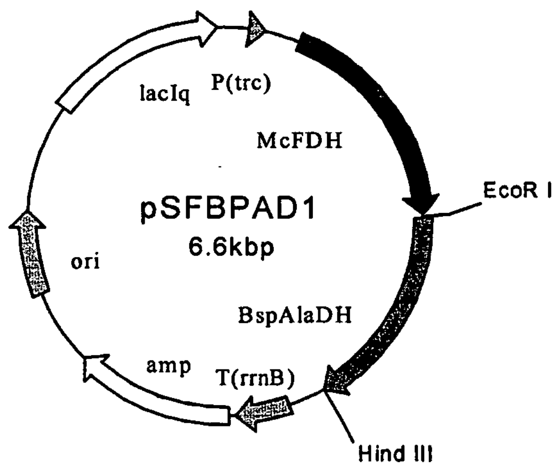


BspAlaDH, *Bacillus sphaericus*-derived alanine dehydrogenase gene

Fig. 11

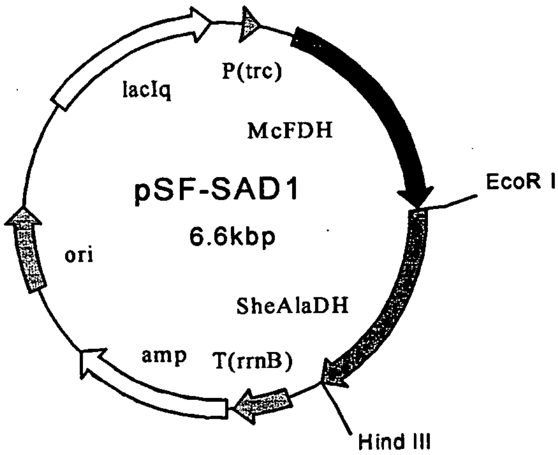


SheAlaDH, *Shewanella* sp.-derived alanine dehydrogenase gene

Fig. 12

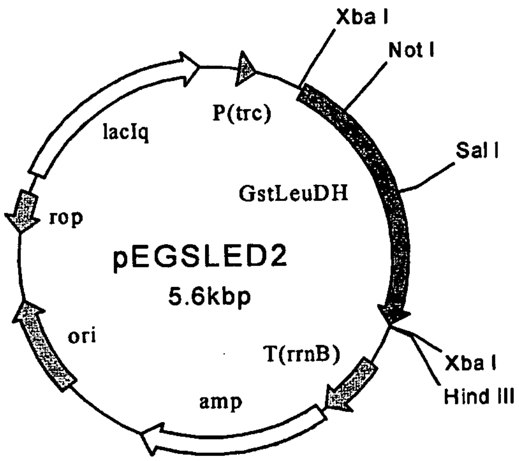


GstLeuDH, *Geobacillus stearothermophilus*-derived alanine dehydrogenase gene

Fig. 13

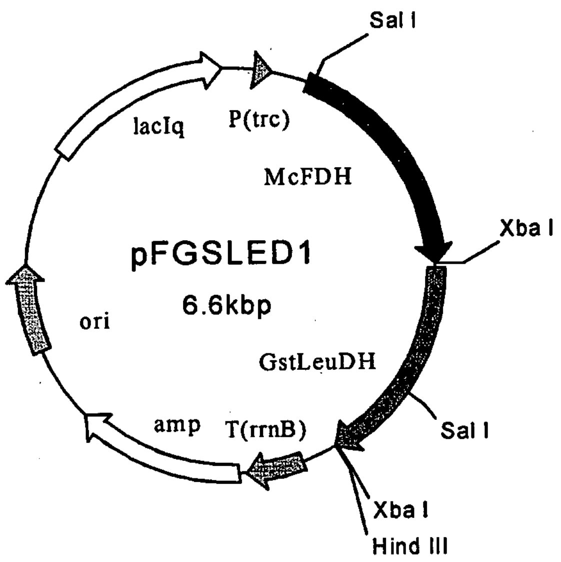


Fig. 14

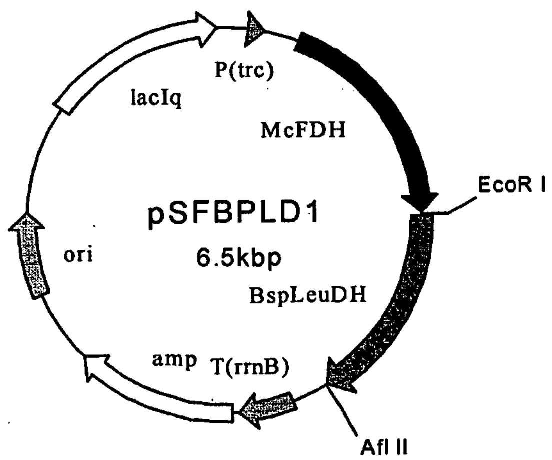


BspLeuDH, *Bacillus sphaericus*-derived leucine dehydrogenase gene

Fig. 15

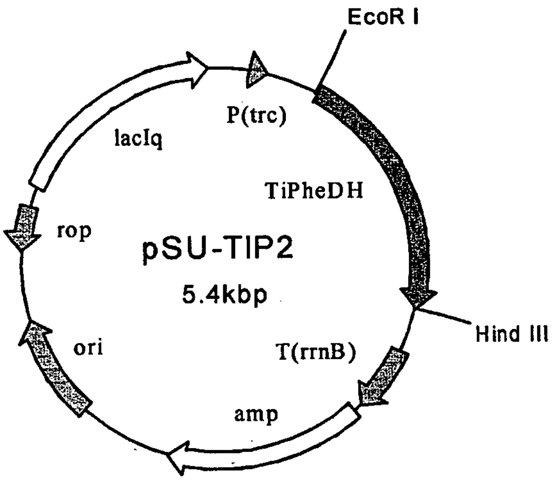


TiPheDH, *Thermoactinomyces intermedius*-derived phenylalanine dehydrogenase gene

Fig. 16

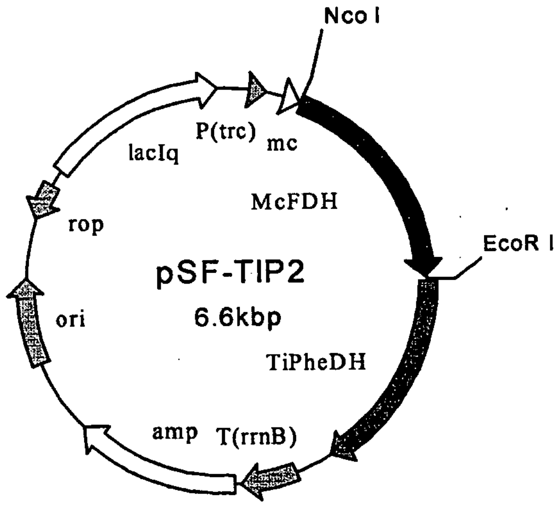

Fig. 17

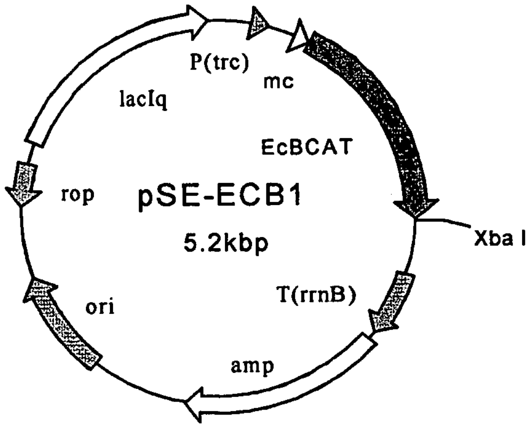


EcBCAT, *Escherichia coli*-derived branched-chain-amino-acid transaminase gene

Fig. 18

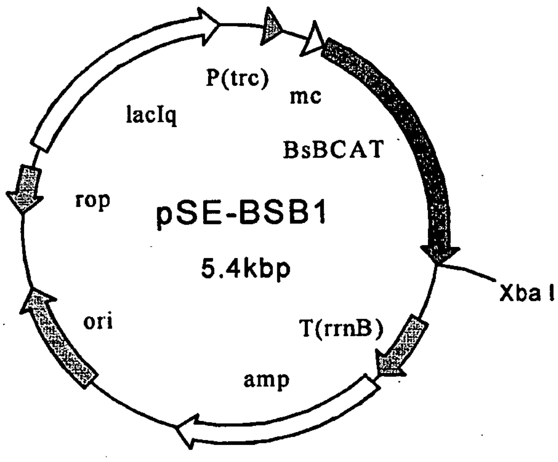


BsBCAT, Bacillus subtilis-derived branched-chain-amino-acid transaminase gene

Fig. 19

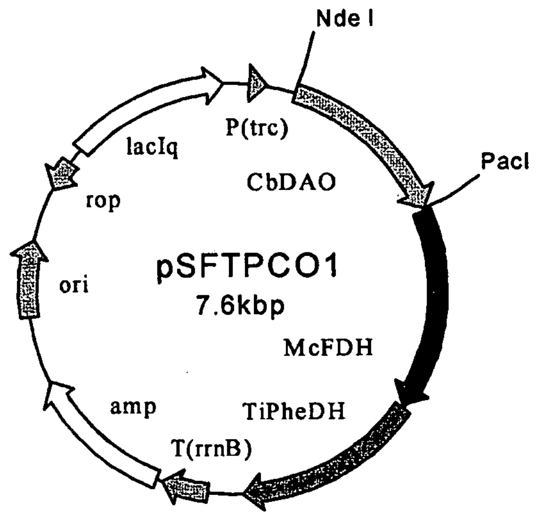
Nde I
PacI
P(trc)
lacIq
rop
CbDAO
ori
pSFTPCO1
7.6kbp
McFDH
amp
TiPheDH
T(rrnB)

Fig. 20

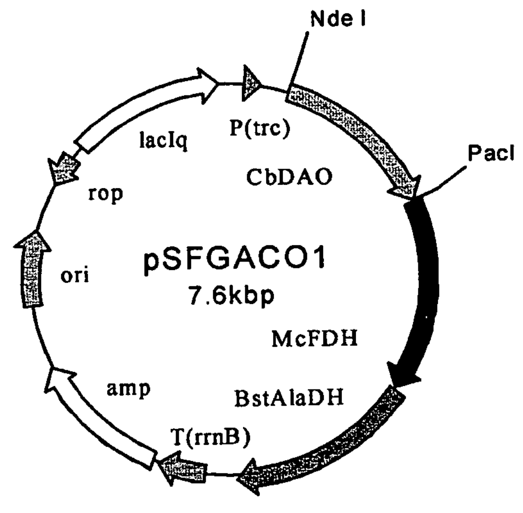
Nde I
PacI
P(trc)
lacIq
rop
CbDAO
ori
pSFGACO1
7.6kbp
McFDH
amp
BstAlaDH
T(rrnB)

## INTERNATIONAL SEARCH REPORT

International application No.
PCT/JP2006/311081

A. CLASSIFICATION OF SUBJECT MATTER
*C12P13/04*(2006.01)i, *C12N15/09*(2006.01)i, *C12N1/15*(2006.01)i, *C12N1/19*(2006.01)i, *C12N1/21*(2006.01)i, *C12N5/10*(2006.01)i, *C12R1/01*(2006.01)n, *C12R1/07*(2006.01)n, *C12R1/19*(2006.01)n, *C12R1/645*(2006.01)n,
According to International Patent Classification (IPC) or to both national classification and IPC

B. FIELDS SEARCHED

Minimum documentation searched (classification system followed by classification symbols)
C12P13/04, C12N15/09, C12N1/15, C12N1/19, C12N1/21, C12N5/10, C12R1/01, C12R1/07, C12R1/19, C12R1/645, C12R1/72

Documentation searched other than minimum documentation to the extent that such documents are included in the fields searched

Electronic data base consulted during the international search (name of data base and, where practicable, search terms used)
CA/WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | PATEL Ramesh N. et al., Enzymatic synthesis of chiral intermediates for Omapatrilat, an antihypertensive drug, Biomolecular Engineering, Vol.17, 2001, pages 167 to 182 | 1-35 |
| Y | JP 10-023896 A (Unitika Ltd.), 27 January, 1998 (27.01.98), Full text (Family: none) | 1-35 |
| P,X | WO 2005/090590 A1 (DEGUSSA AG.), 29 September, 2005 (29.09.05), Full text & DE 102004008445 A1 & US 2006/0063238 A1 | 1-35 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 September, 2006 (05.09.06) | 12 September, 2006 (12.09.06) |
| Name and mailing address of the ISA/ Japanese Patent Office | Authorized officer |
| Facsimile No. | Telephone No. |

Form PCT/ISA/210 (second sheet) (April 2005)

INTERNATIONAL SEARCH REPORT

International application No.
PCT/JP2006/311081

Continuation of A. CLASSIFICATION OF SUBJECT MATTER
(International Patent Classification (IPC))

*C12R1/72*(2006.01)n

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H1023896 A
- WO 2005090590 A
- EP 1375649 A
- JP H07108225 B
- JP S63157986 A
- JP S61239887 A
- JP S61146183 A
- JP 2002065270 A
- JP H01153084 A
- JP 2000189170 A
- JP H05284973 A
- JP S57183799 A
- EP 537456 A
- JP H08173170 A
- JP 2001506868 W
- JP 2003199595 A


### Non-patent literature cited in the description


- *Method Enzymol.,* 1957, vol. 3, 554
- *Agric. Biol. Chem.,* October 1987, vol. 51, 729
- *Biotechnol. Bioeng.,* 1994, vol. 14, 1288
- *Biomolecular Engineering,* 2001, vol. 17, 167
- *Chem. Comm.,* 2002, vol. 246
- *Agric. Biol. Chem.,* vol. 51, 729
- *Biochemistry,* 1987, vol. 26, 3612
- *Yeast,* 2000, vol. 16, 1217
- *J. Biotechnol.,* 2003, vol. 104, 5
- *Environ. Microbiol.,* 2002, vol. 4, 799
- *J. Bacteriol.,* 1997, vol. 58, 115
- *J. Biochem.,* 1990, vol. 108, 1063
- *J. Bacteriol.,* 1994, vol. 176, 1500
- *Curr. Microbiol.,* 2000, vol. 41, 290
- *Nature,* 2001, vol. 413, 852
- *J. Gen. Microbiol.,* 1987, vol. 133, 745-754
- *Biochemistry,* 1990, vol. 29, 1009
- *Biochimie,* 1989, vol. 71, 559
- *Appl. Environ. Microbiol.,* 1999, vol. 65, 4014
- *J. Biochem. Mol. Biol.,* 2003, vol. 36, 545
- *J. Bacteriol.,* 1998, vol. 180, 6298
- *Biochemistry,* 1992, vol. 203, 81
- *Biochemistry,* 1993, vol. 217, 469
- *Biochemistry,* 1988, vol. 27, 9056
- *Eur. J. Biochem.,* 1993, vol. 216, 539
- *Appl. Environ. Microbiol.,* 2004, vol. 70, 937
- *J. Biochem.,* 1991, vol. 109, 371
- *Arch. Microbiol.,* 1989, vol. 153, 12
- *Plant Mol. Biol.,* 1999, vol. 39, 149
- *Physiol. Chem. Phys.,* 1978, vol. 10, 483
- *J. Biol. Chem.,* 1967, vol. 242, 3614
- *Anal. Biochem.,* 1979, vol. 95, 188
- *Biochim. Biophys. Acta,* 1976, vol. 445, 622
- *J. Biol. Chem.,* 1975, vol. 250, 7819
- *Biochem. J.,* 1975, vol. 147, 327
- *J. Biochem.,* 1985, vol. 97, 993
- *J. Bacteriol.,* 1997, vol. 179, 496
- *Biochem. Biophys. Res. Commun.,* 1985, vol. 133, 134
- *J. Biol. Chem.,* 1979, vol. 254, 11664-11668
- *J. Biol. Chem.,* 1979, vol. 254, 4245-4252
- Biseibutsugaku Kisokoza 8 Idensikougaku. Kyoritsu Shuppan Co., Ltd,
- *Adv. Biochem. Eng.,* 1990, vol. 43, 75-102
- *Yeast,* 1992, vol. 8, 423-488
- *Nature,* 1985, vol. 315, 592-594
- *Gene,* 1985, vol. 39, 281
- *Mol. Gen. Genet.,* 1984, vol. 196, 175
- *FEMS Microbiol. Lett.,* 1985, vol. 26, 239
- *Appl. Environ. Microbiol.,* 1985, vol. 50, 94
- *J. Bacteriol.,* 1979, vol. 137, 614
- *J. Gen. Microbiol.,* 1992, vol. 138, 1003
- **HOPWOOD et al.** Genetic Manipulation of Streptomyces: A Laboratory Manual. Cold Spring Harbor Laboratories, 1985
- *Mol. Gen. Genet.,* 1986, vol. 203, 468-478
- *Gene,* 1991, vol. 103, 97-99
- *Gene,* 1995, vol. 165, 149-150
- *Actinomycetol,* 1997, vol. 11, 46-53
- *J. Bacteriol.,* 1981, vol. 145, 382-390
- *Mol. Cell. Biol.,* 1986, vol. 6, 80
- *EMBO J.,* 1987, vol. 6, 729
- *Nucleic Acids Res.,* 1985, vol. 13, 4267
- *Agri. Biol. Chem.,* 1990, vol. 54, 2521
- *Yeast,* 1991, vol. 7, 431-443

- *Mol. Cell. Biol.,* 1985, vol. 5, 3376
- *Nucleic Acids Res.,* 1987, vol. 15, 3859
- *Agri. Biol. Chem.,* 1987, vol. 51, 1587
- *Trends in Biotechnology,* 1989, vol. 7, 283-287
- *Biotechnology,* 1989, vol. 7, 596-603
- *Meth. Cell Biol.,* 1975, vol. 29, 39
- *Nucleic Acids Res.,* 1980, vol. 8, 4321
- *Appl. Environ. Mictobiol.,* 1999, vol. 65, 4014
- *Methods Enzymol.,* 1987, vol. 152, 116